(19) Europäisches Patentamt
European Patent Office
Office européen des brevets

(11) EP 4 061 366 B1

(12) **EUROPEAN PATENT SPECIFICATION**

(45) Date of publication and mention of the grant of the patent:
**25.02.2026 Bulletin 2026/09**

(21) Application number: **20824760.1**

(22) Date of filing: **18.11.2020**

(51) International Patent Classification (IPC):
***A61K 31/4402*** *(2006.01)* ***A61K 31/444*** *(2006.01)*
***A61P 35/00*** *(2006.01)* ***A61P 35/02*** *(2006.01)*

(52) Cooperative Patent Classification (CPC):
**A61K 31/444; A61K 31/4402; A61P 35/00; A61P 35/02**

(86) International application number:
**PCT/US2020/060955**

(87) International publication number:
**WO 2021/101929 (27.05.2021 Gazette 2021/21)**

(54) **INHIBITORS OF HISTONE DEMETHYLASES (PFI-63 AND PFI-90) FOR THE TREATMENT OF CANCER AND FOR THE INHIBITION OF HISTONE DEMETHYLASE IN CELLS**

HISTONE-DEMETHYLASE INHIBITOREN (PFI-63 UND PFI-90) ZUR KREBSBEHANDLUNG UND ZUR INHIBIERUNG DER HISTONE-DEMETHYLASE IN ZELLEN

INHIBITEURS DE L’HISTONE DEMETHYLASE (PFI-63 ET PFI-90) POUR LE TRAITEMENT DU CANCER ET POUR L’INHIBITION DE L’HISTONE DEMETHYLASE DANS LES CELLULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.11.2019 US 201962936722 P**

(43) Date of publication of application:
**28.09.2022 Bulletin 2022/39**

(73) Proprietor: **The United States of America, as represented by the Secretary, Department of Health and Human Services**
**Bethesda, MD 20892-6911 (US)**

(72) Inventors:
- **KHAN, Javed**
  **Rockville, Maryland 20855 (US)**
- **HAWLEY, Robert G.**
  **Bethesda, Maryland 20814 (US)**
- **WOLDEMICHAEL, Girma M.**
  **Bethesda, Maryland 20892 (US)**
- **PEACH, Megan L.**
  **Frederick, Maryland 21702 (US)**

(74) Representative: **Maiwald GmbH**
**Elisenhof**
**Elisenstraße 3**
**80335 München (DE)**

(56) References cited:
**WO-A1-2017/214413**

- **KINFE REDDA ET AL: "Syntheses of N-substituted 2(3,4)-pyridylcarboxylic acid hydrazides with analgesic and antiinflammatory activity", JOURNAL OF MEDICINAL CHEMISTRY, vol. 22, no. 9, 1 September 1979 (1979-09-01), pages 1079 - 1082, XP055156684, ISSN: 0022-2623, DOI: 10.1021/jm00195a013**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 061 366 B1

## Description

CROSS REFERENCE TO RELATED APPLICATIONS

This application claims the benefit of U.S. Provisional Patent Application No. 62/936722, filed November 18, 2019.

STATEMENT REGARDING FEDERALLY SPONSORED RESEARCH OR DEVELOPMENT

This invention was made with Government support under Grant Number ZIA BC 010806 awarded by the NCI Intramural Program. The government holds certain rights in this invention.

MATERIAL SUBMITTED ELECTRONICALLY

Submitted herewith is a computer-readable nucleotide sequence listing submitted concurrently herewith and identified as follows: one 1,281 byte ASCII (Text) file named "750948_ST25.TXT," created on November 17, 2020.

BACKGROUND OF THE INVENTION

Histone demethylases are enzymes that remove methyl groups from histone proteins. The enzymes alter transcriptional regulation by controlling methylation levels in histones and, in turn, regulate the chromatin state at specific gene loci. These enzymes play a role in epigenetic modification mechanisms. Specifically, histone demethylases play a role in the epigenetic landscape of the chromatin environment in various cancer cells.

Histone demethylases are therefore a possible target for the treatment of cancer. Accordingly, compositions and methods that may be used to modify the function of histone demethylases are needed. WO2017/214413 concerns certain imidazo(1,2-a)pyridine derivatives which are histone demethylase inhibitors and can be used to treat cancer, including rhabdomyosarcoma.

BRIEF SUMMARY OF THE INVENTION

The invention provides a pharmaceutical composition for use in treating cancer in a mammal. The composition comprises, consists essentially of or consists of one or more compounds selected from the group consisting of:

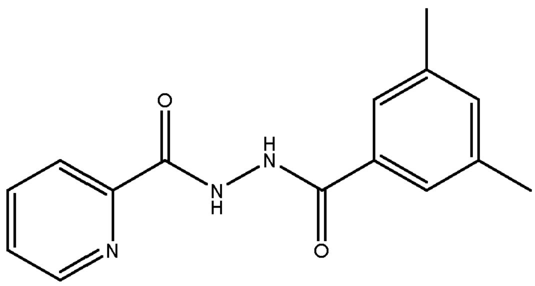
,
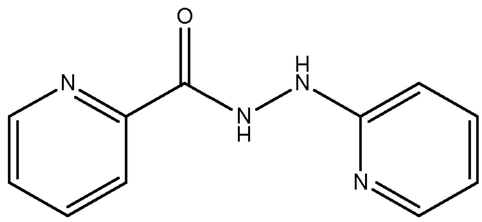
,

and pharmaceutically acceptable salts thereof, to the mammal.

The invention also provides a method for inhibiting a histone demethylase in cells *in vitro* comprising, consisting essentially of or consisting of contacting the cells with one or more compounds selected from the group consisting of:

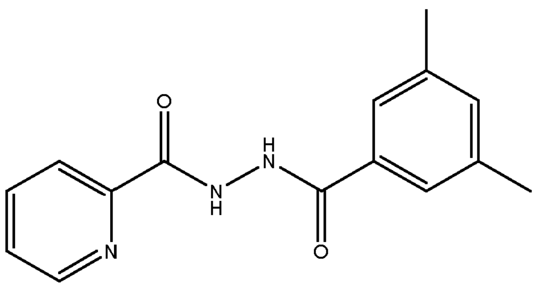
,
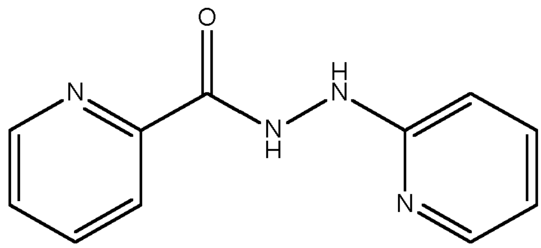
,

and pharmaceutically acceptable salts thereof.

BRIEF DESCRIPTION OF THE SEVERAL VIEWS OF THE DRAWING(S)

**Figure 1** is a visual representation of the pGreenFire green fluorescent protein (GFP)-luciferase reporter sequence (SEQ ID NO: 1) used in a high throughput screen for identifying compounds that disrupt the function of the PAX3-FOXO1 fusion transcription factor in RH4 fusion positive rhabdomyosarcoma cells (see Gryder et al., Cancer Discov. 7: 884-899 (2017)).

**Figure 2** depicts exemplary data of PAX3-FOXO1 function with respect to the pGreenFire green fluorescent protein (GFP)-luciferase reporter sequence (SEQ ID NO: 1) used for the high throughput screening assay.

**Figure 3** provides exemplary images depicting visualization via the pGreenFire green fluorescent protein (GFP)-luciferase reporter in PAX3-FOXO1 fusion positive rhabdomyosarcoma cells but not in RD fusion negative rhabdomyosarcoma cells.

**Figure 4** is a visual representation of the screening strategy employed to identify compounds that disrupt the function of the PAX3-FOXO1 fusion transcription factor in RH4 fusion positive rhabdomyosarcoma cells (see Gryder et al., Nat. Commun. 10: 3004 (2019)).

**Figure 5** depicts a box plot of weighted averages obtained from the initial high throughput screening assay. As can be seen in the Figure, of 62,643 tested compounds, 573 were identified as hits by the initial screen.

**Figure 6** is a visual depiction of the high throughput screening assay. As can be seen in the Figure, the follow up screen identified 64 compounds with selective activity.

**Figure 7** depicts an exemplary dose response curve for compound PFI-63 obtained during a dose response follow-up screen of the initial hits.

**Figure 8** is a diagram depicting the effect of PFI-63 on the cell number as measured by confluency of the RH4 and RH30 fusion positive rhabdomyosarcoma cell lines.

**Figure 9 is** a diagram depicting the effect of PFI-63 on the cell number as measured by confluency of the RH30.19luc fusion positive rhabdomyosarcoma cell lines.

**Figure 10** depicts an exemplary dose response curve for PFI-63 in RH4 cells at the 72 hour time point. The observed $IC_{50}$ is 3660 nM.

**Figure 11** depicts an exemplary dose response curve for PFI-63 in RH30 cells at the 72 hour time point. The observed $IC_{50}$ is 4815 nM.

**Figure 12** depicts the results of a gene set enrichment assay (GSEA; see Subramanian et al., Proc. Natl. Acad. Sci. USA, 102: 15545-15550 (2005)) investigating the cellular effects of PFI-63. The results indicate that targets of the PAX3-FOXO1 fusion transcription factor are downregulated in RH4 cells in the presence of PFI-63. For information on the gene set used for comparison, see Ebauer et al., Oncogene, 26: 7267-7281 (2007).

**Figure 13** depicts the results of a gene set enrichment assay (GSEA) investigating the cellular effects of PFI-63. The results indicate that targets of the PAX3-FOXO1 fusion transcription factor are downregulated in RH4 cells in the presence of PFI-63. For information on the gene set used for comparison, see Davicioni et al., Cancer Res., 66: 6936-6946 (2006).

**Figure 14** depicts the results of a gene set enrichment assay (GSEA) investigating the cellular effects of PFI-63. The results indicate that targets of the PAX3-FOXO1 fusion transcription factor are downregulated in RH4 cells in the presence of PFI-63. For information on the gene set used for comparison, see Cao et al., Cancer Res., 70: 6497-6508 (2010).

**Figure 15** depicts the results of a gene set enrichment assay (GSEA) investigating the cellular effects of PFI-63. The results indicate that targets of the PAX3-FOXO1 fusion transcription factor are downregulated in RH4 cells in the presence of PFI-63. For information on the gene set used for comparison, see Gryder et al., Cancer Discov., 7: 884-899 (2017).

**Figure 16** depicts the results of a gene set enrichment assay (GSEA) investigating the cellular effects of PFI-63. The results indicate that genes that are induced upon KDM3B suppression are upregulated in RH4 cells treated with PFI-63. For information on the gene set used for comparison, see Kuroki et al., Stem Cell Reports, 10: 1340-1354 (2018).

**Figure 17** depicts the results of a gene set enrichment assay (GSEA) investigating the cellular effects of PFI-63. The results indicate that genes that are induced upon KDM3B suppression are upregulated in RH4 cells treated with PFI-63. For information on the gene set used for comparison, see An et al., Biochem. Biophys. Res. Commun., 508: 576-582 (2019).

**Figure 18** depicts the results of a gene set enrichment assay (GSEA) investigating the cellular effects of PFI-63. The results indicate that RH4 cells treated with PFI-63 also experience upregulation of targets associated with myogenic

differentiation. For information on the gene set used for comparison, see Liberzon et al., Cell Systems, 1: 417-425 (2015).

**Figure 19** depicts the results of a gene set enrichment assay (GSEA) investigating the cellular effects of PFI-63. The results indicate that RH4 cells treated with PFI-63 also experience upregulation of targets associated with apoptosis. For information on the gene set used for comparison, see Liberzon et al. Cell Systems, 1: 417-425 (2015).

**Figure 20** is an image of the results obtained from a Western blot indicating that treatment with PFI-63 is associated with an increase in MYOG (a transcriptional activator that plays a role in muscle differentiation) in RH4 cells.

**Figure 21** is an image of the results obtained from a Western blot indicating that cleaved PARP (associated with apoptosis) increases in the presence of PFI-63 in RH4 cells.

**Figure 22** depicts the results of a CRISPR/Cas9 genome-scale loss of function time course screen in RH30.19luc cells demonstrating the gene sets that are negatively enriched in the presence of PFI-63 compared to DMSO (no drug). This indicates that inactivation of the genes contained within the gene sets by the single guide RNAs causes selective depletion of the cells from the pool of cells in the presence of PFI-63; i.e., the genes (including targets of histone lysine demethylases) were synthetically lethal with PFI-63.

**Figure 23** depicts the results of a gene set enrichment assay (GSEA) investigating the cellular effects of PFI-63. The results indicate that RH4 cells treated with PFI-63 experience upregulation of targets that are also upregulated by JIB-04, a known multi-histone lysine demethylase inhibitor. For information on the gene set used for comparison, see Wang et al., Nat. Commun., 4: 2035 (2013).

**Figure 24** depicts the results of a gene set enrichment assay (GSEA) investigating the cellular effects of PFI-63. The results indicate that RH4 cells treated with PFI-63 experience upregulation of targets that are also upregulated by GSK-J4, another known multi-histone lysine demethylase inhibitor. For information on the gene set used for comparison, see Hookway, E., The Role of the Lysine Demethylases KDM5 and KDM6 in Bone Malignancies, Ph.D. Thesis, University of Oxford, 2016.

**Figure 25** is an image depicting the results obtained from a Western blot showing a global increase in H3K27me3 (a repressive epigenetic modification to lysine 27 of the histone H3 protein), H3K4me3 (an activating epigenetic modification to lysine 4 of the histone H3 protein), and H3K9me3 (a repressive epigenetic modification to lysine 9 of the histone H3 protein) in cells treated with PFI-63.

**Figure 26** is a diagram illustrating the activating and repressive methylation sites on Histone H3.

**Figure 27** depicts the results of computer based predictions identifying numerous proteins, including several KDMs as potential targets of PFI-63.

**Figure 28** is a computer generated image depicting the predicted docking configuration of PFI-63 in the catalytic domain of KDM3B. For information on the crystal structure of the catalytic domain of KDM3B/JMJD1B used for the simulations, see Vollmar et al., DOI: 10.2210/pdb4C8D/pdb (initial deposition on September 30, 2013, with latest revision on January 24, 2018).

**Figure 29** is a computer generated image depicting the predicted docking configuration of PFI-63 in the catalytic domain of KDM3B.

**Figure 30** is a computer generated image depicting the predicted docking configuration of PFI-63 in the catalytic domain of KDM3B.

**Figure 31** is a computer generated image depicting an overlay of the catalytic domains of various histone lysine demethylases illustrating the similarities in structure (see Hoffmann et al., Mol. Oncol., 6: 683-703 (2012)).

**Figure 32** depicts an exemplary dose response curve for PFI-90 in RH4 cells at the 72 hour time point. The observed $IC_{50}$ is 812 nM.

**Figure 33** depicts an exemplary dose response curve for PFI-90 in RH30 cells at the 72 hour time point. The observed $IC_{50}$ is 3200 nM.

**Figure 34** depicts the results of a gene set enrichment assay (GSEA) investigating the cellular effects of PFI-90. The results indicate that targets of the PAX3-FOXO1 fusion transcription factor are downregulated in RH4 cells in the presence of PFI-90. For information on the gene set used for comparison, see Davicioni et al., Cancer Res., 66:6936-6946, 2006.

**Figure 35** depicts the results of a Gene Set Enrichment Assay investigating the cellular effects of PFI-90. The results indicate that targets of the PAX3-FOXO1 fusion transcription factor are downregulated in RH4 cells in the presence of PFI-90. For information on the gene set used for comparison, see Cao et al., Cancer Res., 70: 6497-6508 (2010).

**Figure 36** depicts the results of a gene set enrichment assay (GSEA) investigating the cellular effects of PFI-90. The results indicate that targets of the PAX3-FOXO1 fusion transcription factor are downregulated in RH4 cells in the presence of PFI-90. For information on the gene set used for comparison, see Gryder et al., Cancer Discov., 7: 884-899 (2017).

**Figure 37** depicts the results of a gene set enrichment assay (GSEA) investigating the cellular effects of PFI-90. The results indicate that genes that are induced upon KDM3B suppression are upregulated in RH4 cells treated with PFI-90. For information on the gene set used for comparison, see Kuroki et al., Stem Cell Reports, 10: 1340-1354

(2018).

**Figure 38** depicts the results of a gene set enrichment assay (GSEA) investigating the cellular effects of PFI-90. The results indicate that genes that are induced upon KDM3B suppression are upregulated in RH4 cells treated with PFI-90. For information on the gene set used for comparison, see An et al., Biochem. Biophys. Res. Commun., 508: 576-582 (2019).

**Figure 39** depicts the results of a gene set enrichment assay (GSEA) investigating the cellular effects of PFI-90. The results indicate that RH4 cells treated with PFI-90 also experience upregulation of targets associated with myogenic differentiation. For information on the gene set used for comparison, see Liberzon et al. Cell Systems, 1: 417-425 (2015).

**Figure 40** depicts the results of a gene set enrichment assay (GSEA) investigating the cellular effects of PFI-90. The results indicate that RH4 cells treated with PFI-90 also experience upregulation of targets associated with apoptosis. For information on the gene set used for comparison, see Liberzon et al. Cell Systems, 1: 417-425 (2015).

**Figure 41** is an image of the results obtained from a Western blot indicating that as the concentration of PFI-90 increases, the methylation of histone H3 also increases.

**Figure 42** is a graphical depiction of the Western blot results indicating that as the concentration of PFI-90 increases, the methylation of histone H3 also increases.

**Figure 43** is a graphical depiction of the results of an investigation into whether PFI-63 induces apoptosis in RH4 and SCMC fusion positive rhabdomyosarcoma cells. As can be seen in the Figure, in both RH4 and SCMC cells, treatment with PFI-63 leads to increased caspase 3, which is associated with apoptosis.

**Figure 44** is a graphical depiction of the results of an investigation into whether PFI-90 induces apoptosis in RH4 and SCMC cells. As can be seen in the Figure, in both RH4 and SCMC cells, treatment with PFI-90 leads to increased caspase 3, which is associated with apoptosis.

**Figure 45** is a graphical depiction of the results of an investigation into whether PFI-90 and PFI-63 induce apoptosis in RH4 cells via annexin V staining. Figure 45 depicts the results obtained for the DMSO control.

**Figure 46** is a graphical depiction of the results of an investigation into whether PFI-90 and PFI-63 induce apoptosis in RH4 cells via annexin V staining. Figure 46 depicts the results obtained for PFI-90, in which an increase in cell death (relative to DMSO control) is observed.

**Figure 47** is a graphical depiction of the results of an investigation into whether PFI-90 and PFI-63 induce apoptosis in RH4 cells via annexin V staining. Figure 47 depicts the results obtained for PFI-63, in which an increase in cell death (relative to DMSO control) is observed.

**Figure 48** is an image depicting the results obtained from a Western blot that indicates that in RH4 cells treated with 3 $\mu$M PFI-90, cleaved PARP (associated with apoptosis) increases at 24 hours.

**Figure 49** is an image depicting the results obtained from a Western blot that indicates that in RH4 cells treated with 3 $\mu$M PFI-90, PAX3-FOXO1 decreases at 24 hours. This coincides with the increase of cleaved PARP depicted in Figure 48.

**Figure 50** is a graphical depiction of the results of screening PFI-90 against various leukemia cell lines in the NCI-60 panel. The results indicate that PFI-90 inhibits cell growth.

**Figure 51** is a graphical depiction of the results of screening PFI-90 against various non-small cell lung cancer cell lines in the NCI-60 panel. The results indicate that PFI-90 inhibits cell growth.

**Figure 52** is a graphical depiction of the results of screening PFI-90 against various CNS cancer cell lines in the NCI-60 panel. The results indicate that PFI-90 inhibits cell growth.

**Figure 53** is a graphical depiction of the results of screening PFI-90 against various melanoma cell lines in the NCI-60 panel. The results indicate that PFI-90 inhibits cell growth.

**Figure 54** is a graphical depiction of the results of screening PFI-90 against various prostate cancer cell lines in the NCI-60 panel. The results indicate that PFI-90 inhibits cell growth.

**Figure 55** is a graphical depiction of the results of screening PFI-90 against various colon cancer cell lines in the NCI-60 panel. The results indicate that PFI-90 inhibits cell growth.

**Figure 56** is a graphical depiction of the results of screening PFI-90 against various ovarian cancer cell lines in the NCI-60 panel. The results indicate that PFI-90 inhibits cell growth.

**Figure 57** is a graphical depiction of the results of screening PFI-90 against various breast cancer cell lines in the NCI-60 panel. The results indicate that PFI-90 inhibits cell growth.

**Figure 58** is a graphical depiction of the results of screening PFI-90 against various renal cancer cell lines in the NCI-60 panel. The results indicate that PFI-90 inhibits cell growth and induces cell death.

**Figure 59** depicts an exemplary dose response curve for PFI-63 in OSA-CL osteosarcoma cells at the 72 hour time point. The observed $IC_{50}$ is 7570 nM.

**Figure 60** depicts an exemplary dose response curve for PFI-90 in OSA-CL cells at the 72 hour time point. The observed $IC_{50}$ is 1895 nM.

**Figure 61** depicts an exemplary dose response curve for PFI-63 in TC-32 Ewing's sarcoma cells at the 72 hour time

point. The observed $IC_{50}$ is 1880 nM.

**Figure 62** depicts an exemplary dose response curve for PFI-90 in TC-32 cells at the 72 hour time point. The observed $IC_{50}$ is 1113 nM.

**Figure 63** depicts NMR peaks for PFI-90. All peaks are clearly resolved. The buffer used was 50mM d-Tris, 80uM $ZnSO_4$ $H_2O$, at pH 7.5. Samples contained 300 μM PFI-90, 100 μM 2-OG, 300 μM NMV, 10% $D_2O$, 5% D6-DMSO.

**Figure 64** depicts NMR peaks obtained for αketoglutarate (also called 2-oxoglutaric acid or "2-OG"). 2-OG peaks are easily resolved. The buffer was 50mM d-Tris, 80uM $ZnSO_4$ $H_2O$, at pH 7.5. Samples contained 300 μM PFI-90, 100 μM 2-OG (where present), 300 μM NMV, 10% $D_2O$, 5% D6-DMSO.

**Figure 65** depicts results from an NMR analysis of PFI-90. As can be seen, chemical shift perturbation upon addition of a truncated KDM3B catalytic domain are not influenced by the presence of 2-OG. The buffer used was 50mM d-Tris, 80uM $ZnSO_4$ $H_2O$, at pH 7.5. Samples contained 300 uM PFI-90, 100 uM 2-OG (where present), 300 uM NMV, 10% D2O, 5% D6-DMSO and 12 uM truncated KDM3B catalytic domain (where present). Samples were normalized against DMSO.

**Figure 66** depicts results from a WaterLOGSY experiment. Results show that PFI-90 binds the truncated KDM3B catalytic domain at 300 μM in the presence and in the absence of 2-OG. 2-OG does not bind at 100 μM. Samples were normalized against NMV

**Figure 67** depicts results from a CPMG experiment. Strong binding of PFI-90 to the truncated KDM3B catalytic domain is observed in the presence and absence of 2-OG. 2-OG does not bind at 100 μM. Complete/near complete height attenuation is observed in 5 out of 9 PFI-90 peaks, 70% attenuation is observed in 1 of 9 peaks and 50% attenuation is observed in 3 of 9 peaks. Samples normalized against NMV. The NMV reference peak is normalized to 1000. Minimal baseline correction was applied to the CPMG spectra (Whitaker smoother filter = 58, smooth factor = 16384).

## DETAILED DESCRIPTION OF THE INVENTION

### *Histone Demethylase Inhibitors*

The inventors have discovered that certain compounds function as histone demethylase inhibitors. One of the compounds is defined by the structure:

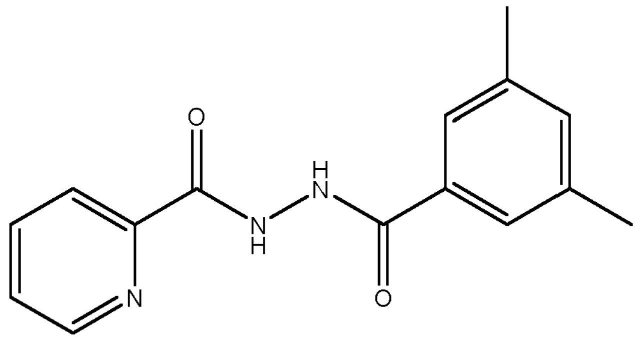

or a pharmaceutically acceptable salt thereof. This compound is referred to herein as "PFI-63" or "UPCMLD0ENAT5834780" (Molport Catalog No. MolPort-004-589-395). Another of the compounds is defined by the structure:

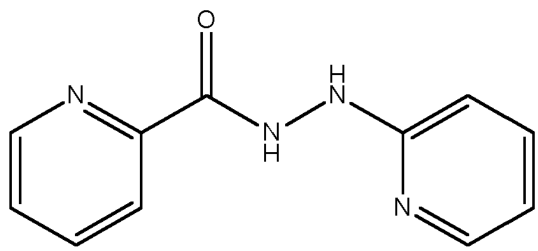

or a pharmaceutically acceptable salt thereof. This compound is referred to herein as PFI-90 (Enamine Catalog No. Z1610556569). The inventors have discovered that PFI-63 and PFI-90, as well as pharmaceutically acceptable salts thereof, can be used to treat cancer in mammals by administering one or more of the compounds to a mammal that has cancer and to inhibiting a histone demethylase in cells by contacting the cells with one or more of the compounds.

As used herein, the term "inhibit", "inhibiting", or "inhibition" means that a biological response is decreased in the presence of a compound when compared to biological response that occurs in the absence of the compound. For example, in an embodiment, a compound of the disclosure is administered to a cell *in vitro* such that the biological activity of a histone demethylase in the cell is reduced when compared to the activity of the histone demethylase in the absence of the compound (i.e., the histone demethylase is "inhibited"). The activity of histone demethylases in the presence and absence of a histone demethylase inhibitor may be determined using conventional medical, diagnostic and laboratory techniques known to those skilled in the art.

Those skilled in the art will be familiar with various histone demethylases. In some embodiments, the inhibited histone demethylase may be a lysine specific histone demethylase (also called "histone lysine demethylase" or "KDM"). Various lysine-specific histone demethylases are known in the art. For example, the KDM1 family includes the histone demethylases KDM1A and KDM1B. KDM1A acts on mono- and dimethylated H3K4 and H3K9 (the $4^{th}$ and $9^{th}$ lysine residues of histone subunit 3, respectively). KDM1B acts on only mono- and dimethylated H3K4. As another example, the KDM2 family includes KDM2A and KDM2B. KDM2A (also referred to as JHDM1A/FBXL11) can act on mono- and dimethylated H3K36 and trimethylated H3K4. KDM2B (also referred to as JHDM1B/FBXL10) acts only on mono- and dimethylated H3K36. As another example, the KDM3 family includes KDM3A, KDM3B, and JMJD1C. KDM3A (also referred to as JHDM2A/JMJD1A/TSGA) can act on mono- and dimethylated H3K9. KDM3B (also referred to as JHDM2B/JMJD1B) can also act on mono- and dimethylated H3K9 (see Brauchle et al., PLoS ONE, 8: e60549 (2013)). The substrates for JMJD1C (also referred to as JHDM2C/TRIP8) have not been elucidated.

In some embodiments the histone demethylase inhibitors inhibit one or more of KDM3A, KDM3B, KDM5A, KDM5B, KDM4B, and KDM6B. In certain embodiments, the histone demethylase inhibitors inhibit the function of KDM3B. KDM3B is known in the art to epigenetically control colorectal cancer through Wnt signaling (see Li et al., Nat. Commun., 8: 15146 (2017) [DOI: 10.1038/ncomms15146]). KDM3B also transcriptionally activates lmo2 in leukemias (see Kim et al., Mol. Cell. Biol., 32: 2917-2933 (2012)). KDM3B is also upregulated in taxane-platin-chemoresistant non-small cell lung cancer and confers hypersensitivity to histone demethylase inhibitors (Dalvi et al., Cell Reports, 19: 1669-1684 (2017)). KDM3B also regulates the transcriptional network of cell-cycle genes in hepatocarcinoma cells (An et al., Biochem. Biophys. Res. Commun., 508: 576-582 (2019)).

In certain embodiments, the inhibited histone demethylases are associated with the function of an oncogene, e.g., an oncogenic transcription factor. For example, in certain embodiments, the inhibited histone demethylases are associated with the function of oncogenic PAX3/7-FOXO1 fusion transcription factors in fusion positive rhabdomyosarcoma. PAX3/7-FOXO1 fusion transcription factors are the result of chromosomal translocations and are associated with the majority of cases of alveolar rhabdomyosarcoma. In some embodiments, the histone demethylase inhibitors of the present disclosure disrupt the oncogenic activity of PAX3/7-FOXO1. In other embodiments, the inhibited histone demethylase(s) are associated with the function of the oncogenic EWS/FLI fusion transcription factor, which may be present in Ewing’s sarcoma. In some embodiments, the histone demethylase inhibitors of the disclosure disrupt the oncogenic activity of EWS/FLI.

The histone demethylase inhibitor may target multiple histone demethylases. For example multiple KDMs may be inhibited. Those skilled in the art will recognize such activity as desirable. For example, by inhibiting multiple KDMs in rhabdomyosarcoma cells, a compound may upregulate myogenesis and apoptosis, while at the same time down regulating PAX3-FOXO1 oncogenic targets. As another example, the ability to target multiple KDMs may reduce the frequency of acquired drug resistance arising as a result of acquisition of mutations in a single therapeutic KDM target.

*Use in Methods of Treatment*

The disclosure further provides but does not claim a method of treating cancer in a mammal comprising, consisting essentially of or consisting of administering to the mammal a therapeutically effective amount of one or both of the compounds described herein or pharmaceutically acceptable salts thereof.

The invention provides a pharmaceutical composition comprising any of the compounds described herein or a pharmaceutically acceptable salt thereof for use in treating cancer in a mammal. The disclosure further provides but does not claim the use of a compound or a pharmaceutically acceptable salt thereof in the manufacture of a medicament for treating cancer in a mammal, wherein the compound is any of the compounds described herein or a pharmaceutically acceptable salt thereof. The medicament typically is a pharmaceutical composition as described herein.

The term "mammal" refers to any mammal including, but not limited to, mammals of the order Logomorpha, such as rabbits; the order Carnivora, including Felines (cats) and Canines (dogs); the order Artiodactyla, including Bovines (cows) and Swines (pigs); or of the order Perssodactyla, including Equines (horses). Mammals include non-human primates, e.g., of the order Primates, Ceboids, or Simoids (monkeys) or of the order Anthropoids (humans and apes). In some embodiments, the mammal may be a mammal of the order Rodentia, such as mice and hamsters. In still other embodiments, the mammal is a non-human primate or a human. In some embodiments, the mammal is a human. In some embodiments, the mammal is a human infant, child or adolescent.

Cancers include, without limitation, leukemias (e.g., acute leukemia, acute lymphocytic leukemia, acute myelocytic (myeloid) leukemia, acute myeloblastic leukemia, acute promyelocytic leukemia, acute myelomonocytic leukemia, acute monocytic leukemia, acute erythroleukemia, chronic leukemia, chronic myelocytic (myeloid) leukemia, and chronic lymphocytic leukemia), polycythemia vera, lymphoma (e.g., Hodgkin's disease or non-Hodgkin's disease), Waldenstrom's macroglobulinemia, multiple myeloma, heavy chain disease, and solid tumors such as sarcomas and carcinomas (e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, (malignant) mesothelioma, Ewing's sarcoma, leiomyosarcoma, rhabdomyosarcoma (including alveolar), colon carcinoma (colon cancer), pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct/intrahepatic bile duct carcinoma, choriocarcinoma, seminoma, embryonal carcinoma, Wilm's tumor, cervical cancer, uterine cancer, testicular cancer, lung carcinoma (lung cancer), small cell lung carcinoma, bladder carcinoma (urinary bladder cancer), epithelial carcinoma, glioma, astrocytoma, medulloblastoma, craniopharyngioma, ependymoma, pinealoma, hemangioblastoma, acoustic neuroma, oligodendroglioma, schwannoma, meningioma, melanoma, neuroblastoma, and retinoblastoma). The cancers may include bone cancer, brain cancer, cancer of the anus, anal canal, or anorectum, cancer of the eye, cancer of the joints, cancer of the neck, gallbladder, or pleura, cancer of the nose, nasal cavity, or middle ear, cancer of the oral cavity, cancer of the vulva, esophageal cancer, gastrointestinal carcinoid tumor, hypopharynx cancer, kidney cancer, larynx cancer, liver cancer, nasopharynx cancer, non-small cell lung cancer, peritoneum, omentum, and mesentery cancer, pharynx cancer, rectal cancer, renal cancer, small intestine cancer, soft tissue cancer, stomach cancer, thyroid cancer, and ureter cancer.

The cancer in the treatment of which the pharmaceutical composition is for use in according to the invention may be responsive to inhibition of a histone demethylase or may be caused by or associated with the function of one or more histone demethylases. In certain embodiments, the cancer is a pediatric cancer, i.e., a form of cancer that occurs primarily in children.

In certain embodiments, the cancer is a transcription driven cancer. As used herein, the term "transcription driven cancer" refers to a cancer in which the primary oncogenic driver is one or more transcription factors, or a chromatin remodeler that have undergone mutations (e.g., translocations) that disrupt the activity of a gene, either by increasing or reducing activity that leads to epigenetic reprogramming of the cells and oncogenic activity. A variety of transcription driven cancers are known to those skilled in the art. Such cancers include, without limitation, anaplastic large cell lymphomas, acute myeloid leukemias, acute lymphoblastic leukemias, acute promyelocytic leukemias, chronic myelogenous leukemias, rhabdomyosarcomas, angiomatoid fibrous histiocytomas, alveolar soft part sarcomas, Ewing's sarcomas, myxoid liposarcomas, myxoid chondrosarcomas, melanomas, liposarcomas, Synovial sarcomas, endometrial stromal sarcomas, Small round-cell sarcomas, and ependymomas.

In some embodiments, the cancer is a transcription driven cancer selected from the group consisting of fusion positive rhabdomyosarcoma and Ewing's sarcoma. For example, in some embodiments, the cancer can be caused by or associated with the function of the PAX3-FOXO1 fusion transcription factor. Such cancers include, e.g., fusion-positive rhabdomyosarcoma.

In some embodiments, the cancer is selected from the group consisting of rhabdomyosarcoma, Ewing's sarcoma, colorectal cancer, ovarian cancer, prostate cancer, CNS cancer, melanoma, breast cancer, leukemia, non-small cell lung cancer, renal cancer, and osteosarcoma.

The terms "treat" and "prevent," as used herein, do not necessarily imply 100% or complete treatment or prevention. Rather, there are varying degrees of treatment or prevention of which one of ordinary skill in the art recognizes as having a potential benefit or therapeutic effect. In this respect, the methods in which the pharmaceutical composition is for use can provide any amount of any level of treatment or prevention in a mammal. Furthermore, the treatment or prevention provided by the method in which the pharmaceutical composition is for use can include treatment or prevention of one or more conditions or symptoms of the diseases described herein being treated or prevented. Also, for purposes herein, "prevention" can encompass delaying the onset of the disease, or a symptom or condition thereof.

*Pharmaceutical Compositions*

The invention provides a pharmaceutical composition, comprising, consisting essentially of or consisting of a compound or salt of PFI-63 and/or PFI-90 as described above and a pharmaceutically acceptable carrier for use in the treatment of cancer.

The phrase "pharmaceutically acceptable salt" is intended to include nontoxic salts synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Generally, nonaqueous media such as ether, ethyl

acetate, ethanol, isopropanol, or acetonitrile are preferred. Lists of suitable salts are found in Remington's Pharmaceutical Sciences, 18th ed., Mack Publishing Company, Easton, PA, 1990, p. 1445, and Journal of Pharmaceutical Science, 66: 2-19 (1977).

Suitable bases include inorganic bases such as alkali and alkaline earth metal bases, e.g., those containing metallic cations such as sodium, potassium, magnesium, calcium and the like. Non-limiting examples of suitable bases include sodium hydroxide, potassium hydroxide, sodium carbonate, and potassium carbonate. Suitable acids include inorganic acids such as hydrochloric acid, hydrobromic acid, hydroiodic acid, sulfuric acid, phosphoric acid, and the like, and organic acids such as p-toluenesulfonic, methanesulfonic acid, benzenesulfonic acid, oxalic acid, p-bromophenylsulfonic acid, carbonic acid, succinic acid, citric acid, benzoic acid, acetic acid, maleic acid, tartaric acid, fatty acids, long chain fatty acids, and the like. Preferred pharmaceutically acceptable salts of disclosed compounds having an acidic moiety include sodium and potassium salts.

It should be recognized that the particular counterion forming a part of any salt of this disclosure is usually not of a critical nature, so long as the salt as a whole is pharmacologically acceptable and as long as the counterion does not contribute undesired qualities to the salt as a whole.

The above compounds and salts may form solvates, or exist in a substantially uncomplexed form, such as the anhydrous form. As used herein, the term "solvate" refers to a molecular complex wherein the solvent molecule, such as the crystallizing solvent, is incorporated into the crystal lattice. When the solvent incorporated in the solvate is water, the molecular complex is called a hydrate. Pharmaceutically acceptable solvates include hydrates, alcoholates such as methanolates and ethanolates, acetonitrilates and the like. These compounds can also exist in polymorphic forms.

The invention contemplates embodiments in which a compound having a chiral center is a substantially pure enantiomer thereof, a racemic mixture thereof, or a mixture containing any proportion of the two enantiomers thereof. The invention also contemplates all stereoisomers and diastereoisomers of the compounds described herein.

The invention is further directed to a pharmaceutical composition comprising, consisting essentially of or consisting of a pharmaceutically acceptable carrier and at least one compound or salt described herein for use in the treatment of cancer.

It is preferred that the pharmaceutically acceptable carrier be one that is chemically inert to the active compound(s) in the composition and one that has no detrimental side effects or toxicity under the conditions of use.

The choice of carrier will be determined in part by the particular compound of the disclosure chosen, as well as by the particular method used to administer the composition. Accordingly, there is a wide variety of suitable formulations of the pharmaceutical composition for use of the invention.

*Modes of Administration*

One skilled in the art will appreciate that various suitable methods of utilizing a compound and administering it to a mammal for the treatment of cancer, which would be useful in the method in which the pharmaceutical composition is for use, are available. Although more than one route can be used to administer a particular compound, a particular route can provide a more immediate and more effective reaction than another route. Accordingly, the described methods in which the pharmaceutical composition is for use are merely exemplary and are in no way limiting.

The dose administered to a mammal should be sufficient to effect the desired response. Such responses include, e.g., reversal or prevention of the negative or deleterious effects of the cancer for which treatment is desired. One skilled in the art will recognize that dosage will depend upon a variety of factors, including the species, age, condition, and body weight of the mammal, as well as the source of the cancer, the particular type of the cancer, and the extent of cancer in the mammal. The size of the dose will also be determined by the route, timing and frequency of administration as well as the existence, nature, and extent of any adverse side-effects that might accompany the administration of a particular compound and the desired physiological effect. It will be appreciated by one of skill in the art that various conditions or disease states may require prolonged treatment involving multiple administrations.

The pharmaceutical composition can be administered parenterally, e.g., intravenously, subcutaneously, intradermally, intramuscularly or intratumorally. Thus, in some embodiments, the invention provides compositions for use for parenteral administration that comprise a solution of the disclosed compound or salt dissolved or suspended in an acceptable carrier suitable for parenteral administration, including aqueous and non-aqueous isotonic sterile injection solutions.

Overall, the requirements for effective pharmaceutical carriers for parenteral compositions are well known to those of ordinary skill in the art. See, e.g., Banker and Chalmers, eds., Pharmaceutics and Pharmacy Practice, J. B. Lippincott Company, Philadelphia, pp. 238-250 (1982), and Toissel, ASHP Handbook on Injectable Drugs, 4th ed., pp. 622-630 (1986). Such solutions can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The compound or salt of the disclosure may be administered in a physiologically acceptable diluent in a pharmaceutical carrier, such as a sterile liquid or mixture of

liquids, including water, saline, aqueous dextrose and related sugar solutions, an alcohol, such as ethanol, isopropanol, or hexadecyl alcohol, glycols, such as propylene glycol or polyethylene glycol, dimethylsulfoxide, glycerol ketals, such as 2,2-dimethyl-1,3-dioxolane-4-methanol, ethers, such as poly(ethyleneglycol) 400, an oil, a fatty acid, a fatty acid ester or glyceride, or an acetylated fatty acid glyceride with or without the addition of a pharmaceutically acceptable surfactant, such as a soap or a detergent, suspending agent, such as pectin, carbomers, methylcellulose, hydroxypropylmethylcellulose, or carboxymethylcellulose, or emulsifying agents and other pharmaceutical adjuvants.

Oils useful in parenteral formulations include petroleum, animal, vegetable, or synthetic oils. Specific examples of oils useful in such formulations include peanut, soybean, sesame, cottonseed, corn, olive, petrolatum, and mineral. Suitable fatty acids for use in parenteral formulations include oleic acid, stearic acid, and isostearic acid. Ethyl oleate and isopropyl myristate are examples of suitable fatty acid esters.

Suitable soaps for use in parenteral formulations include fatty alkali metal, ammonium, and triethanolamine salts, and suitable detergents include (a) cationic detergents such as, for example, dimethyl dialkyl ammonium halides, and alkyl pyridinium halides, (b) anionic detergents such as, for example, alkyl, aryl, and olefin sulfonates, alkyl, olefin, ether, and monoglyceride sulfates, and sulfosuccinates, (c) nonionic detergents such as, for example, fatty amine oxides, fatty acid alkanolamides, and polyoxyethylenepolypropylene copolymers, (d) amphoteric detergents such as, for example, alkyl-beta-aminopropionates, and 2-alkyl-imidazoline quaternary ammonium salts, and (e) mixtures thereof.

The parenteral formulations can contain preservatives and buffers. In order to minimize or eliminate irritation at the site of injection, such compositions may contain one or more nonionic surfactants having a hydrophile-lipophile balance (HLB) of from about 12 to about 17. The quantity of surfactant in such formulations will typically range from about 5 to about 15% by weight. Suitable surfactants include polyethylene sorbitan fatty acid esters, such as sorbitan monooleate and the high molecular weight adducts of ethylene oxide with a hydrophobic base, formed by the condensation of propylene oxide with propylene glycol. The parenteral formulations can be presented in unit-dose or multi-dose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets of the kind previously described.

Formulations suitable for oral administration can consist of (a) liquid solutions, such as a therapeutically effective amount of the disclosed compound dissolved in diluents, such as water, saline, or orange juice, (b) capsules, sachets, tablets, lozenges, and troches, each containing a predetermined amount of the active ingredient, as solids or granules, (c) powders, (d) suspensions in an appropriate liquid, and (e) suitable emulsions. Liquid formulations may include diluents, such as water and alcohols, for example, ethanol, benzyl alcohol, and the polyethylene alcohols, either with or without the addition of a pharmaceutically acceptable surfactant, suspending agent, or emulsifying agent. Capsule forms can be of the ordinary hard- or soft-shelled gelatin type containing, for example, surfactants, lubricants, and inert fillers, such as lactose, sucrose, calcium phosphate, and corn starch. Tablet forms can include one or more of lactose, sucrose, mannitol, corn starch, potato starch, alginic acid, microcrystalline cellulose, acacia, gelatin, guar gum, colloidal silicon dioxide, croscarmellose sodium, talc, magnesium stearate, calcium stearate, zinc stearate, stearic acid, and other excipients, colorants, diluents, buffering agents, disintegrating agents, moistening agents, preservatives, flavoring agents, and pharmacologically compatible excipients. Lozenge forms can comprise the active ingredient in a flavor, usually sucrose and acacia or tragacanth, as well as pastilles comprising, consisting essentially of or consisting of the active ingredient in an inert base, such as gelatin and glycerin, or sucrose and acacia, emulsions, gels, and the like containing, in addition to the active ingredient, such excipients as are known in the art.

Suitable doses and dosage regimens can be determined by conventional range-finding techniques known to those of ordinary skill in the art. Generally, treatment is initiated with smaller dosages that are less than the optimum dose of the compound. Thereafter, the dosage is increased by small increments until the optimum effect under the circumstances is reached. The method in which the pharmaceutical composition is for use typically will involve the administration of about 0.001 to about 300 mg of one or more of the compounds described above per kg body weight of the individual. The administration can involve about 0.001 mg, about 0.01 mg, about 0.1 mg, about 1 mg, about 5 mg, about 10 mg, about 20 mg, about 50 mg, about 100 mg, about 200 mg, or about 300 mg or more of one or more of the compounds described above per kg body weight of the individual. Alternatively, or in addition, the administration can involve about 300 mg, about 200 mg, about 100 mg, about 50 mg, about 20 mg, about 10 mg, about 5 mg, about 1 mg, about 0.1 mg, about 0.01 mg, or about 0.001 mg or less of one or more of the compounds described above per kg body weight of the individual. Thus, the administration can be bounded by any two of the aforementioned endpoints. For example, the administration can be about 0.001 mg to about 200 mg, about 0.001 mg to about 1 mg, about 0.01 mg to about 50 mg, about 0.1 mg to about 20 mg, about 1 mg to about 10 mg, about 1 mg to about 20 mg, about 10 mg to about 50 mg, or any other combination of endpoints, of one or more of the compounds described above per kg body weight of the individual.

*Additional Methods*

Embodiments of the current invention also include methods for inhibiting histone demethylase(s) in a cell *in vitro* comprising, consisting essentially of or consisting of contacting the cell with one or more of PCFI-63, PFI-90, and pharmaceutically acceptable salts thereof.

The cell may be any suitable cell. Such cells include, for example, cells derived from mammalian sources. In some embodiments, the cells are human.

The cell may be derived from any tissue type. For example, such cell types include, without limitation, epithelial, endothelial, smooth-muscle, neural, cardiac, and immune cells. An illustrative list of eukaryotic cell types that can be used includes stem cells; pluripotent stem cells; primary cells; fibroblasts; motile cells, ciliated cells; cancer cells including cervix, ovary, colorectal breast, prostate, bladder, pancreas, kidney, lung, salivary gland, testis, cecum, liver, colon, mammary gland, vulva, stomach, pleura, bladder, brain, bone, bone marrow, lymph, eye, connective tissue, pituitary gland, muscle, heart, spleen, skin, uterus, endometrium cells, and epithelial cells; endothelial cells; blood cells; neural cells; secretory cells including adrenal gland cells; contractile cells including smooth muscle cells and skeletal muscle cells; hepatocytes; adipocytes; lymphocytes; macrophages; T-cells; B- cells; dendritic cells; neurons; chondrocytes; and stem cells including embryonic, fetal, amniotic, adult and induced pluripotent stem cells.

In some embodiments, the cell may be a cancer cell. For example, the cells may be derived from, without limitation, leukemias (e.g., acute leukemia, acute lymphocytic leukemia, acute myelocytic (myeloid) leukemia, acute myeloblastic leukemia, acute promyelocytic leukemia, acute myelomonocytic leukemia, acute monocytic leukemia, acute erythroleukemia, chronic leukemia, chronic myelocytic (myeloid) leukemia, and chronic lymphocytic leukemia), polycythemia vera, lymphoma (e.g., Hodgkin's disease and non-Hodgkin's disease), Waldenstrom's macroglobulinemia, multiple myeloma, heavy chain disease, and solid tumors such as sarcomas and carcinomas (e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, (malignant) mesothelioma, Ewing's sarcoma, leiomyosarcoma, rhabdomyosarcoma (including alveolar), colon carcinoma (colon cancer), pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct/intrahepatic bile duct carcinoma, choriocarcinoma, seminoma, embryonal carcinoma, Wilm's tumor, cervical cancer, uterine cancer, testicular cancer, lung carcinoma (lung cancer), small cell lung carcinoma, bladder carcinoma (urinary bladder cancer), epithelial carcinoma, glioma, astrocytoma, medulloblastoma, craniopharyngioma, ependymoma, pinealoma, hemangioblastoma, acoustic neuroma, oligodendroglioma, schwannoma, meningioma, melanoma, neuroblastoma, and retinoblastoma). The cancers may include bone cancer, brain cancer, cancer of the anus, anal canal, or anorectum, cancer of the eye, cancer of the joints, cancer of the neck, gallbladder, or pleura, cancer of the nose, nasal cavity, or middle ear, cancer of the oral cavity, cancer of the vulva, esophageal cancer, gastrointestinal carcinoid tumor, hypopharynx cancer, kidney cancer, larynx cancer, liver cancer, nasopharynx cancer, non-small cell lung cancer, peritoneum, omentum, and mesentery cancer, pharynx cancer, rectal cancer, renal cancer, small intestine cancer, soft tissue cancer, stomach cancer, thyroid cancer, and ureter cancer. In certain embodiments, the cells are rhabdomyosarcoma cells or Ewing's sarcoma cells.

The cells may be cancer cells derived from cancers responsive to inhibition of a histone demethylase or cancers caused by or associated with the function of one or more histone demethylases. In certain embodiments, the cells are derived from a pediatric cancer, i.e., a form of cancer that occurs primarily in children.

In certain embodiments, the cell is a cancer cell derived from a transcription driven cancer. In some embodiments, the cell is derived from a transcription driven cancer selected from the group consisting of fusion positive rhabdomyosarcoma and Ewing's sarcoma. For example, in some embodiments, the cancer can be caused by or associated with the function of the PAX3-FOXO1 fusion transcription factor. Such cancers include, e.g., fusion-positive rhabdomyosarcoma.

In some embodiments, the cell is a cancer cell derived from a cancer selected from the group consisting of rhabdomyosarcoma, Ewing's sarcoma, colorectal cancer, ovarian cancer, prostate cancer, CNS cancer, melanoma, breast cancer, leukemia, non-small cell lung cancer, renal cancer, and osteosarcoma.

The methods are carried out *in vitro.* The cells may have been obtained, stored and utilized in any suitable matter according to procedures known in the art. In some embodiments, the cells may have been harvested from a mammal, such as, for example, harvested from a tumor present in the mammal. In some embodiments, the cells may have been obtained via cell culture. Cell cultures suitable for the invention include, without limitation, continuous cell lines (e.g., with an immortal phenotype), primary cell cultures, finite cell lines (e.g., non-transformed cells), and any other cell population maintained in vitro, including oocytes and embryos.

The cell may be contacted with the compounds of the disclosure via any suitable method. For example, suitable methods may include, without limitation, the delivery of the therapeutic compound directly to the cell, delivery of the compound to the vicinity of the cell, delivery of the compound directly to a tumor, delivery of the compound to the vicinity of a tumor, administration of the compound to a patient that has a tumor, or any combination thereof.

## EXAMPLES

The following examples further illustrate the invention but, of course, should not be construed as in any way limiting its scope.

## EXAMPLE 1

This example describes an assay used to identify compounds that disrupt the function of the PAX3-FOXO1 fusion transcription factor in RH4 fusion-positive rhabdomyosarcoma cells.

The screen was performed using two derivatives of RH4 FP-RMS cells:

(1) "ALK-Luc cells" - RH4 cells expressing a pGreenFire green fluorescent protein (GFP)-luciferase reporter (SEQ ID NO: 1) controlled by the PAX3-FOXO1-dependent intronic ALK super enhancer (for reporting on PAX3-FOXO1 activity) (Figures 1 and 2); and

(2) "CMV-Luc cells" - control RH4 cells in which the reporter is transcribed from a constitutively active cytomegalovirus (CMV) promoter.

An Example of visualization via the ALK-Luc reporter is depicted in Figure 3.

The cells were enriched for reporter expression levels and used for assay development. A high throughput screening assay was developed after optimization of cell seeding density, length of incubation of cells prior to and post treatment with test compounds, and effect of passaging among other factors. The screen was designed to identify compounds that suppressed PAX3-FOXO1 activity without disrupting viability (at a short time points) or general transcription. The assay simultaneously monitors the function of the PAX3-FOXO1 super enhancer (also referred to as "ALK super enhancer"), general transcriptional activity as well as cell viability.

The assay was validated for sensitivity and reproducibility over three separate runs using 3000 randomly chosen screening compounds with high correlation (coefficient median R2 = 0.87, range R2 = 0.86-0.90).

The visual summary of the screening strategy is illustrated in Figure 4. ALK-Luc cells were seeded in both white-walled and white-bottomed 384-well plates (Perkin Elmer, Cat# 6007658) for luciferase assays and in clear 384-well plates (Perkin Elmer, Cat# 6007688) for viability assay (XTT reduction) by transferring 27 μL of cell suspensions (seeding 3000 cells per well) into each well. The plates were then transferred into an incubator for 18-20 h. The CMV-Luc cells were similarly seeded into white plates and incubated.

A pure compound library of 62,643 compounds consisting of synthetic and natural products described previously (Grohar et al., J. Natl. Cancer Inst., 103: 962-978 (2011)) was then used in a high throughput screening campaign to identify inhibitors of PAX3-FOXO1. Stock DMSO Solutions of these compounds were used to prepare 100 μM solutions in growth medium in 384-well plates also containing 16 wells each of a positive control (Actinomycin D, Sigma, Cat# A1410) and negative control (DMSO). Screening compounds along with the positive and negative controls were added to assay plates by transferring 3 μL of the prepared 100 μM dilutions using an automated liquid handler (Agilent, Bravo). Treated plates were incubated for 24 h. At the end of this period, treated plates were allowed to equilibrate to room temperature for 30 minutes. SteadyLite Plus luciferase assay reagent powder (PerkinElmer, Cat# 6066759) was reconstituted in its buffer in parallel and was also allowed to equilibrate to room temperature.

After transferring 30 μL of the luciferase assay reagent using a liquid handler, plates were further incubated at room temperature for 10 minutes. Finally, luminescence measurements were carried out using a multi-label microplate reader (BMG, Pherastar FSX) set in luminescence mode. Cell viability was assessed by adding XTT reagent (10 μL per well) in clear plates containing treated ALK-Luc cells. XTT treated plates were read using a plate reader in absorbance mode at 450 nm (PerkinElmer, Envision) after further incubation for 1 hour to allow cells to metabolize XTT to a colored formazan product. Luminescence and XTT absorbance values were normalized to the average of the negative controls on each plate and were calculated as a percentage of these controls. Performance of the screening assay was routinely monitored via calculation of Z-factor (Zhang et al., J. Biomol. Screen., 4: 67-73 (1999)) with Z' for all plates found to be ≥0.7.

A weighted mean of percent-control of the RH4 ALK-Luc (L), RH4 XTT (X), and RH4 CMV-Luc (C) values for each test compound was determined employing the formula:

$$\text{Weighted average} = (4L + 2C + X)/7$$

Compounds with weighted averages above 60 were identified as hits (outliers) after preparing a boxplot of weighted averages (see Figure 5). This resulted in the identification of 573 compounds as hits.

EXAMPLE 2

This Example describes hit confirmation and further screening of the identified compounds.

RH4 ALK-Luc and RH4 CMV-Luc cells were seeded into separate white-walled and bottomed 384-well plates and were incubated and treated with screen hits and epigenome compounds. The highest test concentration of these compounds (10-fold) were prepared from DMSO stock solutions via dilution in medium. Final DMSO concentration in assay wells was 0.2% or less. Ten-fold serial dilutions of test compounds were made using an automated liquid handler, and 3 μL of each dilution was added to each well of plates containing RH4 ALK-Luc and RH4 CMV-Luc cells in 27 μL medium. Each test concentration was assayed in quadruplicate. Corresponding DMSO dilutions were used as control. Treated plates were then read following the same procedure used for screening described above. Selective activity was confirmed for 64 compounds (see Figure 6).

An exemplary dose response curve for PFI-63 is presented as Figure 7.

EXAMPLE 3

This example describes the identification and initial characterization of PFI-63.

Among the 64 compounds identified above, 33 compounds of unknown mechanism of action were identified. One of them, PFI-63, had significant suppression of PAX3-FOXO1 super enhancer activity, and an $IC_{50}$ of ~2-6 μM (Figures 8-9). When rhabdomyosarcoma cells are treated with PFI-63 it showed an elongation of cells as confirmed by morphological imaging and apoptosis as measured by cleaved PARP Westerns.

A cell confluence assay was performed to further assess the cytotoxicity of PFI-63 in RH4 and RH30 rhabdomyosarcoma cells. Confluence data was collected via photographic images of cells treated with PFI-63 at the following time points: 24 hours, 48 hours, 72 hours, 96 hours and 120 hours. Exemplary dose response curves at 72 hours are presented as Figures 10 and 11 in RH4 and RH30 cells, respectively. The results indicate that PFI-63 is toxic to rhabdomyosarcoma cells (i.e. RH4 and RH30 cells) with an $IC_{50}$ of approximately 3-5 μM.

Gene set enrichment analyses (GSEA) were performed according to methods known in the art to evaluate protein expression in cells treated with PFI-63 as compared to control cells. Briefly, gene expression in RH4 cells treated with PFI-63 was assessed by RNASeq, and the results were statistically compared with various pre-existing gene sets. The results indicate that targets of the PAX3-FOXO1 fusion transcription factor are downregulated in the presence of PFI-63 (see Figures 12-15). The results also indicate that genes that are induced upon KDM3B suppression are upregulated in RH4 cells treated with PFI-63 (see Figures 16 and 17). The results also indicate that RH4 cells treated with PFI-63 experience upregulation of targets associated with myogenic differentiation and apoptosis (see Figures 18 and 19).

The GSEA results were supported by analysis via Western blot. Western blot analysis indicated that PFI-63 was associated with an increase in MYOG (Figure 20), which is a transcriptional activator that plays a role in muscle differentiation. Cleaved PARP, associated with apoptosis, also increases in the presence of PFI-63 (Figure 21).
This example confirms that PFI-63 disrupts PAX3-FOXO1 super enhancer circuitry. PFI-63 is also associated with upregulation of targets associated with myogenic differentiation and apoptosis.

EXAMPLE 4

This example describes further characterization of PFI-63.

To identify specific proteins targeted by PFI-63, a genome-scale loss of function time course screen was performed in RH30.19luc cells using two CRISPR/Cas9 libraries (GeCKOv2 and TKOv3) with and without treatment with PFI-63. It was hypothesized that the targets or pathways suppressed by the compound and the genes that are required for survival upon treatment with PFI-63 will be synthetically lethal. The CRISPR/Cas9 screen demonstrated that targets of histone lysine demethylases were synthetically lethal with PFI-63 (see Figure 22).

Accordingly, the results from the CRISPR/Cas9 screen suggested comparison of PFI-63 with other active agents with known activity, including JIB-04 and GSK-J4, known multi-histone lysine demethylase inhibitors. The GSEA results demonstrated that targets upregulated by JIB-04 and GSK-J4 are also upregulated in the presence of PFI-63 (see Figures 23 and 24). This indicates that PFI-63 is in the same class as JIB-04 and GSK-J4, i.e., a histone lysine demethylase inhibitor.

Western blot analysis confirmed that, in cells treated with PFI-63, there is a global increase in H3K27me3 (a repressive epigenetic modification to lysine 27 of the histone H3 protein), H3K4me3 (an activating epigenetic modification to lysine 4 of the histone H3 protein), and H3K9me3 (a repressive epigenetic modification to lysine 9 of the histone H3 protein) (Figure 25). A diagram illustrating this activity is provided as Figure 26.

This example further indicates that PFI-63 is a lysine-specific demethylase (KDM) inhibitor.

EXAMPLE 5

This example describes software based docking predictions and analysis confirming that PFI-63 is a KDM inhibitor and specifically docks in the catalytic domain of KDM3B.

Computer based predictions identified numerous proteins, including several KDMs that are potential targets of PFI-63 (see Figure 27). Computer simulations confirm that PFI-63 docks in the catalytic domain of KDM3B. Figures, 28, 29 and 30, illustrate the predicted docking configuration at increasing levels of magnification and detail. Those skilled in the art will be aware that that the docking sites of KDMs are quite similar (see Figure 31), and therefore, these results indicate that PFI-63 may also bind other KDMs.

EXAMPLE 6

This example describes PFI-63 inhibitory activity as determined using a histone lysine demethylase panel.

PFI-63 was sent to Eurofins (www.eurofins.com) for direct enzymatic inhibition activity analysis. The enzymatic activity of each of the full length protein was analyzed using known substrates such as methylated lysines. The proteins were treated with various concentrations of PFI-63. The results are presented in Table 1.

TABLE 1

| TARGET (JMJ) | TARGET (KDM) | CONCENTRATION (M) | READOUT | MEAN INHIBITION (%) |
|---|---|---|---|---|
| JMJD1B | KDM3B | 1.0E-05 | Enzymatic activity | 84.87 |
| JMJD1B | KDM3B | 1.0E-06 | Enzymatic activity | 32.63 |
| JMJD1B | KDM3B | 1.0E-07 | Enzymatic activity | 7.088 |
| JMJD1B | KDM3B | 1.0E-08 | Enzymatic activity | 7.050 |
| JARID1B | KDM5B | 1.0E-05 | Enzymatic activity | 57.79 |
| JARID1B | KDM5B | 1.0E-06 | Enzymatic activity | 2.21 |
| JARID1B | KDM5B | 1.0E-07 | Enzymatic activity | 11.75 |
| JARID1B | KDM5B | 1.0E-08 | Enzymatic activity | 6.82 |
| JARID1A | KDM5A | 1.0E-05 | Enzymatic activity | 60.58 |
| JARID1A | KDM5A | 1.0E-06 | Enzymatic activity | 11.63 |
| JARID1A | KDM5A | 1.0E-07 | Enzymatic activity | -0.15 |
| JARID1A | KDM5A | 1.0E-08 | Enzymatic activity | -7.59 |
| JMJD2B | KDM4B | 1.0E-05 | Enzymatic activity | 38.21 |
| JMJD2B | KDM4B | 1.0E-06 | Enzymatic activity | 9.80 |
| JMJD2B | KDM4B | 1.0E-07 | Enzymatic activity | 8.33 |
| JMJD2B | KDM4B | 1.0E-08 | Enzymatic activity | 4.03 |
| JMJD3 | KDM6B | 1.0E-05 | Enzymatic activity | 41.67 |
| JMJD3 | KDM6B | 1.0E-06 | Enzymatic activity | 5.38 |
| JMJD3 | KDM6B | 1.0E-07 | Enzymatic activity | -2.27 |
| JMJD3 | KDM6B | 1.0E-08 | Enzymatic activity | -1.80 |
| PHF8 | KDM7B | 1.0E-05 | Enzymatic activity | 61.29 |
| PHF8 | KDM7B | 1.0E-06 | Enzymatic activity | -15.40 |
| PHF8 | KDM7B | 1.0E-07 | Enzymatic activity | 1.57 |
| PHF8 | KDM7B | 1.0E-08 | Enzymatic activity | -14.20 |
| JMJD1A | KDM3A | 1.0E-05 | Enzymatic activity | 55.09 |
| JMJD1A | KDM3A | 1.0E-06 | Enzymatic activity | 1.35 |
| JMJD1A | KDM3A | 1.0E-07 | Enzymatic activity | 1.90 |

(continued)

| TARGET (JMJ) | TARGET (KDM) | CONCENTRATION (M) | READOUT | MEAN INHIBITION (%) |
|---|---|---|---|---|
| JMJD1A | KDM3A | 1.0E-08 | Enzymatic activity | -8.56 |
| FBXL10 | KDM2B | 1.0E-05 | Enzymatic activity | 26.26 |
| FBXL10 | KDM2B | 1.0E-06 | Enzymatic activity | -2.61 |
| FBXL10 | KDM2B | 1.0E-07 | Enzymatic activity | -0.81 |
| FBXL10 | KDM2B | 1.0E-08 | Enzymatic activity | -9.52 |
| UTX | KDM6A | 1.0E-05 | Enzymatic activity | 20.45 |
| UTX | KDM6A | 1.0E-06 | Enzymatic activity | -1.25 |
| UTX | KDM6A | 1.0E-07 | Enzymatic activity | 4.06 |
| UTX | KDM6A | 1.0E-08 | Enzymatic activity | -5.08 |

The results at concentrations $1x10^{-5}$ M and $1x10^{-6}$ M are summarized in Table 2.

TABLE 2

| KDM | TARGET | CONC | UNIT | MEASUREMENT | VALUE |
|---|---|---|---|---|---|
| KDM3B | H3K9me1/2(3) | 1.0E-05 | M | Mean % Inhibition | 84.87 |
| KDM5A | H3K4me3 | 1.0E-05 | M | Mean % Inhibition | 60.58 |
| KDM5B | H3K4me3 | 1.0E-05 | M | Mean % Inhibition | 57.79 |
| KDM3A | H3K9me1/2(3) | 1.0E-05 | M | Mean % Inhibition | 55.09 |
| KDM6B | H3K27me3 | 1.0E-05 | M | Mean % Inhibition | 41.67 |
| KDM4B | H3K9me3 | 1.0E-05 | M | Mean % Inhibition | 38.21 |

| KDM | TARGET | CONC | UNIT | MEASUREMENT | VALUE |
|---|---|---|---|---|---|
| KDM3B | H3K9me1/2(3) | 1.0E-06 | M | Mean % Inhibition | 32.63 |
| KDM5A | H3K4me3 | 1.0E-06 | M | Mean % Inhibition | 11.63 |
| KDM4B | H3K4me3 | 1.0E-06 | M | Mean % Inhibition | 9.80 |
| KDM6B | H3K27me3 | 1.0E-06 | M | Mean % Inhibition | 5.38 |
| KDM5B | H3K4me3 | 1.0E-06 | M | Mean % Inhibition | 2.21 |
| KDM3A | H3K9me3 | 1.0E-06 | M | Mean % Inhibition | 1.35 |

Accordingly, it was observed that PFI-63 inhibited multiple demethylases with the top hit KDM3B (JMJD1B; see Tables 1 and 2) with an $IC_{50}$ of ~ 2-3 μM.

EXAMPLE 7

This example describes the identification and initial characterization of a second histone demethylase inhibitor, PFI-90.

To search for additional histone demethylase inhibitors, 25 compounds structurally similar were screened in the manner described in Example 2. RH4 ALK-Luc and RH4 CMV-Luc cells in 27 μL medium were seeded into separate white-walled and bottomed 384-well plates. Ten-fold serial dilutions of the 25 test compounds were made using an automated liquid handler, and 3 μL of each dilution was added to each well of plates containing cells. Each test concentration was assayed in quadruplicate. Corresponding DMSO dilutions were used as control. Treated plates were then read following the same procedure used for screening described in Example 1 above. Table 3 presents exemplary data for each tested compound at 10 μM and 1 μM dilutions.

TABLE 3

| Compound | Reporter | Avg Absorbance @ 10 μM | Avg Absorbance @ 1 μM |
|---|---|---|---|
| 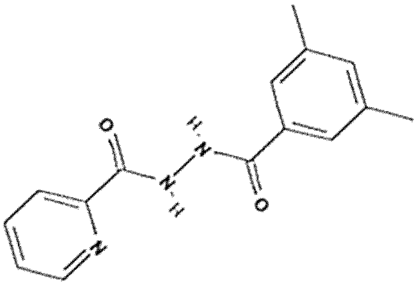 PFI-63 | ALK-Luc | 40.1 | 91.4 |
| | CMV-Luc | 74.5 | 92.3 |
| | | | |
| | ALK-Luc | 89.0 | 90.9 |
| | CMV-Luc | 103.1 | 83.0 |
| 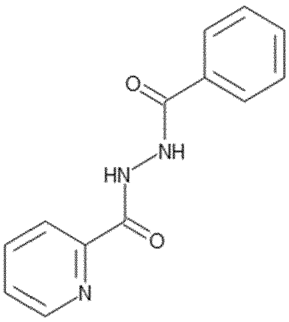 PFI-65 | ALK-Luc | 109.2 | 103.5 |
| | CMV-Luc | 122.1 | 117.9 |
| 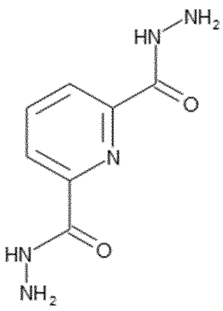 PFI-66 | ALK-Luc | 91.3 | 91.6 |
| | CMV-Luc | 97.0 | 113.3 |
| 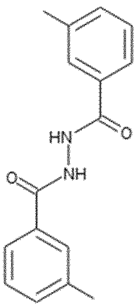 PFI-67 | ALK-Luc | 112.8 | 94.6 |
| | CMV-Luc | 102.8 | 107.0 |

(continued)

| Compound | Reporter | Avg Absorbance @ 10 μM | Avg Absorbance @ 1 μM |
|---|---|---|---|
| 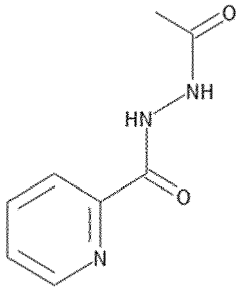 PFI-68 | ALK-Luc | 98.0 | 95.0 |
| | CMV-Luc | 105.4 | 107.2 |
| 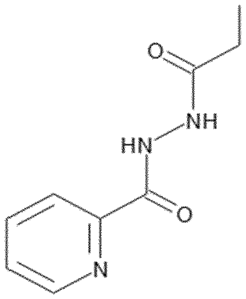 PFI-69 | ALK-Luc | 94.4 | 104.3 |
| | CMV-Luc | 98.5 | 100.3 |
| 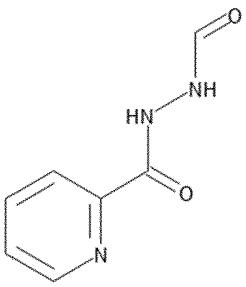 PFI-70 | ALK-Luc | 93.3 | 99.7 |
| | CMV-Luc | 103.6 | 108.0 |
| 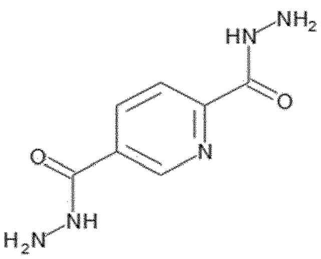 PFI-71 | ALK-Luc | 108.6 | 100.1 |
| | CMV-Luc | 101.4 | 100.9 |
| 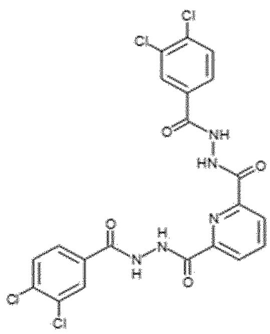 PFI-72 | ALK-Luc | 91.1 | 97.2 |
| | CMV-Luc | 87.7 | 97.2 |

(continued)

| Compound | Reporter | Avg Absorbance @ 10 μM | Avg Absorbance @ 1 μM |
|---|---|---|---|
| 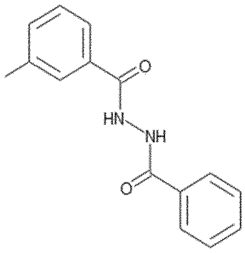 PFI-73 | ALK-Luc | 122.8 | 98.3 |
| | CMV-Luc | 119.4 | 113.2 |
| 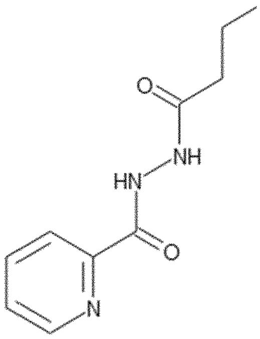 PFI-74 | ALK-Luc | 94.7 | 102.4 |
| | CMV-Luc | 103.9 | 101.0 |
| 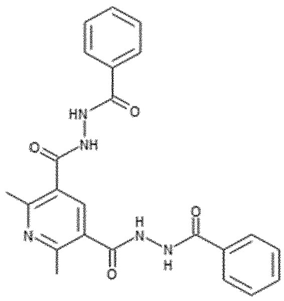 PFI-75 | ALK-Luc | 110.5 | 100.2 |
| | CMV-Luc | 104.9 | 96.5 |
| 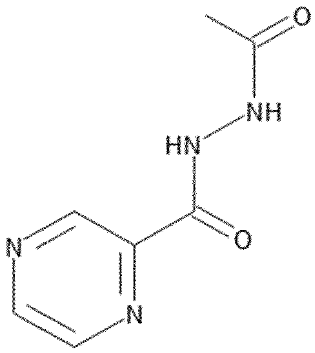 PFI-76 | ALK-Luc | 100.2 | 105.6 |
| | CMV-Luc | 102.5 | 97.9 |

(continued)

| Compound | Reporter | Avg Absorbance @ 10 μM | Avg Absorbance @ 1 μM |
|---|---|---|---|
| 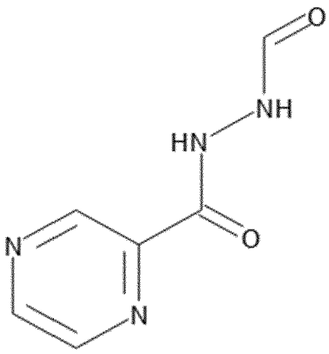 PFI-77 | ALK-Luc | 96.5 | 95.8 |
| | CMV-Luc | 106.1 | 99.0 |
| 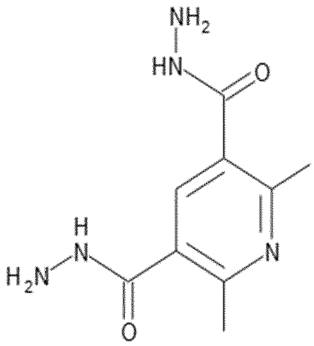 PFI-78 | ALK-Luc | 95.3 | 100.3 |
| | CMV-Luc | 104.3 | 95.6 |
| 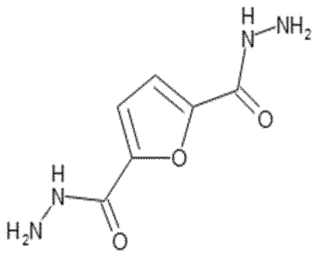 PFI-79 | ALK-Luc | 97.6 | 97.0 |
| | CMV-Luc | 101.5 | 95.0 |
| 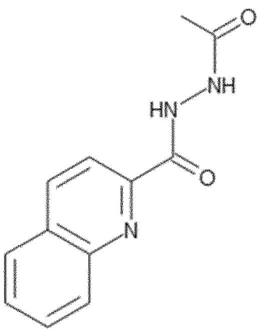 PFI-80 | ALK-Luc | 96.6 | 90.1 |
| | CMV-Luc | 90.7 | 105.4 |
| 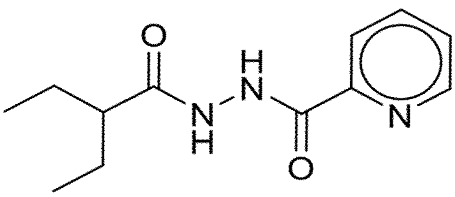 PFI-81 | ALK-Luc | 89.1 | 97.7 |
| | CMV-Luc | 86.1 | 105.1 |

(continued)

| Compound | Reporter | Avg Absorbance @ 10 µM | Avg Absorbance @ 1 µM |
|---|---|---|---|
| 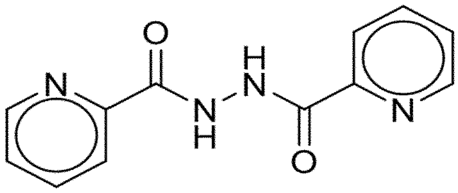 PFI-82 | ALK-Luc | 107.1 | 112.4 |
| | CMV-Luc | 136.3 | 103.8 |
| 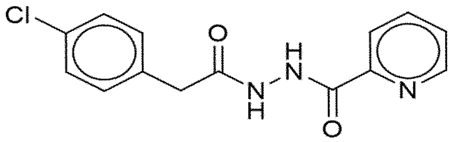 PFI-83 | ALK-Luc | 32.2 | 97.8 |
| | CMV-Luc | 90.6 | 107.2 |
| 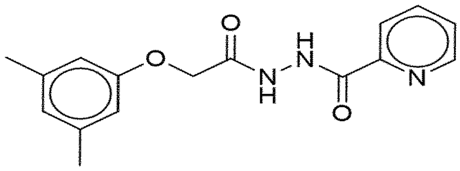 PFI-84 | ALK-Luc | 56.5 | 99.4 |
| | CMV-Luc | 74.8 | 100.8 |
| 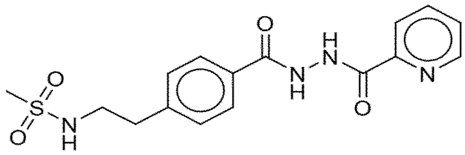 PFI-85 | ALK-Luc | 100.2 | 99.7 |
| | CMV-Luc | 108.9 | 101.7 |
| 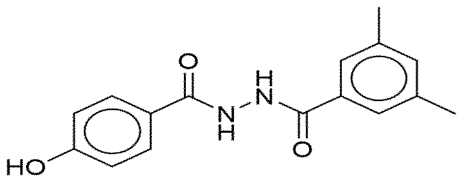 PFI-86 | ALK-Luc | 84.1 | 97.7 |
| | CMV-Luc | 104.7 | 92.6 |
| 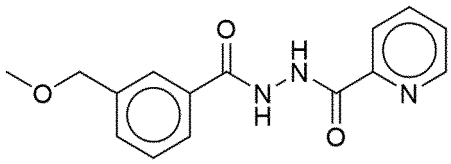 PFI-87 | ALK-Luc | 120.4 | 115.5 |
| | CMV-Luc | 151.1 | 123.7 |
| 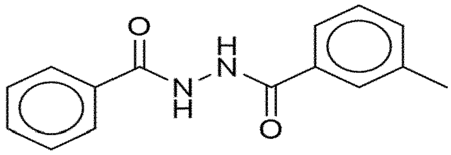 PFI-88 | ALK-Luc | 121.4 | 91.6 |
| | CMV-Luc | 118.7 | 102.8 |
| 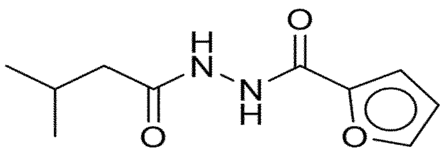 PFI-89 | ALK-Luc | 92.5 | 95.1 |
| | CMV-Luc | 98.3 | 97.7 |

(continued)

| Compound | Reporter | Avg Absorbance @ 10 µM | Avg Absorbance @ 1 µM |
|---|---|---|---|
| 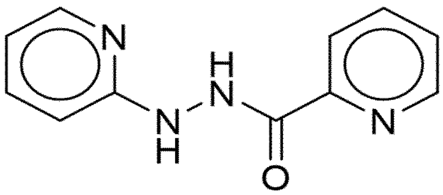 PFI-90 | ALK-Luc | 34.0 | 55.9 |
| | CMV-Luc | 160.9 | 78.0 |

The screen identified a second potential histone demethylase inhibitor, designated PFI-90.

A cell confluence assay was performed to assess the cytotoxicity of PFI-90 in RH4 and RH30 rhabdomyosarcoma cells. Confluence data was collected via photographic images of cells treated with PFI-90 at the following time points: 24 hours, 48 hours, 72 hours, 96 hours and 120 hours. Exemplary dose response curves at 72 hours are presented as Figures 32 and 33 in RH4 and RH30 cells, respectively. The results indicate that PFI-90 is more cytotoxic to rhabdomyosarcoma cells than PFI-63.

GSEA assays like those performed in Example 3 above, were also performed to assess PFI-90. The results for PFI-90 were similar to those obtained for PFI-63. Targets of the PAX3-FOXO1 fusion transcription factor are downregulated in RH4 cells in the presence of PFI-90 (see Figures 34-36). The results also indicate that genes that are induced upon KDM3B suppression are upregulated in RH4 cells treated with PFI-90 (see Figures 37-38). The results also indicate that cells treated with PFI-90 experience upregulation of targets associated with myogenic differentiation and apoptosis (see Figures 39-40).

PFI-90 also has improved solubility relative to PFI-63, with a Log P value of 0.82 compared with a Log P value of 2.36 for PFI-63.

EXAMPLE 8

Analysis via Western blot confirms that PFI-90 inhibits lysine demethylase activity. Figure 41 shows that as the concentration of PFI-90 increases, the methylation of histone H3 also increases. These results are summarized in Figure 42.

As described above, PFI-90 inhibitory activity was determined by Eurofins using enzymatic inhibition activity analysis of a panel of histone demethylases. The results are presented in Table 4.

TABLE 4

| TARGET (JMJ) | TARGET (KDM) | CONCENTRATION (M) | READOUT | MEAN INHIBITION (%) |
|---|---|---|---|---|
| JMJD1B | KDM3B | 1.0E-05 | Enzymatic activity | 87.14 |
| JMJD1B | KDM3B | 5.0E-06 | Enzymatic activity | 81.56 |
| JMJD1B | KDM3B | 2.5E-06 | Enzymatic activity | 70.58 |
| JMJD1B | KDM3B | 1.3E-06 | Enzymatic activity | 55.46 |
| JMJD1B | KDM3B | 6.3E-07 | Enzymatic activity | 36.89 |
| JMJD1B | KDM3B | 3.1E-07 | Enzymatic activity | 30.81 |
| JMJD1B | KDM3B | 1.6E-07 | Enzymatic activity | 24.47 |
| JMJD1B | KDM3B | 7.8E-08 | Enzymatic activity | 14.32 |
| JARID1A | KDM5A | 1.0E-05 | Enzymatic activity | 83.32 |
| JARID1A | KDM5A | 5.0E-06 | Enzymatic activity | 64.60 |
| JARID1A | KDM5A | 2.5E-06 | Enzymatic activity | 51.29 |
| JARID1A | KDM5A | 1.3E-06 | Enzymatic activity | 20.23 |
| JARID1A | KDM5A | 6.3E-07 | Enzymatic activity | 15.42 |
| JARID1A | KDM5A | 3.1E-07 | Enzymatic activity | 7.18 |

(continued)

| TARGET (JMJ) | TARGET (KDM) | CONCENTRATION (M) | READOUT | MEAN INHIBITION (%) |
|---|---|---|---|---|
| JARID1A | KDM5A | 1.6E-07 | Enzymatic activity | 2.33 |
| JARID1A | KDM5A | 7.8E-08 | Enzymatic activity | -10.90 |
| JMJD2B | KDM4B | 1.0E-05 | Enzymatic activity | 82.94 |
| JMJD2B | KDM4B | 5.0E-06 | Enzymatic activity | 71.59 |
| JMJD2B | KDM4B | 2.5E-06 | Enzymatic activity | 52.51 |
| JMJD2B | KDM4B | 1.3E-06 | Enzymatic activity | 38.48 |
| JMJD2B | KDM4B | 6.3E-07 | Enzymatic activity | 27.94 |
| JMJD2B | KDM4B | 3.1E-07 | Enzymatic activity | 11.55 |
| JMJD2B | KDM4B | 1.6E-07 | Enzymatic activity | 16.27 |
| JMJD2B | KDM4B | 7.8E-08 | Enzymatic activity | 41.56 |
| JMJD3 | KDM6B | 1.0E-05 | Enzymatic activity | 46.80 |
| JMJD3 | KDM6B | 5.0E-06 | Enzymatic activity | 17.41 |
| JMJD3 | KDM6B | 2.5E-06 | Enzymatic activity | 11.55 |
| JMJD3 | KDM6B | 1.3E-06 | Enzymatic activity | 8.42 |
| JMJD3 | KDM6B | 6.3E-07 | Enzymatic activity | 6.81 |
| JMJD3 | KDM6B | 3.1E-07 | Enzymatic activity | -0.60 |
| JMJD3 | KDM6B | 1.6E-07 | Enzymatic activity | 25.15 |
| JMJD3 | KDM6B | 7.8E-08 | Enzymatic activity | 8.60 |

The results at concentrations $1x10^{-5}$ M and $1x10^{-6}$ M are summarized in Table 5.

TABLE 5

| KDM | TARGET | CONC | UNIT | MEASUREMENT | VALUE |
|---|---|---|---|---|---|
| KDM3B | H3K9me1/2(3) | 1.0E-05 | M | Mean % Inhibition | 87.1 |
| KDM5A | H3K4me3 | 1.0E-05 | M | Mean % Inhibition | 83.3 |
| KDM4B | H3K9me3 | 1.0E-05 | M | Mean % Inhibition | 82.9 |
| KDM6B | H3K27me3 | 1.0E-05 | M | Mean % Inhibition | 46.8 |

| KDM | TARGET | CONC | UNIT | MEASUREMENT | VALUE |
|---|---|---|---|---|---|
| KDM3B | H3K9me1/2(3) | 1.0E-06 | M | Mean % Inhibition | 55.5 |
| KDM4B | H3K4me3 | 1.0E-06 | M | Mean % Inhibition | 38.5 |
| KDM5A | H3K4me3 | 1.0E-06 | M | Mean % Inhibition | 20.2 |
| KDM6B | H3K27me3 | 1.0E-06 | M | Mean % Inhibition | 8.4 |

Accordingly, it was observed that PFI-90 inhibited multiple demethylases with the top hit KDM3B (JMJD1B; see Table 5).

EXAMPLE 9

The impact of PFI-63 and PFI-90 on apoptosis in rhabdomyosarcoma cells was investigated by assaying caspase 3 levels in the cells, and by annexin V staining. In both RH4 and SCMC human rhabdomyosarcoma cells, PFI-63 and PFI-90 lead to increased caspase 3 (Figures 43 and 44). Annexin staining indicates an increase in cell death relative to

DMSO control (Figure 45) for RH4 cells treated with PFI-90 (Figure 46) and PFI-63 (Figure 47).

Analysis via Western Blot also shows an increase in apoptosis. In RH4 cells treated with PFI-90 at 3 μM, an increase in cleaved PARP (a marker for apoptosis) is observed at 24 hours (Figure 48). This coincides with a decrease in PAX3-FOXO1 which is also observed at 24 hours (Figure 49).

This example further demonstrates that PFI-63 and PFI-90 increase apoptosis in RH4 and SCMC cells.

EXAMPLE 10

This example describes the testing of PFI-90 against the NCI-60 panel. The NCI-60 cancer cell line panel is a collection of 60 human cancer cell lines maintained by the National Cancer Institute (see Shoemaker et al., Nat. Rev. Cancer, 6: 813-823 (2006)). Briefly, the percentage growth of the cancer cells lines was measured in the presence of PFI-90 at various concentrations. Results indicate that PFI-90 has the potential to treat a range of cancers.

Figure 50 shows PFI-90's inhibition of various leukemia cell lines. At higher concentrations PFI-90 slows and/or stops the growth of the cells. Similar results were obtained for non-small cell lung cancers (Figure 51), CNS cancers (Figure 52), melanoma (Figure 53), prostate cancer (Figure 54), colon cancer (Figure 55), ovarian cancer (Figure 56) and breast cancers (Figure 57). In renal cancer cell lines, PFI-90 inhibits growth and leads to cell death (Figure 58).

EXAMPLE 11

This example describes testing of PFI-63 and PFI-90 in Ewing's sarcoma and osteosarcoma cells.

A cell confluence assay was performed to further assess the cytotoxicity of PFI-63 in in OSA-CL osteosarcoma cells. Confluence data was collected via photographic images of cells treated with PFI-63 at the following time points: 24 hours, 48 hours, 72 hours, 96 hours and 120 hours. Exemplary dose response curves at 72 hours are presented as Figures 59 and 60 for PFI-63 and PFI-90 respectively. The results indicate that PFI-63 is toxic to osteosarcoma cells with an $IC_{50}$ of approximately 7.5 μM. The results indicate that PFI-90 is toxic to osteosarcoma cells with an $IC_{50}$ of approximately 2 μM.

A similar assay was performed to assess the cytotoxicity of PFI-63 and PFI-90 in TC-32 Ewing's sarcoma cells. Exemplary dose response curves at 72 hours are presented as Figures 61 and 62 for PFI-63 and PFI-90 respectively. The results indicate that PFI-63 is toxic to Ewing's sarcoma cells with an $IC_{50}$ of approximately 2 μM. The results indicate that PFI-90 is toxic to Ewing's sarcoma cells with an $IC_{50}$ of approximately 1 μM.

This example indicates that PFI-90 and PFI-63 are cytotoxic to Ewing's sarcoma and osteosarcoma cells.

Example 12

Nuclear magnetic resonance (NMR) binding assays confirmed specific binding of PFI-90 to the catalytic domain of KDM3B. A WaterLOGSY assay and a Carr-Purcell Meiboom-Gill (CPMG) based assay were used to investigate protein-ligand binding affinity between PFI-90 and a truncated KDM3B catalytic domain.

WaterLOGSY (Water-Ligand Observed via Gradient Spectroscopy) is a Proton NMR experiment used in drug-discovery to validate the presence of a reversible binding interaction of mild to high affinity from a small molecule to a label-free target (e.g., a protein). In traditional proton NMR experiments, a series of peaks are generated that represent the structure of a solitary small molecule, whereas the WaterLOGSY experiment examines the behavior of a small molecule's peaks in the presence of a potential target. This is achieved by observing differences in interaction between a small molecule interacting with water co-occupying a binding cleft on a target as compared to water dispersed freely in solution. The experiment then depicts the ratio of bound to unbound small molecule by phasing proton NMR spectra on the X-axis in opposing directions depending on the magnitude of that ratio (i.e., increasing ratios above 1 will phase positively, decreasing ratios below 1 will phase negatively).

CPMG (Carr-Purcell-Meiboom-Gill) Relaxation Editing Proton NMR is another drug-discovery NMR experiment that may be used in conjunction with WaterLOGSY to orthogonally validate the presence of a reversible binding interaction of mild to high affinity from a small molecule to a label-free target. In contrast to WaterLOGSY, all peaks are phased above the X-axis. Instead, this experiment filters out the proportion of peak height that is contributed from the quantity of small molecule bound to a target of high molecular weight (e.g., a protein). Once the attenuation of peak height is equivalent to a 20% reduction, the compound is said to bind, with an increasing ratio of bound to unbound small molecules manifesting in increasing signal attenuation.

For reference, Figure 63 depicts NMR peaks for PFI-90. While the peaks are complex, all relevant peaks are clearly resolved. Figure 64 depicts NMR peaks obtained for αketoglutarate (also called 2-oxoglutaric acid or "2-OG"), a substrate of histone demethylases. 2-OG peaks are easily resolved. Figure 65 depicts results from a 1D 1H analysis of PFI-90. Chemical shift perturbation upon addition of the truncated KDM3B catalytic domain are not influences by 2-OG presence.

In the WaterLOGSY experiment, PFI-90 was found to bind at 300 μM in the presence and in the absence of 2-OG.

2-OG did not bind at 100 μM (see Figure 66).

In the CPMG experiment, strong binding of PFI-90 was observed in the presence and absence of 2-OG. 2-OG did not bind at 100 μM. Complete/near complete height attenuation is observed in 5 out of 9 PFI-90 peaks, 70% attenuation is observed in 1 of 9 peaks and 50% attenuation is observed in 3 of 9 peaks (see Figure 67).

These NMR assays established that PFI-90 binds to the truncated KDM3B catalytic domain.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

## Claims

1. A pharmaceutical composition comprising (a) one or more compounds selected from the group consisting of

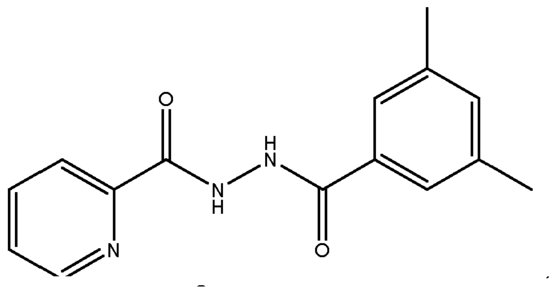

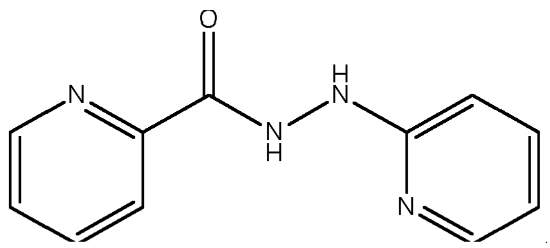

and pharmaceutically acceptable salts thereof, and (b) a pharmaceutically acceptable carrier for use in the treatment of cancer in a mammal,

optionally wherein the cancer is a transcription driven cancer,
optionally wherein the cancer is selected from the group consisting of rhabdomyosarcoma, Ewing's sarcoma, colorectal cancer, ovarian cancer, prostate cancer, CNS cancer, melanoma, breast cancer, leukemia, non-small cell lung cancer, renal cancer, and osteosarcoma,
optionally wherein the cancer is Ewing's sarcoma, rhabdomyosarcoma, or alveolar rhabdomyosarcoma,
and optionally wherein the cancer is selected from the group consisting of PAX3-FOXO1 fusion transcription factor positive rhabdomyosarcoma and PAX7-FOXO1 fusion transcription factor positive rhabdomyosarcoma.

2. The composition for use of claim 1, wherein a histone demethylase is inhibited by the compound in the mammal.

3. The composition for use of claim 2, wherein the histone demethylase is a lysine-specific histone demethylase.

4. The composition for use of claim 3, wherein the lysine specific histone demethylase is selected from the group consisting of KDM3A, KDM3B, KDM5A, KDM5B, KDM4B, and KDM6B, optionally wherein the histone demethylase is KDM3B.

5. The composition for use of claim 1, wherein the cancer is PAX3-FOXO1 fusion transcription factor positive rhabdomyosarcoma, and wherein the function of the PAX3-FOXO1 fusion transcription factor is disrupted by the compound in the mammal.

6. The composition for use of claim 1, wherein the cancer is PAX7-FOXO1 fusion transcription factor positive rhabdomyosarcoma, and wherein the function of the PAX7-FOXO1 fusion transcription factor is disrupted by the compound in the mammal.

7. A method of inhibiting a histone demethylase in cells *in vitro* comprising contacting the cells with one or more compounds selected from the group consisting of:

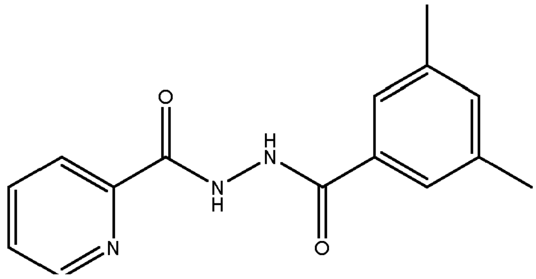

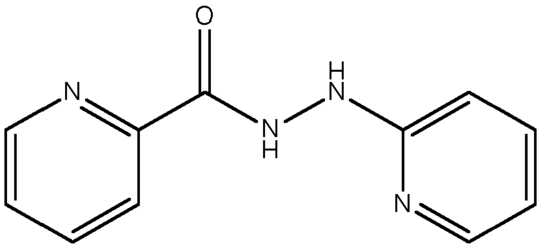

,

and pharmaceutically acceptable salts thereof, and (b) and a pharmaceutically acceptable carrier, optionally wherein the compound is:

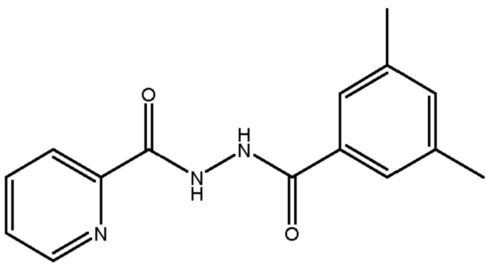

,

or

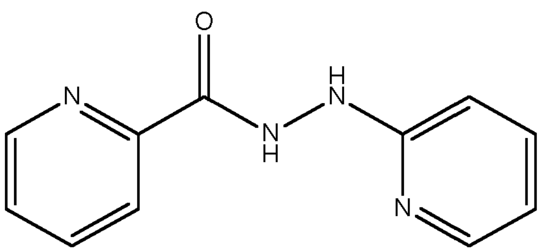

.

**8.** The method of claim 7, wherein the cells are human cells,

optionally wherein the cells are cancer cells,
optionally wherein the cancer cells are selected from the group consisting of rhabdomyosarcoma, Ewing's sarcoma, colorectal cancer, ovarian cancer, prostate cancer, CNS cancer, melanoma, breast cancer, leukemia, non-small cell lung cancer, renal cancer, and osteosarcoma cells, and
optionally wherein the cancer cells are Ewing's sarcoma cells, rhabdomyosarcoma cancer cells, or alveolar rhabdomyosarcoma cells.

**9.** The method of claim 8, wherein the cancer cells are selected from the group consisting of PAX3-FOXO1 fusion transcription factor positive rhabdomyosarcoma and PAX7-FOXO1 fusion transcription factor positive rhabdomyosarcoma.

**10.** The method of claim 8, wherein the cancer is PAX3-FOXO1 fusion transcription factor positive rhabdomyosarcoma, and wherein the function of the PAX3-FOXO1 fusion transcription factor is disrupted by the inhibition of the histone demethylase.

**11.** The method of claim 9, wherein the cancer is PAX7-FOXO1 fusion transcription factor positive rhabdomyosarcoma, and wherein the function of the PAX7-FOXO1 fusion transcription factor is disrupted by the inhibition of the histone demethylase.

**12.** The method of any one of claims 7-11, wherein the histone demethylase is a lysine specific histone demethylase.

**13.** The method of claim 12, wherein the lysine specific histone demethylase is selected from the group consisting of KDM3A, KDM3B, KDM5A, KDM5B, KDM4B, and KDM6B, optionally wherein the lysine specific histone demethylase is KDM3B.

**14.** The composition for the use of claim 1, wherein the compound is

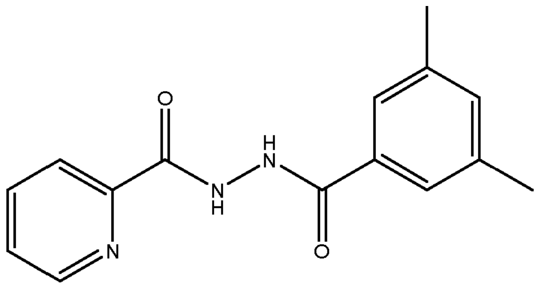

.

**15.** The composition for the use of claim 1, wherein the compound is

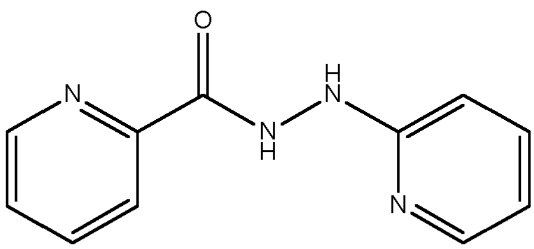

.

**Patentansprüche**

**1.** Pharmazeutische Zusammensetzung, umfassend (a) eine oder mehrere Verbindungen, ausgewählt aus der Gruppe bestehend aus

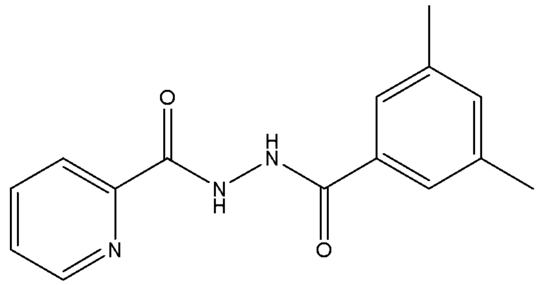

,

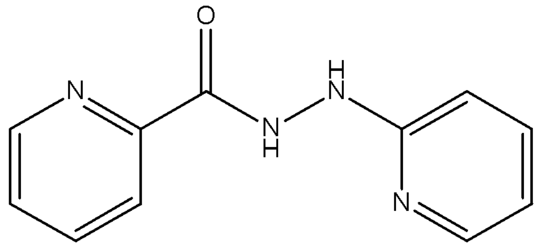

,

und pharmazeutisch verträglichen Salzen davon, und (b) einem pharmazeutisch verträglichen Träger zur Verwendung bei der Behandlung von Krebs bei einem Säugetier,

wobei der Krebs optional ein transkriptionsgetriebener Krebs ist,
wobei der Krebs optional ausgewählt ist aus der Gruppe bestehend aus Rhabdomyosarkom, Ewing-Sarkom, Darmkrebs, Eierstockkrebs, Prostatakrebs, ZNS-Krebs, Melanom, Brustkrebs, Leukämie, nicht-kleinzelligem Lungenkrebs, Nierenkrebs und Osteosarkom,
wobei der Krebs optional ein Ewing-Sarkom, ein Rhabdomyosarkom oder ein alveoläres Rhabdomyosarkom ist, und wobei der Krebs optional aus der Gruppe ausgewählt ist, die aus PAX3-FOXO1-Fusions-Transkriptionsfaktor-positivem Rhabdomyosarkom und PAX7-FOXO1-Fusions-Transkriptionsfaktor-positivem Rhabdomyosarkom besteht.

**2.** Die Zusammensetzung zur Verwendung nach Anspruch 1, wobei eine Histon-Demethylase durch die Verbindung im Säugetier gehemmt wird.

**3.** Die Zusammensetzung zur Verwendung nach Anspruch 2, wobei die Histon-Demethylase eine Lysin-spezifische Histon-Demethylase ist.

**4.** Die Zusammensetzung zur Verwendung nach Anspruch 3, wobei die lysinspezifische Histon-Demethylase aus der Gruppe ausgewählt ist, die aus KDM3A, KDM3B, KDM5A, KDM5B, KDM4B und KDM6B besteht, wobei die Histon-Demethylase optional KDM3B ist.

**5.** Die Zusammensetzung zur Verwendung gemäß Anspruch 1, wobei der Krebs ein PAX3-FOXO1-Fusions-Transkriptionsfaktor-positives Rhabdomyosarkom ist und wobei die Funktion des PAX3-FOXO1-Fusions-Transkriptionsfaktors durch die Verbindung im Säugetier gestört wird.

**6.** Die Zusammensetzung zur Verwendung nach Anspruch 1, wobei der Krebs ein PAX7-FOXO1-Fusions-Transkriptionsfaktor-positives Rhabdomyosarkom ist und wobei die Funktion des PAX7-FOXO1-Fusions-Transkriptionsfaktors durch die Verbindung im Säugetier gestört wird.

**7.** Verfahren zur Hemmung einer Histon-Demethylase in Zellen *in vitro,* umfassend das Inkontaktbringen der Zellen mit einer oder mehreren Verbindungen, ausgewählt aus der Gruppe bestehend aus:

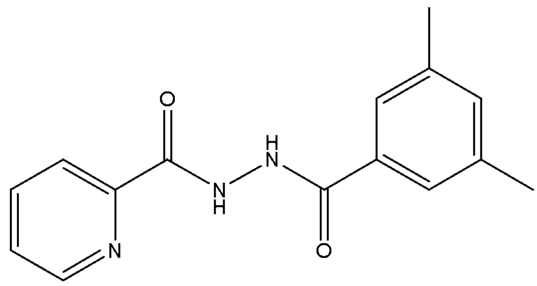

,

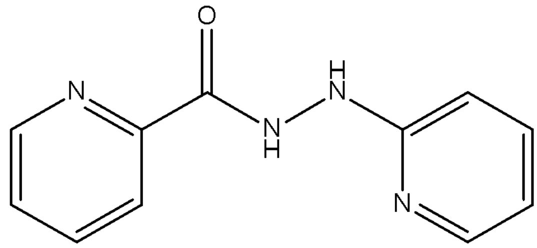

,

und pharmazeutisch verträglichen Salzen davon, und (b) und einem pharmazeutisch verträglichen Träger, wobei die Verbindung optional

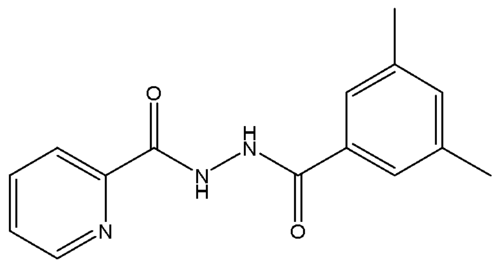

,

oder

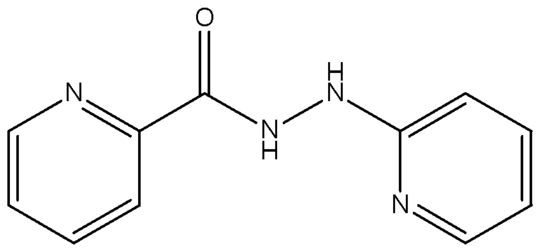

ist.

**8.** Verfahren nach Anspruch 7, wobei die Zellen menschliche Zellen sind,

wobei die Zellen optional Krebszellen sind,
wobei die Krebszellen optional aus der Gruppe ausgewählt sind, die aus Rhabdomyosarkom-, Ewing-Sarkom-, Darmkrebs-, Eierstockkrebs-, Prostatakrebs-, ZNS-Krebs-, Melanom-, Brustkrebs-, Leukämie-, nicht-kleinzelligem Lungenkrebs-, Nierenkrebs- und Osteosarkomzellen besteht, und
wobei die Krebszellen optional Ewing-Sarkom-Zellen, Rhabdomyosarkom-Krebszellen oder alveoläre Rhabdomyosarkom-Zellen sind.

**9.** Verfahren nach Anspruch 8, wobei die Krebszellen aus der Gruppe ausgewählt sind, die aus PAX3-FOXO1-Fusions-Transkriptionsfaktor-positivem Rhabdomyosarkom und PAX7-FOXO1-Fusions-Transkriptionsfaktor-positivem Rhabdomyosarkom besteht.

**10.** Verfahren nach Anspruch 8, wobei der Krebs ein PAX3-FOXO1-Fusions-Transkriptionsfaktor-positives Rhabdomyosarkom ist und wobei die Funktion des PAX3-FOXO1-Fusions-Transkriptionsfaktors durch die Hemmung der Histon-Demethylase gestört wird.

**11.** Verfahren nach Anspruch 9, wobei der Krebs ein PAX7-FOXO1-Fusions-Transkriptionsfaktor-positives Rhabdomyosarkom ist und wobei die Funktion des PAX7-FOXO1-Fusions-Transkriptionsfaktors durch die Hemmung der Histon-Demethylase gestört wird.

**12.** Verfahren nach einem der Ansprüche 7 bis 11, wobei die Histon-Demethylase eine lysinspezifische Histon-Demethylase ist.

**13.** The method of claim 12, wherein the lysine specific histone demethylase is selected from the group consisting of KDM3A, KDM3B, KDM5A, KDM5B, KDM4B, and KDM6B, optionally wherein the lysine specific histone demethylase is KDM3B.

**14.** Die Zusammensetzung zur Verwendung gemäß Anspruch 1, wobei die Verbindung

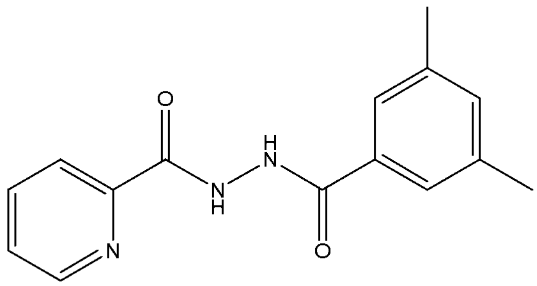

ist.

**15.** Die Zusammensetzung zur Verwendung gemäß Anspruch 1, wobei die Verbindung

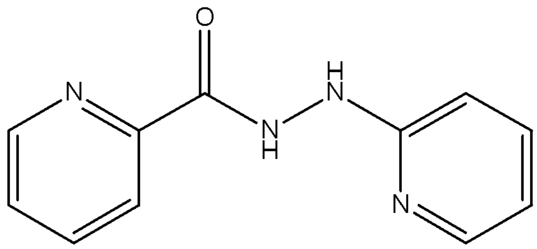

ist.

## Revendications

**1.** Composition pharmaceutique comprenant (a) un ou plusieurs composés sélectionnés dans le groupe constitué par

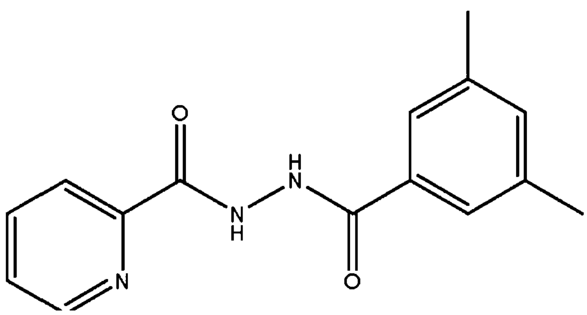

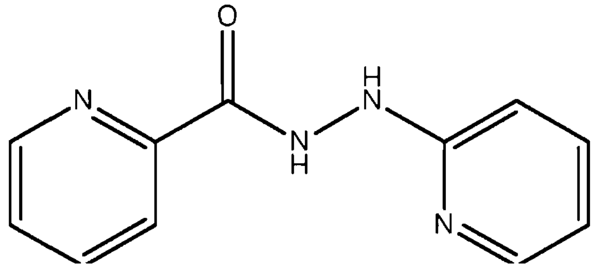

et des sels pharmaceutiquement acceptables de ceux-ci, et (b) un support pharmaceutiquement acceptable pour utilisation dans le traitement d'un cancer chez un mammifère,
facultativement sachant que le cancer est un cancer entraîné par transcription,
facultativement sachant que le cancer est sélectionné dans le groupe constitué par le rhabdomyosarcome, le sarcome d'Ewing, le cancer colorectal, le cancer de l'ovaire, le cancer de la prostate, le cancer du SNC, le mélanome, le cancer du sein, la leucémie, le cancer du poumon non à petites cellules, le cancer du rein, et l'ostéosarcome,
facultativement sachant que le cancer est le sarcome d'Ewing, le rhabdomyosarcome, ou le rhabdomyosarcome alvéolaire,
et facultativement sachant que le cancer est sélectionné dans le groupe constitué par le rhabdomyosarcome positif pour le facteur de transcription de fusion PAX3-FOXO1 et le rhabdomyosarcome positif pour le facteur de transcription de fusion PAX7-FOXO1.

2. La composition pour utilisation de la revendication 1, sachant qu'une histone déméthylase est inhibée par le composé chez le mammifère.

3. La composition pour utilisation de la revendication 2, sachant que l'histone déméthylase est une histone déméthylase spécifique de la lysine.

4. La composition pour utilisation de la revendication 3, sachant que l'histone déméthylase spécifique de la lysine est sélectionnée dans le groupe constitué par KDM3A, KDM3B, KDM5A, KDM5B, KDM4B, et KDM6B, facultativement sachant que l'histone déméthylase est KDM3B.

5. La composition pour utilisation de la revendication 1, sachant que le cancer est le rhabdomyosarcome positif pour le facteur de transcription de fusion PAX3-FOXO1, et sachant que la fonction du facteur de transcription de fusion PAX3-FOXO1 est perturbée par le composé chez le mammifère.

6. La composition pour utilisation de la revendication 1, sachant que le cancer est le rhabdomyosarcome positif pour le facteur de transcription de fusion PAX7-FOXO1, et sachant que la fonction du facteur de transcription de fusion PAX7-FOXO1 est perturbée par le composé chez le mammifère.

7. Procédé d'inhibition d'une histone déméthylase dans des cellules *in vitro* comprenant la mise en contact des cellules avec un ou plusieurs composés sélectionnés dans le groupe constitué par :

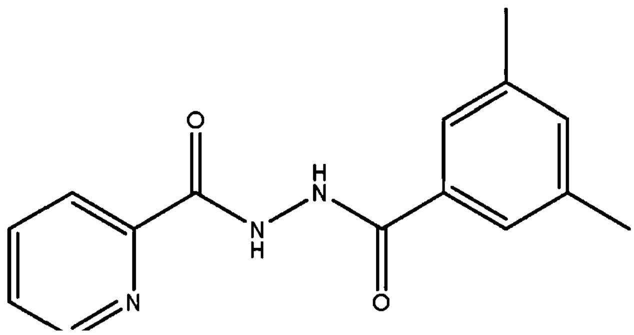

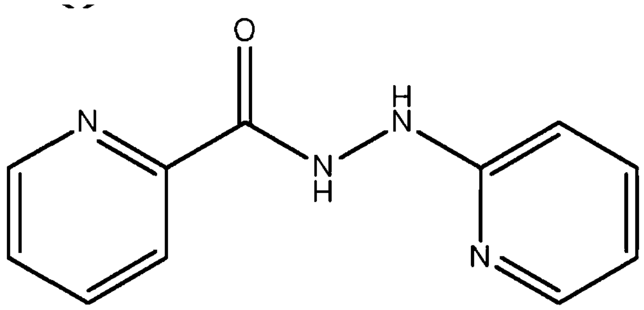
,

et des sels pharmaceutiquement acceptables de ceux-ci, et (b) un support pharmaceutiquement acceptable, facultativement sachant que le composé est :

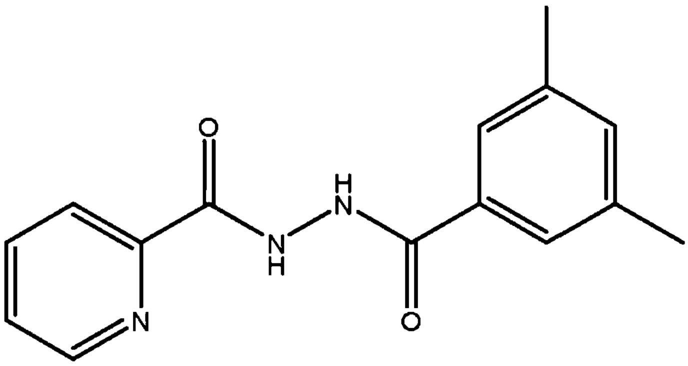
,

ou

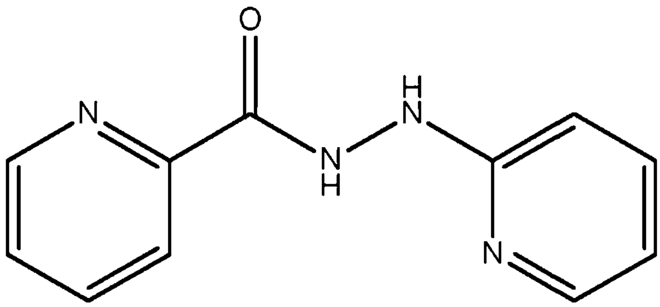
.

**8.** Le procédé de la revendication 7, sachant que les cellules sont des cellules humaines,

facultativement sachant que les cellules sont des cellules cancéreuses,
facultativement sachant que les cellules cancéreuses sont sélectionnées dans le groupe constitué par les cellules du rhabdomyosarcome, du sarcome d'Ewing, du cancer colorectal, du cancer de l'ovaire, du cancer de la prostate, du cancer du SNC, du mélanome, du cancer du sein, de la leucémie, du cancer du poumon non à petites cellules, du cancer du rein, et de l'ostéosarcome, et
facultativement sachant que les cellules cancéreuses sont des cellules du sarcome d'Ewing, des cellules cancéreuses du rhabdomyosarcome, ou des cellules du rhabdomyosarcome alvéolaire.

**9.** Le procédé de la revendication 8, sachant que les cellules cancéreuses sont sélectionnées dans le groupe constitué par le rhabdomyosarcome positif pour le facteur de transcription de fusion PAX3-FOXO1 et le rhabdomyosarcome positif pour le facteur de transcription de fusion PAX7-FOXO1.

**10.** Le procédé de la revendication 8, sachant que le cancer est le rhabdomyosarcome positif pour le facteur de transcription de fusion PAX3-FOXO1, et sachant que la fonction du facteur de transcription de fusion PAX3-FOXO1 est perturbée par l'inhibition de l'histone déméthylase.

**11.** Le procédé de la revendication 9, sachant que le cancer est le rhabdomyosarcome positif pour le facteur de transcription de fusion PAX7-FOXO1, et sachant que la fonction du facteur de transcription de fusion PAX7-FOXO1 est perturbée par l'inhibition de l'histone déméthylase.

**12.** Le procédé de l'une quelconque des revendications 7 à 11, sachant que l'histone déméthylase est une histone déméthylase spécifique de la lysine.

**13.** Le procédé de la revendication 12, sachant que l'histone déméthylase spécifique de la lysine est sélectionnée dans le groupe constitué par KDM3A, KDM3B, KDM5A, KDM5B, KDM4B, et KDM6B, facultativement sachant que l'histone déméthylase spécifique de la lysine est KDM3B.

**14.** La composition pour l'utilisation de la revendication 1, sachant que le composé est

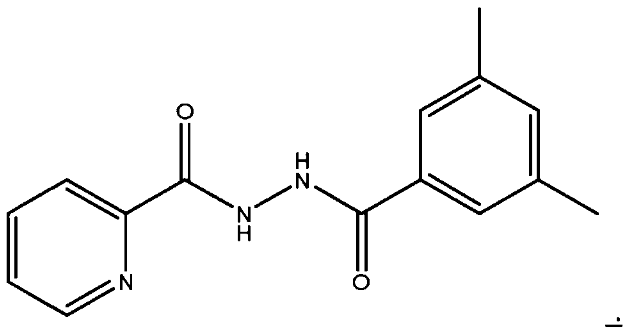

**15.** La composition pour l'utilisation de la revendication 1, sachant que le composé est

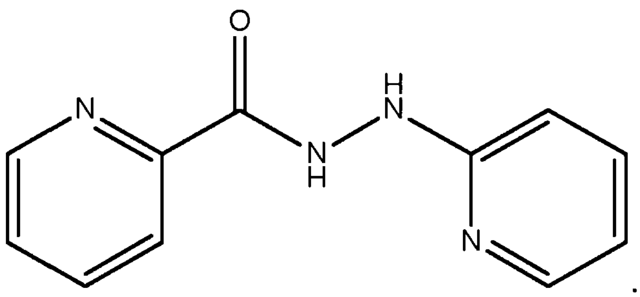

**SEQ ID NO: 1**

ALK Enhancer Sequence

TGCCTGGGTAAAGAAGCTAGGGAATTGGGGGATATGGACATAGAGTGATCATGTTTATTCT
CTTTCCAACAAAGGCCTGAATTAACAAACACAAAATGGCTCTGCTGTCAAGAAAAACACAG
TGGGTAGATACACTTGAAAGTCACTTCTGAAAGAGACCTTGAAGAAGGGATGGAGAATGTT
TGGGGTGGACAGAAACTCCTCCAAGAAAGACAATGAAAGACAACAAGTTAG**GTCACG**GTGG
CTAAAGAGGAGACAATGATTTGTTGTCTGACTCTCCAGCTACGATGGGGCTGTAACTAATG
AGGTTATTCTGGTTACAAAGGAAGCATTCAGCCCCTTAATGAAAAACTCTTTGGCAACTGG
TGTTAGGGAAAGAAAAAACAAACCAGCAGCTTATTATTAGTTCCACAGCAAGTTAATTAAT
TTTTGCCAGTCACTTTGGGTCACTTGCTGGACTGAGCATCTGTCTCACTCAACTGAACAGC
TGGAGGGATGGTGGGTGTCTCTGCGGGCCATGCATGTGTCCTGTTCCGGGAGGAGAAGGGA
GAGCTAACTACAAGGCTGAAAAAACGTGAGCTTCCAGTGTTCATCTGCTCCTCTCCTGT

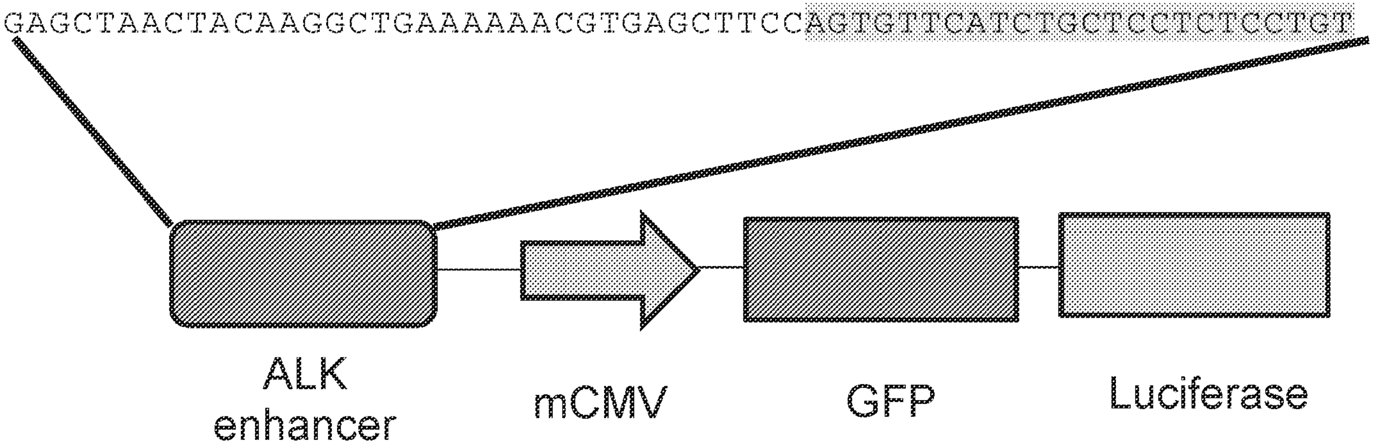


FIG. 1

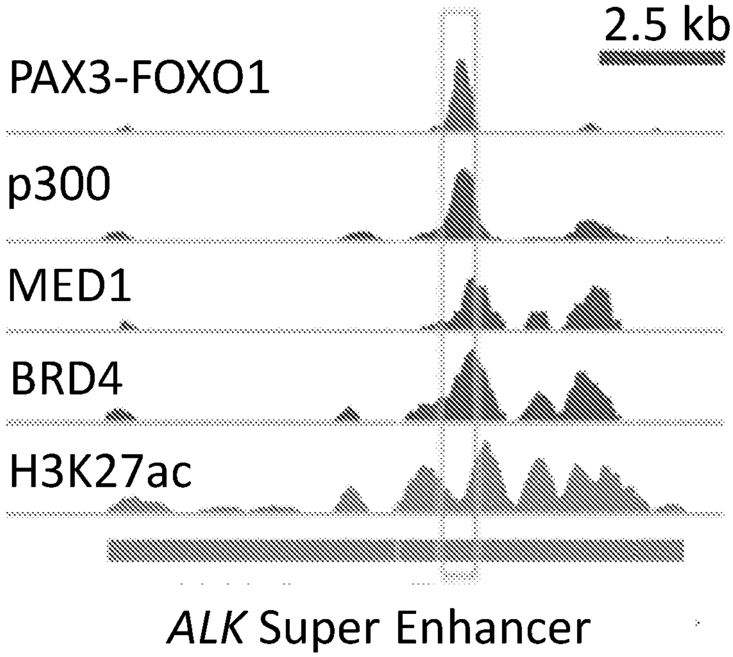


FIG. 2

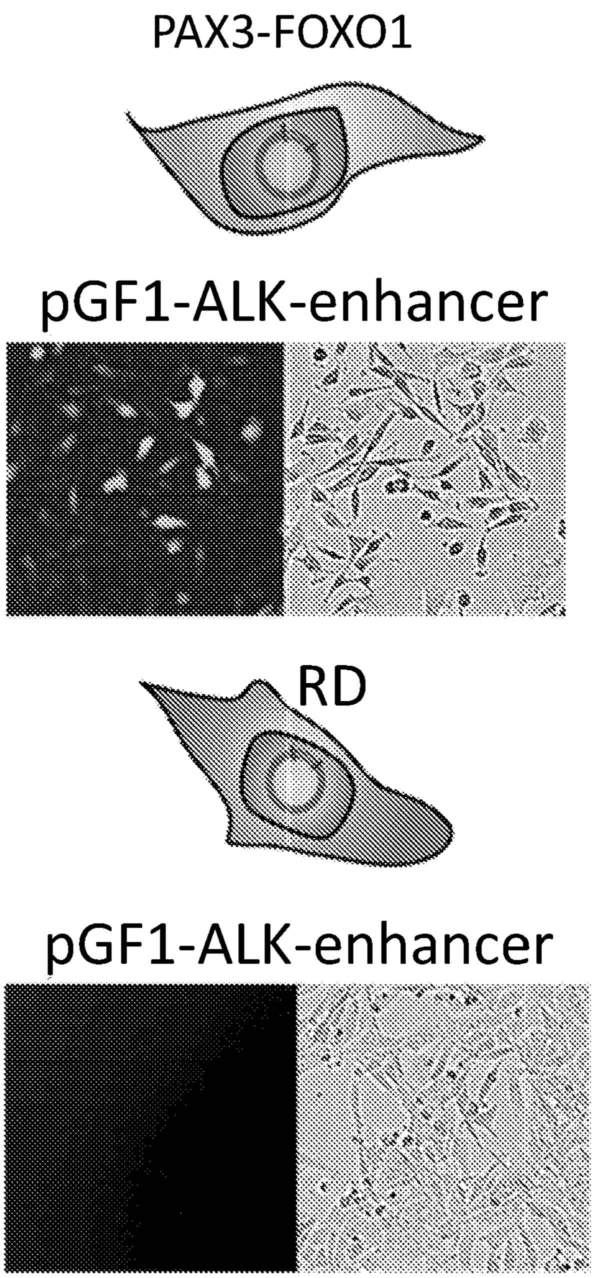


FIG. 3

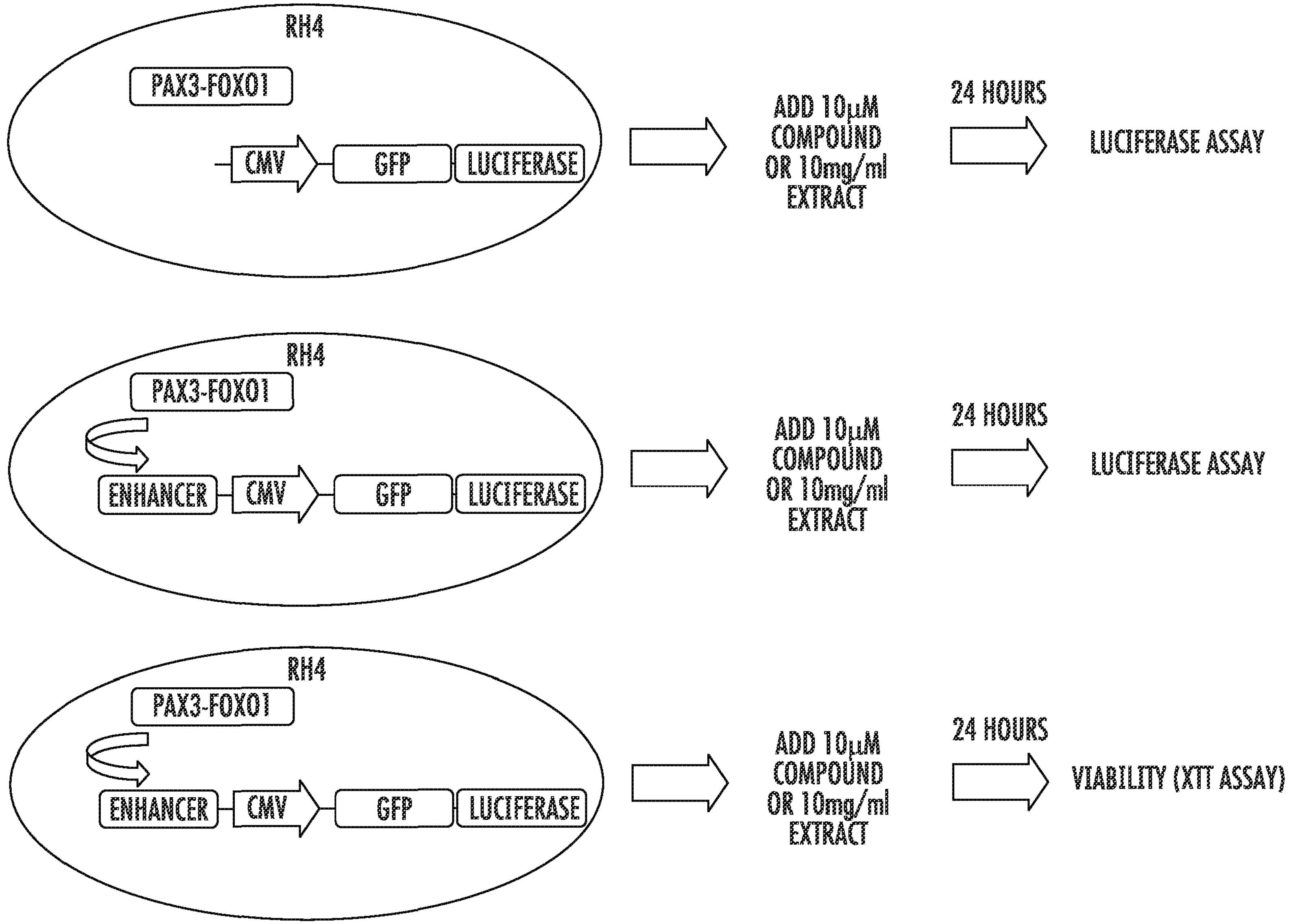
RH4
PAX3-FOXO1
CMV
GFP
LUCIFERASE
ADD 10μM COMPOUND OR 10mg/ml EXTRACT
24 HOURS
LUCIFERASE ASSAY
RH4
PAX3-FOXO1
ENHANCER
CMV
GFP
LUCIFERASE
ADD 10μM COMPOUND OR 10mg/ml EXTRACT
24 HOURS
LUCIFERASE ASSAY
RH4
PAX3-FOXO1
ENHANCER
CMV
GFP
LUCIFERASE
ADD 10μM COMPOUND OR 10mg/ml EXTRACT
24 HOURS
VIABILITY (XTT ASSAY)

FIG. 4

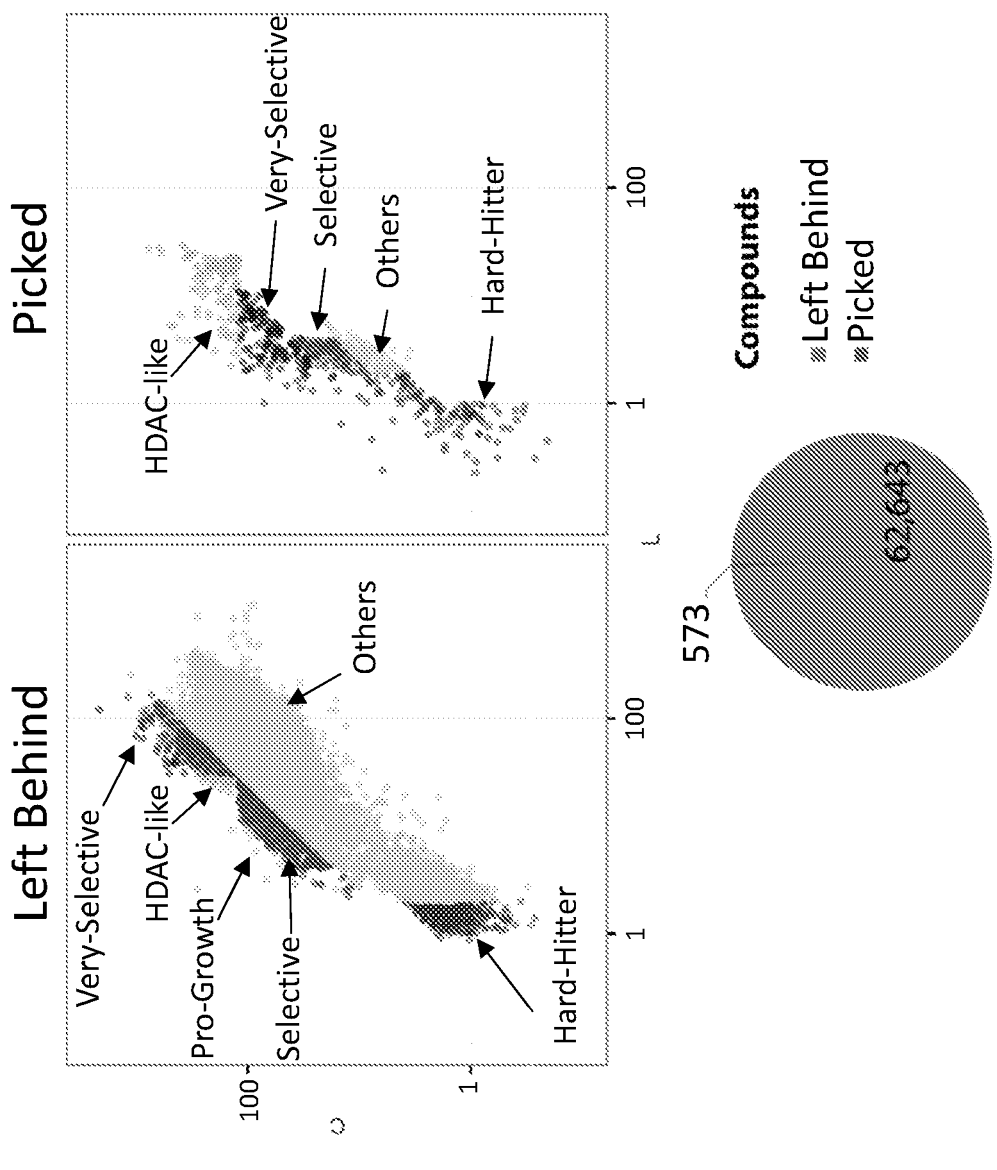
Left Behind
Very-Selective
HDAC-like
Pro-Growth
Selective
Others
Hard-Hitter
100
1
Picked
HDAC-like
Very-Selective
Selective
Others
Hard-Hitter
100
1
573
62,643
Compounds
Left Behind
Picked

FIG. 5

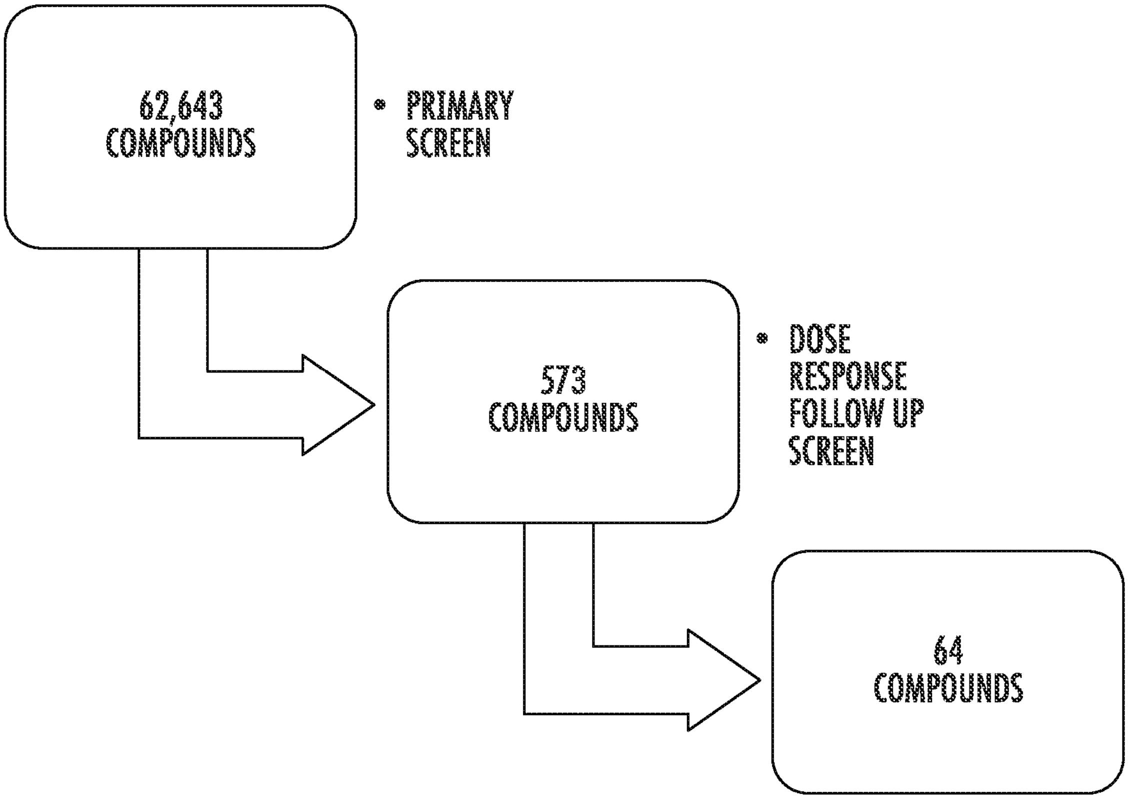
62,643
COMPOUNDS
• PRIMARY
SCREEN
573
COMPOUNDS
• DOSE
RESPONSE
FOLLOW UP
SCREEN
64
COMPOUNDS

FIG. 6

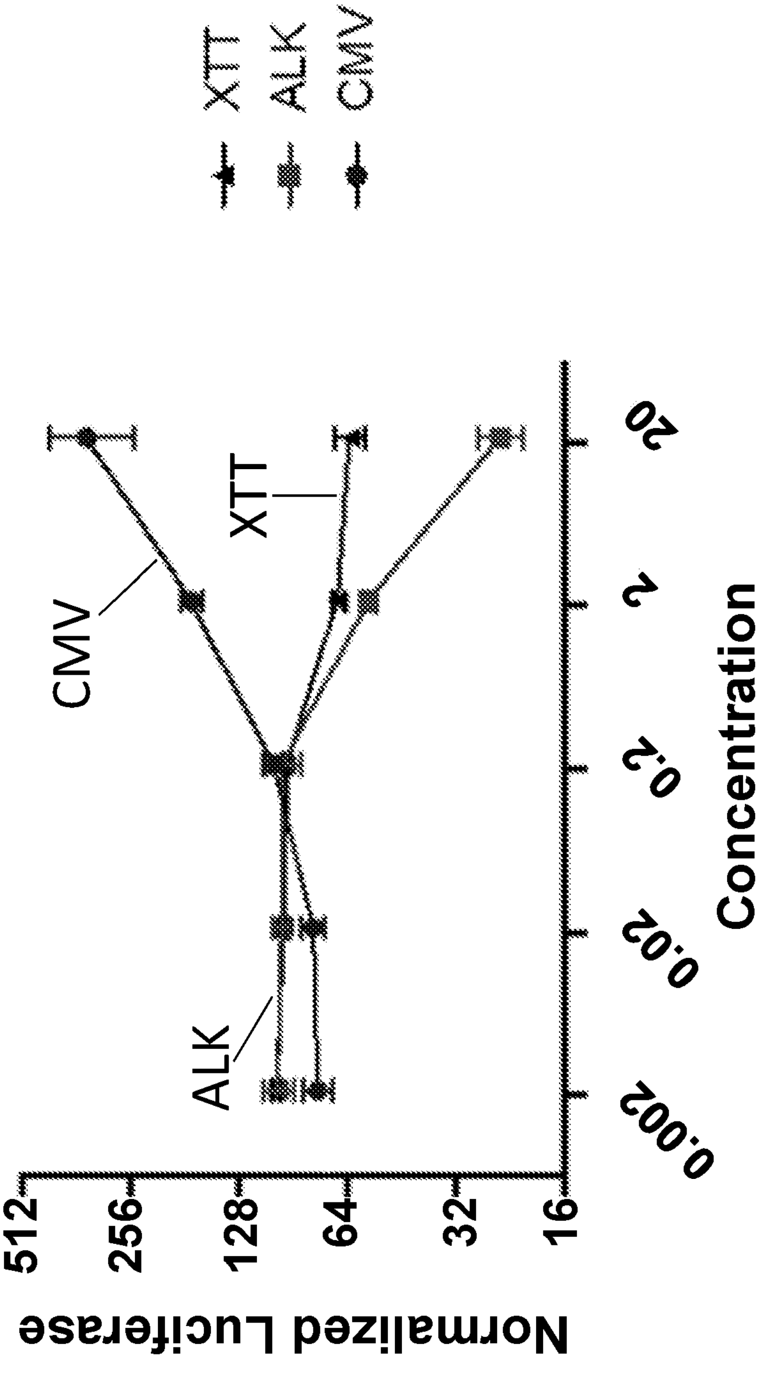
XTT
ALK
CMV
Normalized Luciferase
512
256
128
64
32
16
CMV
XTT
ALK
0.002
0.02
0.2
2
20
Concentration

FIG. 7

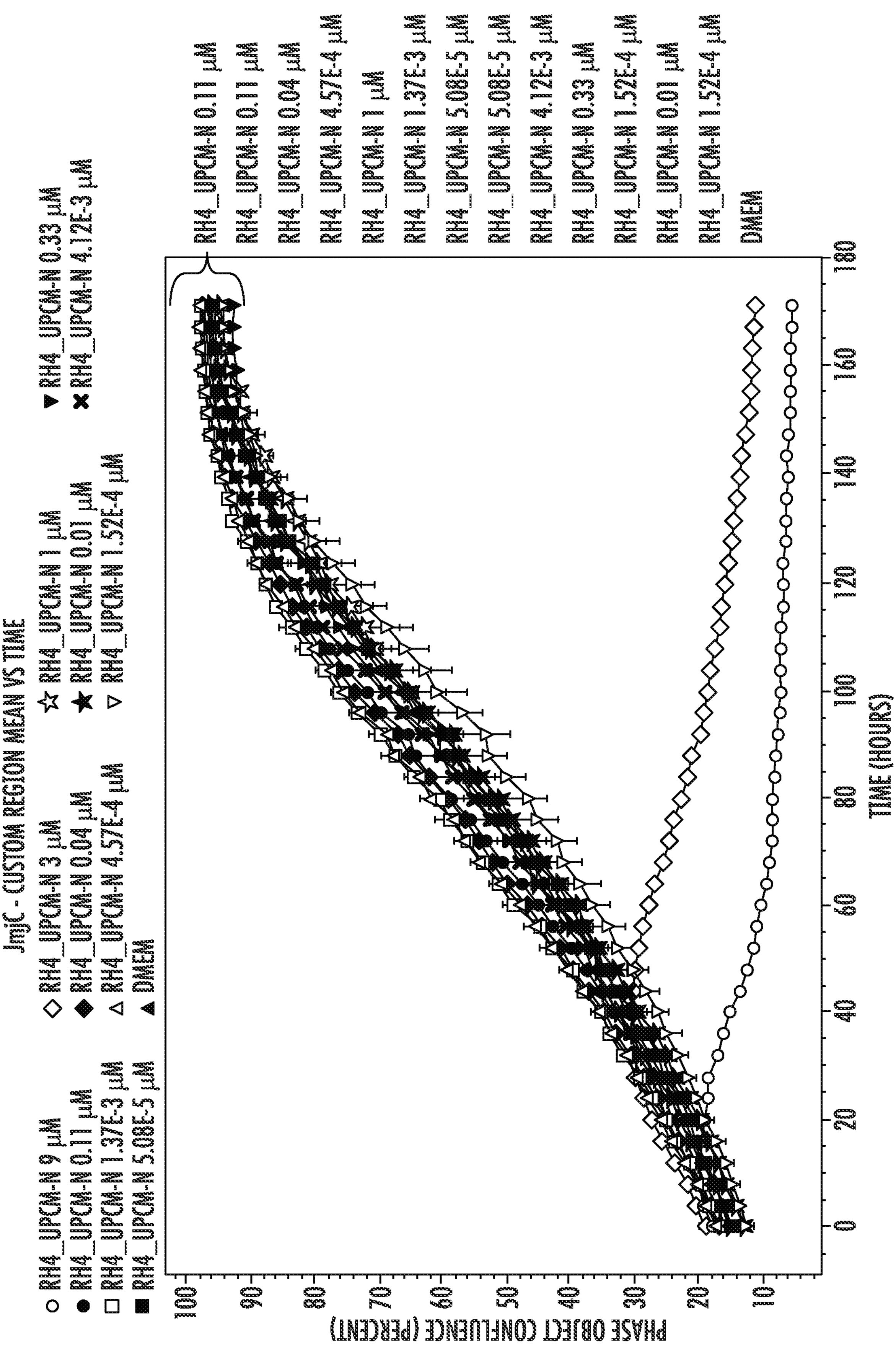


$IC_{50}$: RH4SEQ, RH4SEQ, (~2.5 µM)

FIG. 8

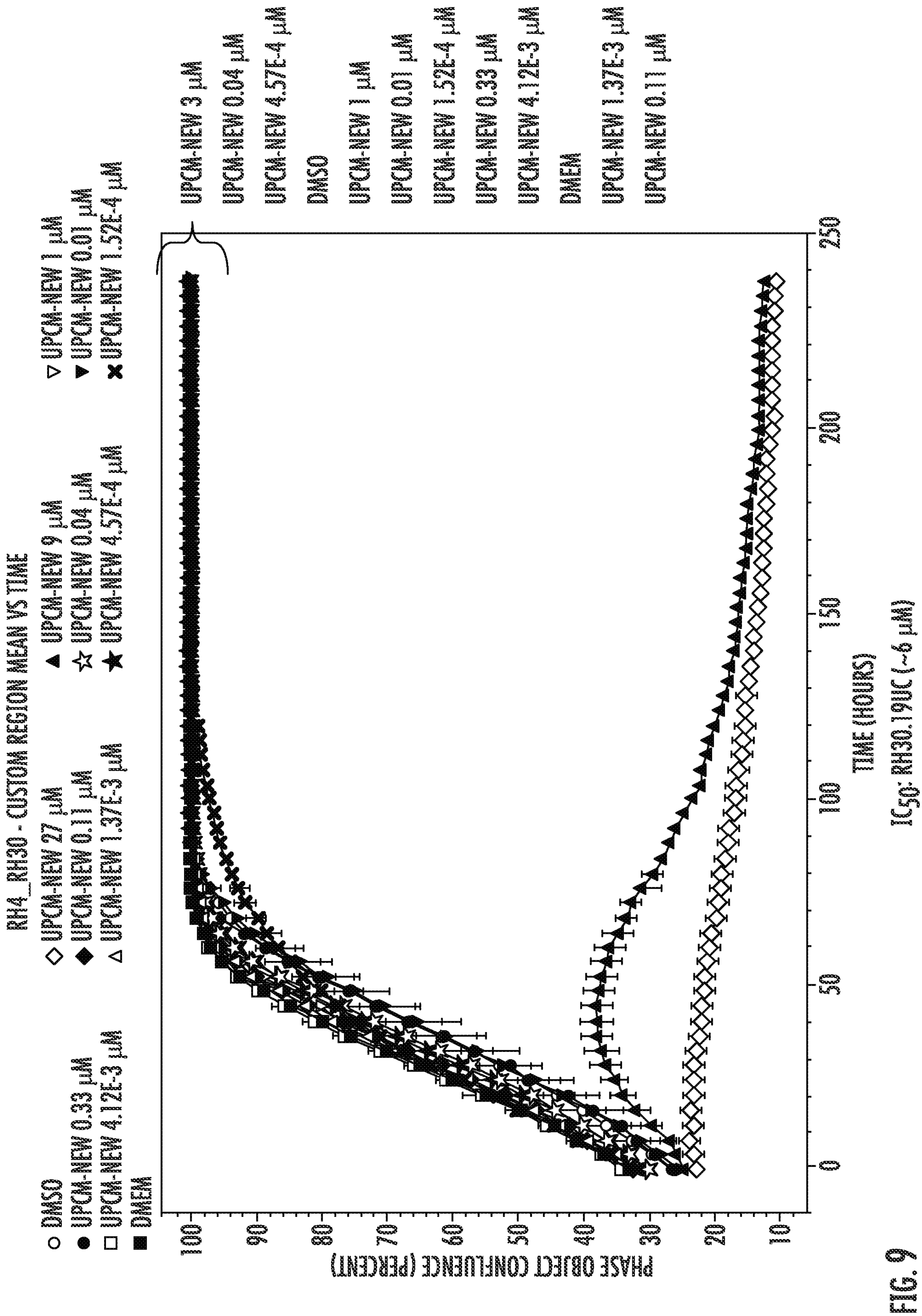
RH4_RH30 - CUSTOM REGION MEAN VS TIME
DMSO
UPCM-NEW 0.33 μM
UPCM-NEW 4.12E-3 μM
DMEM
UPCM-NEW 27 μM
UPCM-NEW 0.11 μM
UPCM-NEW 1.37E-3 μM
UPCM-NEW 9 μM
UPCM-NEW 0.04 μM
UPCM-NEW 4.57E-4 μM
UPCM-NEW 1 μM
UPCM-NEW 0.01 μM
UPCM-NEW 1.52E-4 μM
UPCM-NEW 3 μM
UPCM-NEW 0.04 μM
UPCM-NEW 4.57E-4 μM
DMSO
UPCM-NEW 1 μM
UPCM-NEW 0.01 μM
UPCM-NEW 1.52E-4 μM
UPCM-NEW 0.33 μM
UPCM-NEW 4.12E-3 μM
DMEM
UPCM-NEW 1.37E-3 μM
UPCM-NEW 0.11 μM
PHASE OBJECT CONFLUENCE (PERCENT)
100
90
80
70
60
50
40
30
20
10
0
50
100
150
200
250
TIME (HOURS)
$IC_{50}$: RH30.19UC (~6 μM)

FIG. 9

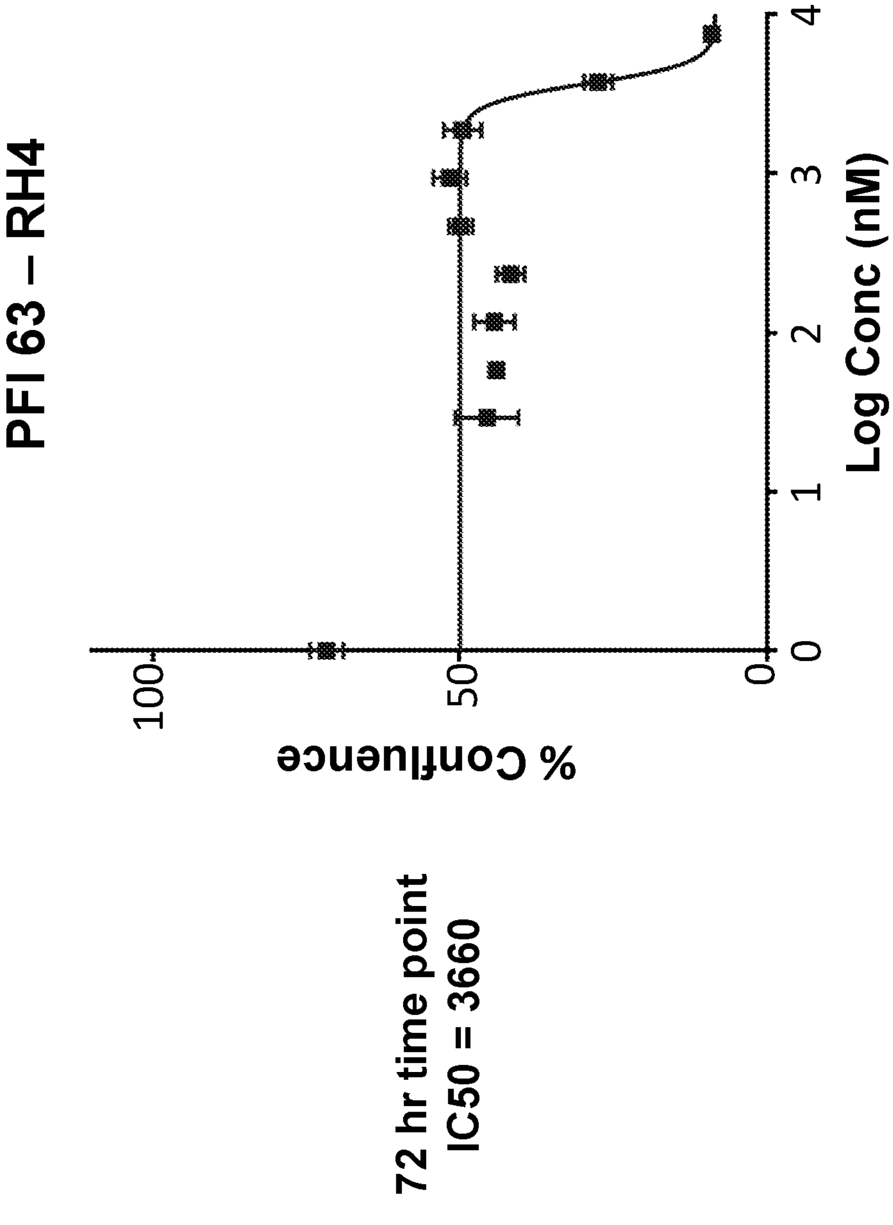
PFI 63 – RH4
% Confluence
100
50
0
0
1
2
3
4
Log Conc (nM)
72 hr time point
IC50 = 3660

FIG. 10

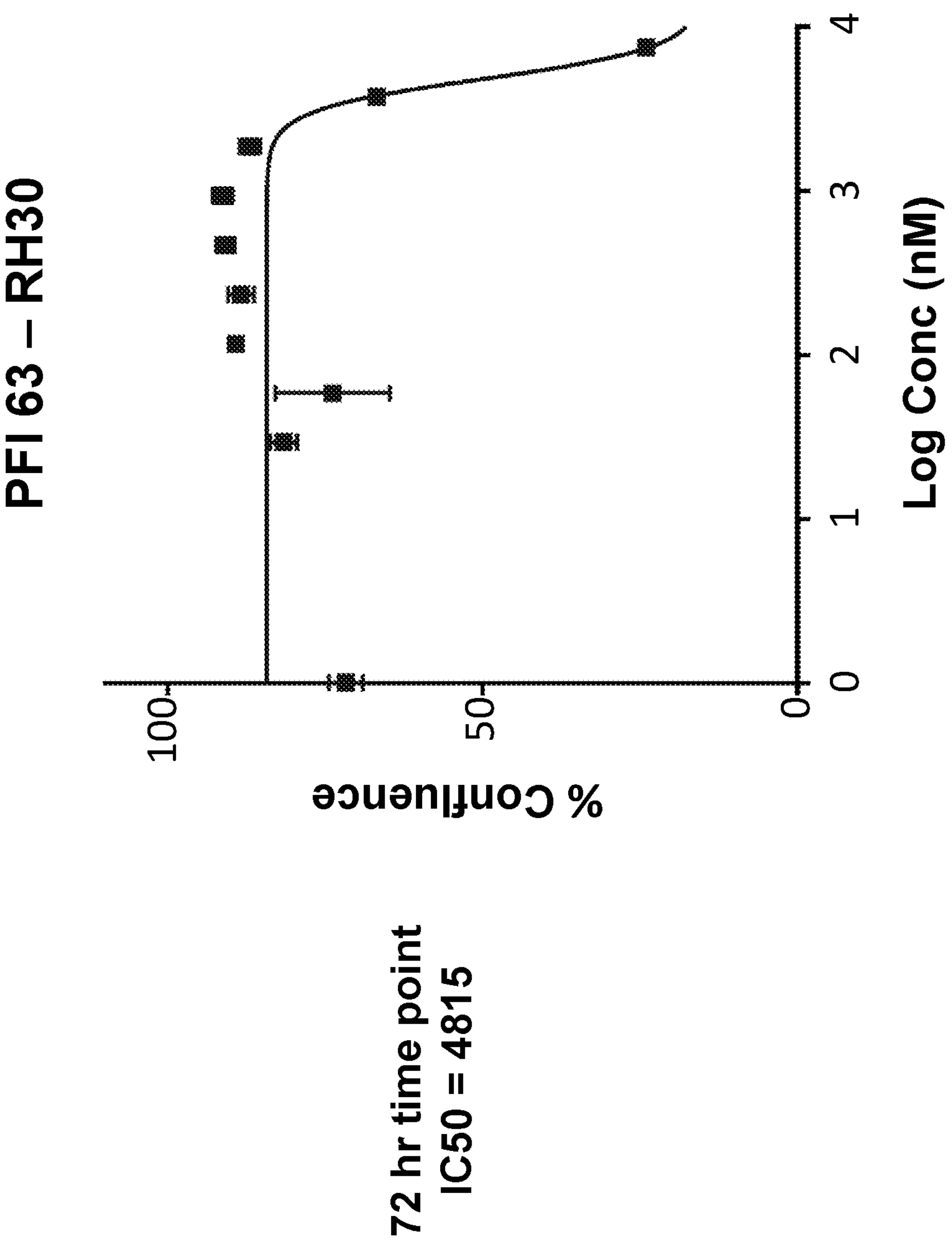
PFI 63 – RH30
% Confluence
100
50
0
0
1
2
3
4
Log Conc (nM)
72 hr time point
IC50 = 4815

FIG. 11

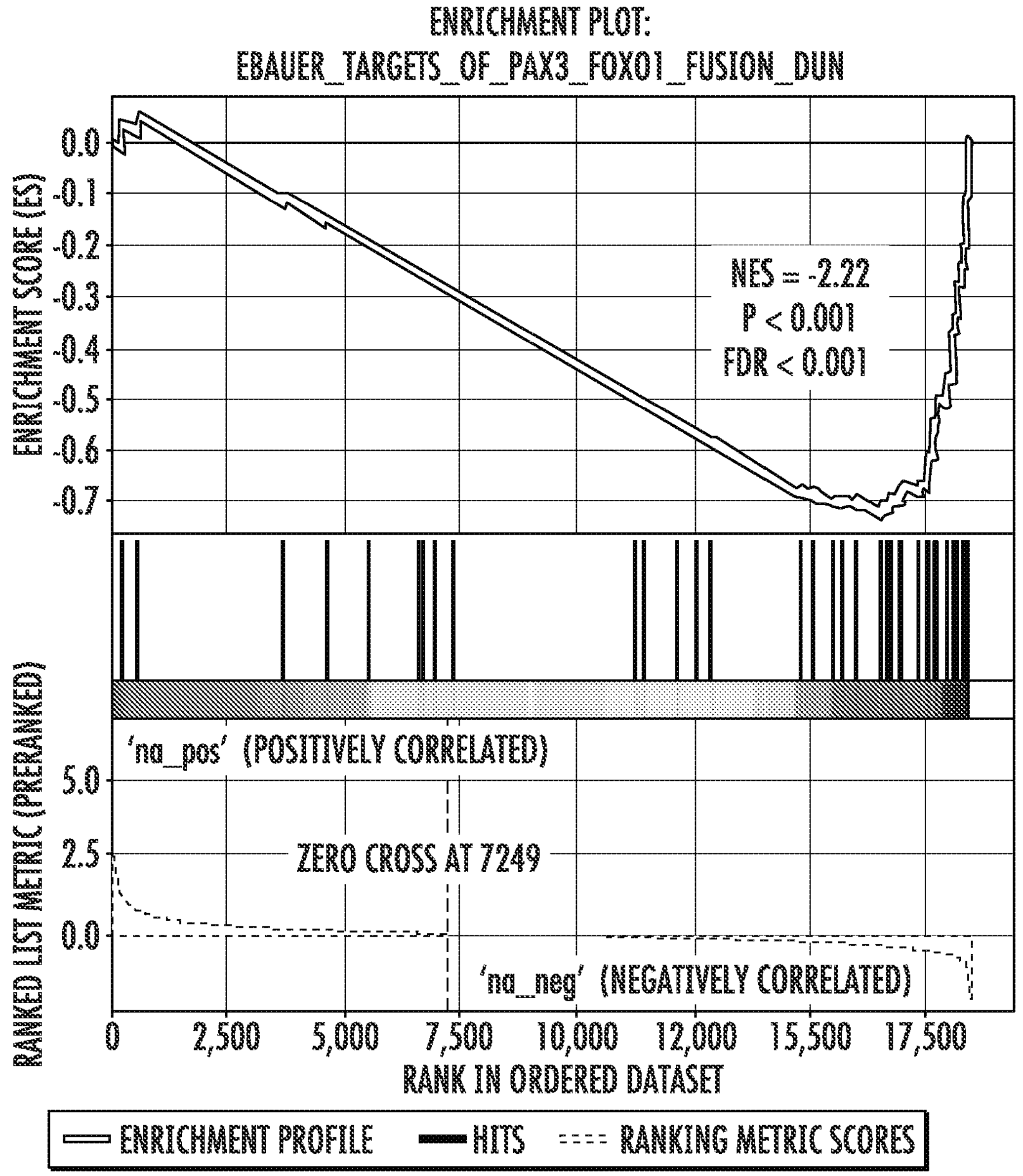
ENRICHMENT PLOT:
EBAUER_TARGETS_OF_PAX3_FOXO1_FUSION_DUN
ENRICHMENT SCORE (ES)
0.0
-0.1
-0.2
-0.3
-0.4
-0.5
-0.6
-0.7
NES = -2.22
P < 0.001
FDR < 0.001
RANKED LIST METRIC (PRERANKED)
'na_pos' (POSITIVELY CORRELATED)
5.0
2.5
0.0
ZERO CROSS AT 7249
'na_neg' (NEGATIVELY CORRELATED)
0
2,500
5,000
7,500
10,000
12,000
15,500
17,500
RANK IN ORDERED DATASET
ENRICHMENT PROFILE
HITS
RANKING METRIC SCORES

FIG. 12

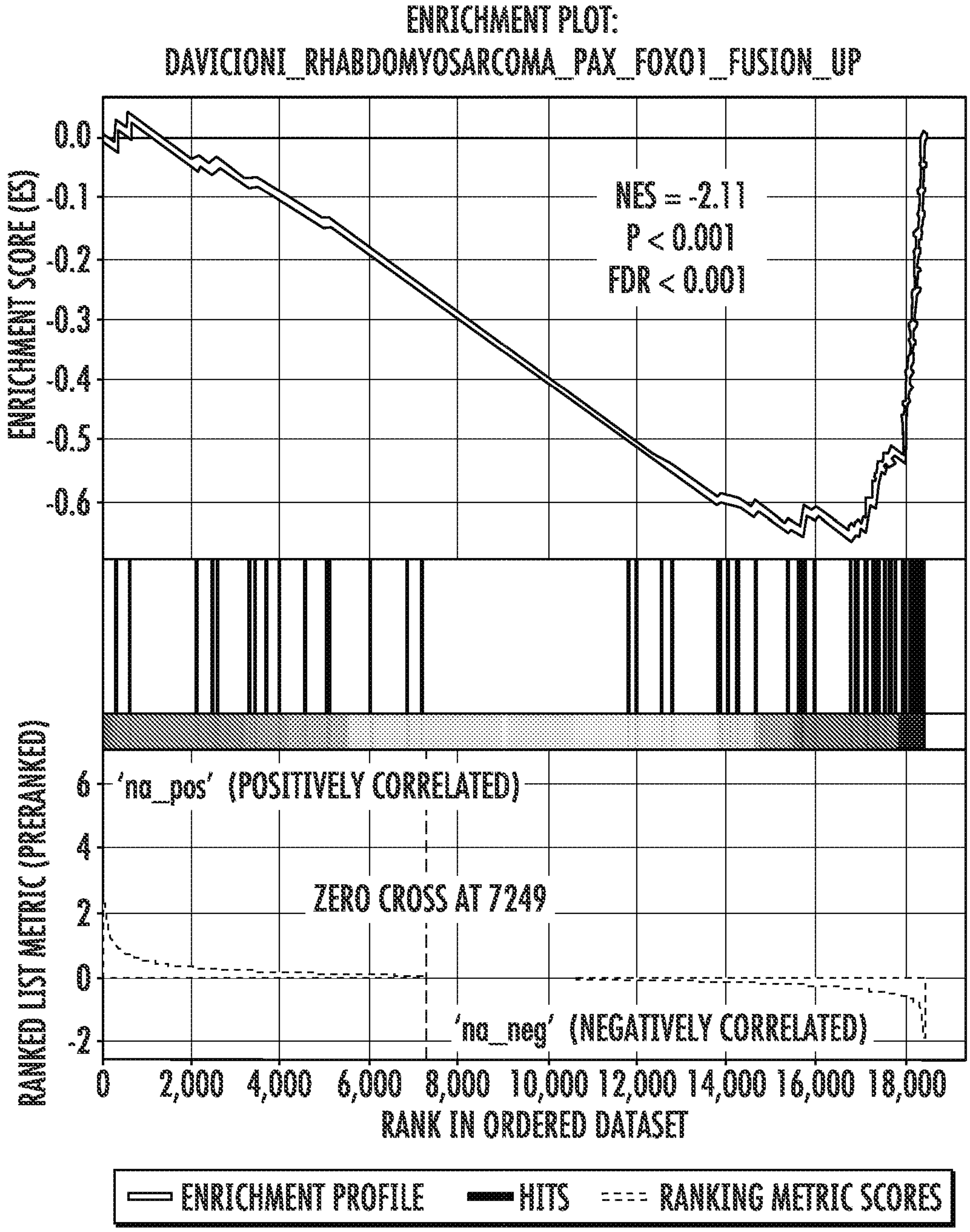
ENRICHMENT PLOT:
DAVICIONI_RHABDOMYOSARCOMA_PAX_FOXO1_FUSION_UP
ENRICHMENT SCORE (ES)
0.0
-0.1
-0.2
-0.3
-0.4
-0.5
-0.6
NES = -2.11
P < 0.001
FDR < 0.001
RANKED LIST METRIC (PRERANKED)
6
4
2
0
-2
'na_pos' (POSITIVELY CORRELATED)
ZERO CROSS AT 7249
'na_neg' (NEGATIVELY CORRELATED)
0
2,000
4,000
6,000
8,000
10,000
12,000
14,000
16,000
18,000
RANK IN ORDERED DATASET
ENRICHMENT PROFILE
HITS
RANKING METRIC SCORES

FIG. 13

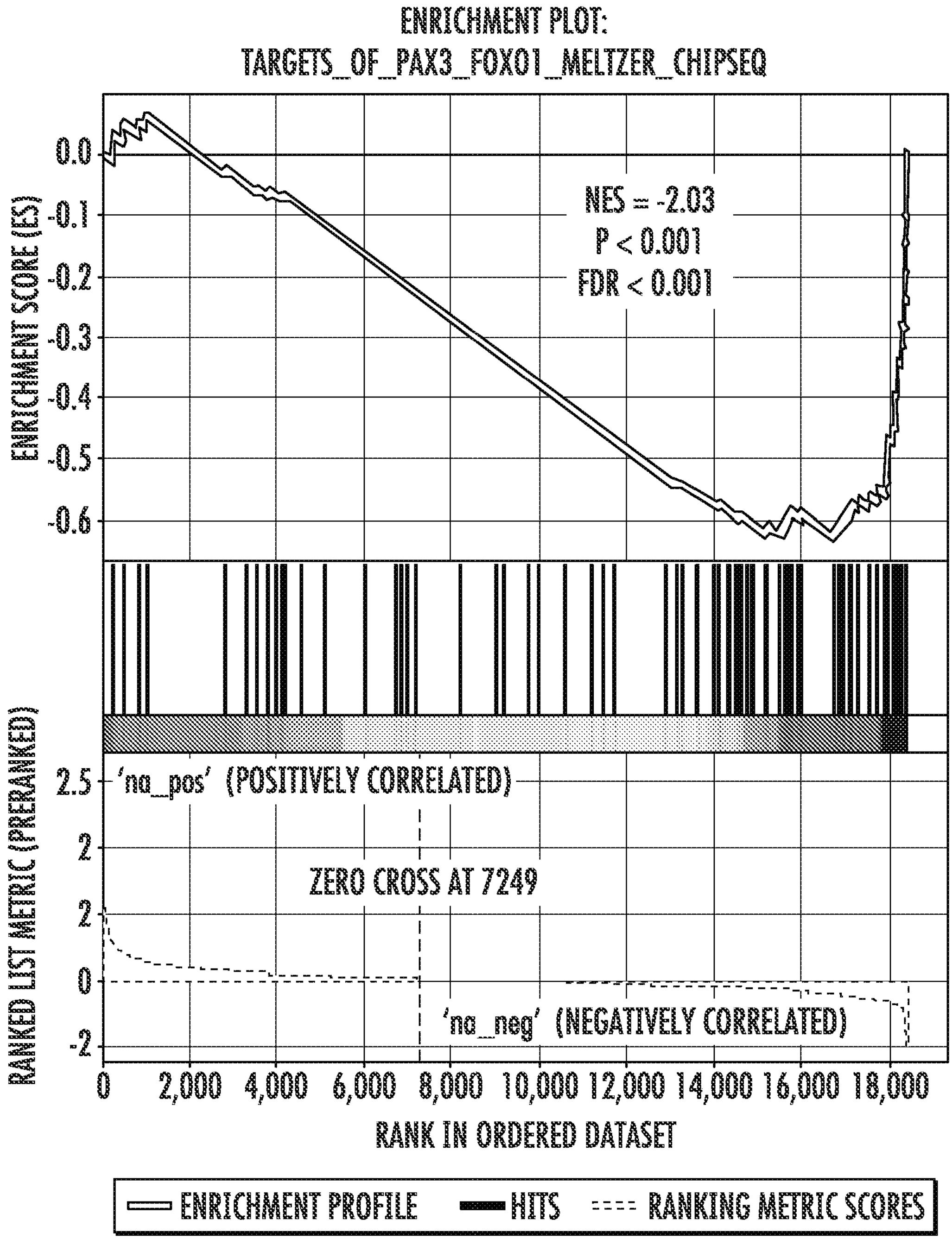
ENRICHMENT PLOT:
TARGETS_OF_PAX3_FOXO1_MELTZER_CHIPSEQ
ENRICHMENT SCORE (ES)
0.0
-0.1
-0.2
-0.3
-0.4
-0.5
-0.6
NES = -2.03
P < 0.001
FDR < 0.001
RANKED LIST METRIC (PRERANKED)
2.5
2
2
0
-2
'na_pos' (POSITIVELY CORRELATED)
ZERO CROSS AT 7249
'na_neg' (NEGATIVELY CORRELATED)
0
2,000
4,000
6,000
8,000
10,000
12,000
14,000
16,000
18,000
RANK IN ORDERED DATASET
ENRICHMENT PROFILE
HITS
RANKING METRIC SCORES

FIG. 14

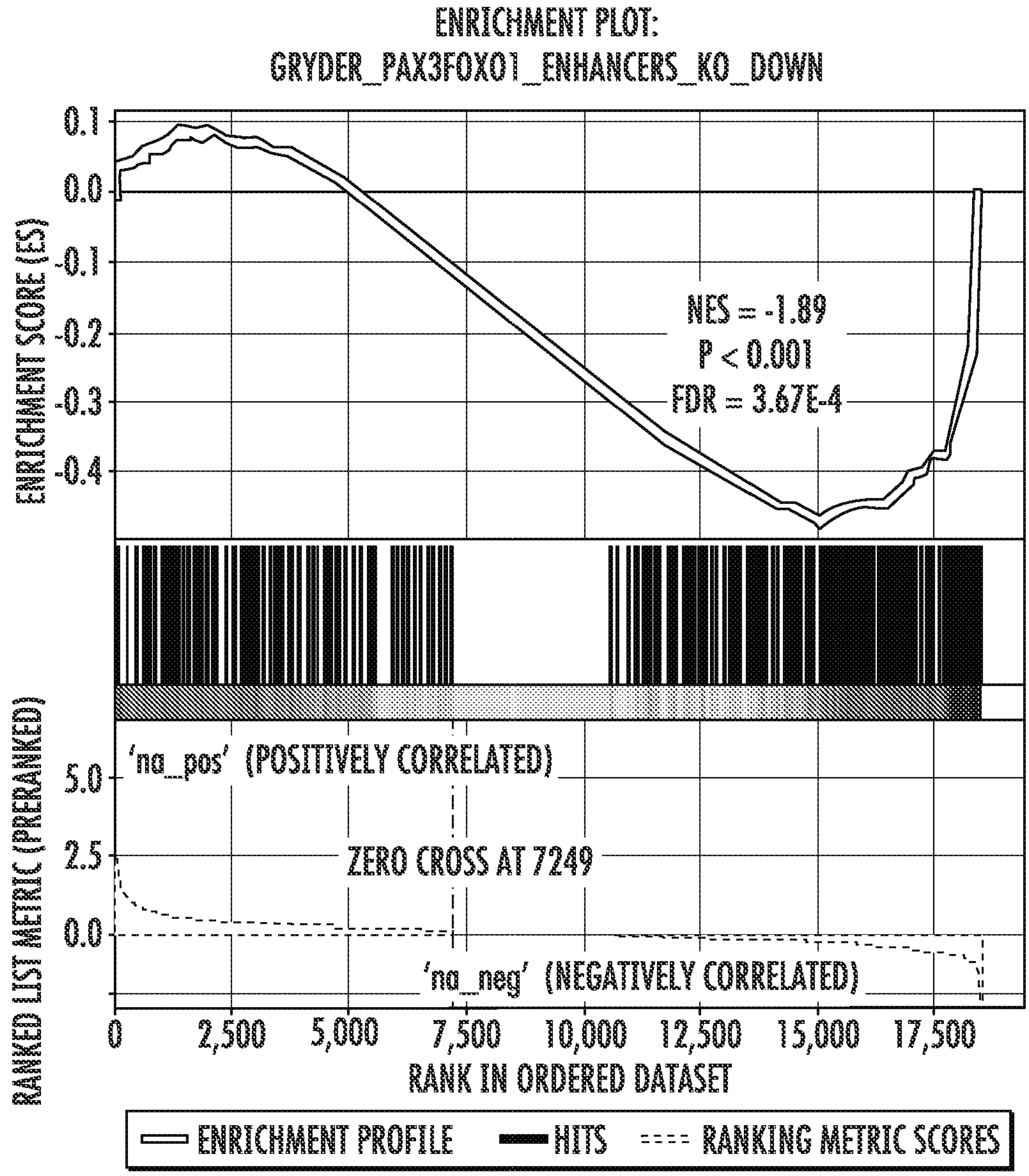
ENRICHMENT PLOT:
GRYDER_PAX3FOXO1_ENHANCERS_KO_DOWN
ENRICHMENT SCORE (ES)
0.1
0.0
-0.1
-0.2
-0.3
-0.4
NES = -1.89
P < 0.001
FDR = 3.67E-4
RANKED LIST METRIC (PRERANKED)
5.0
2.5
0.0
'na_pos' (POSITIVELY CORRELATED)
ZERO CROSS AT 7249
'na_neg' (NEGATIVELY CORRELATED)
0
2,500
5,000
7,500
10,000
12,500
15,000
17,500
RANK IN ORDERED DATASET
ENRICHMENT PROFILE
HITS
RANKING METRIC SCORES

FIG. 15

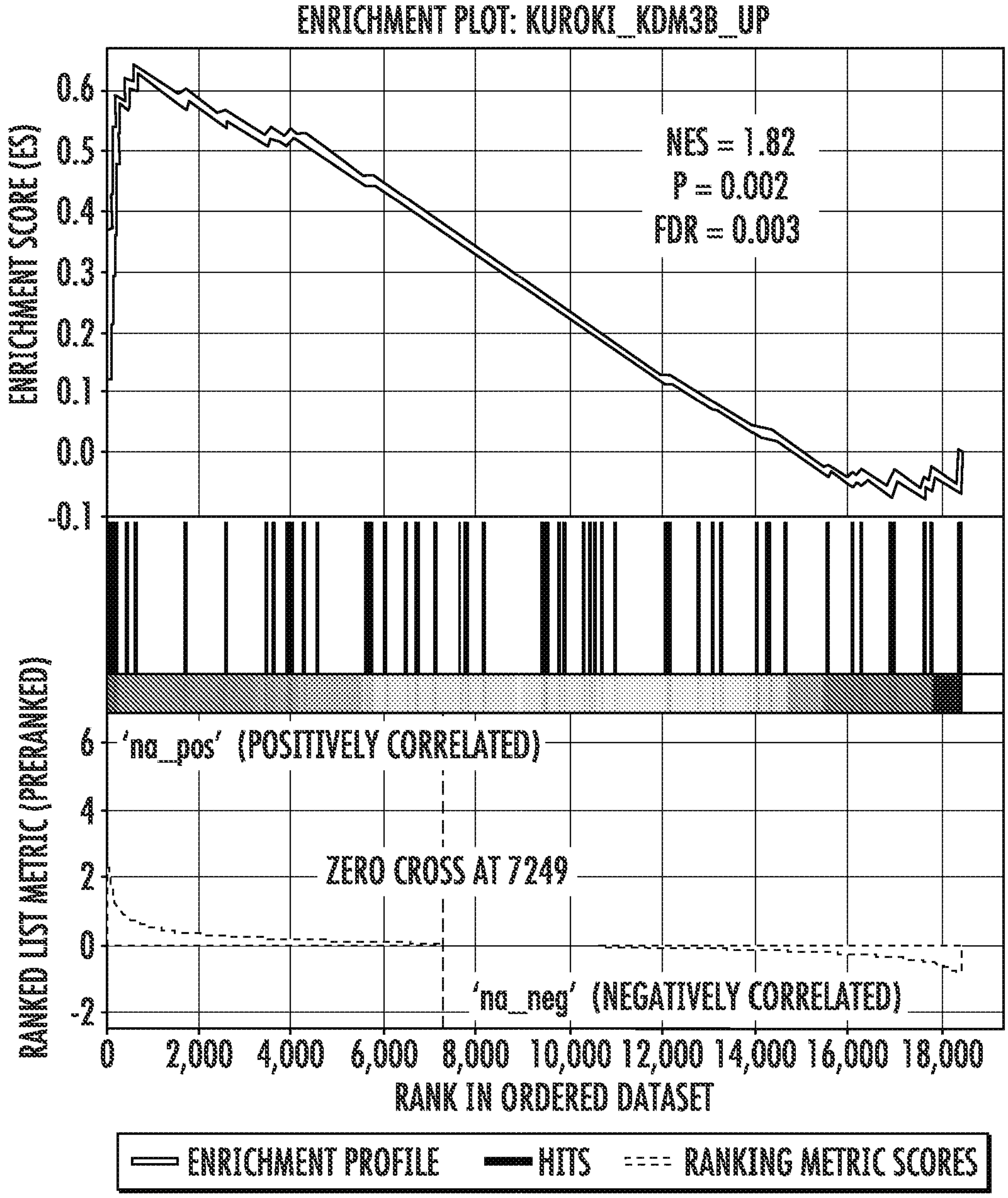
ENRICHMENT PLOT: KUROKI_KDM3B_UP
ENRICHMENT SCORE (ES)
0.6
0.5
0.4
0.3
0.2
0.1
0.0
-0.1
NES = 1.82
P = 0.002
FDR = 0.003
RANKED LIST METRIC (PRERANKED)
6
4
2
0
-2
'na_pos' (POSITIVELY CORRELATED)
ZERO CROSS AT 7249
'na_neg' (NEGATIVELY CORRELATED)
0
2,000
4,000
6,000
8,000
10,000
12,000
14,000
16,000
18,000
RANK IN ORDERED DATASET
ENRICHMENT PROFILE
HITS
RANKING METRIC SCORES

FIG. 16

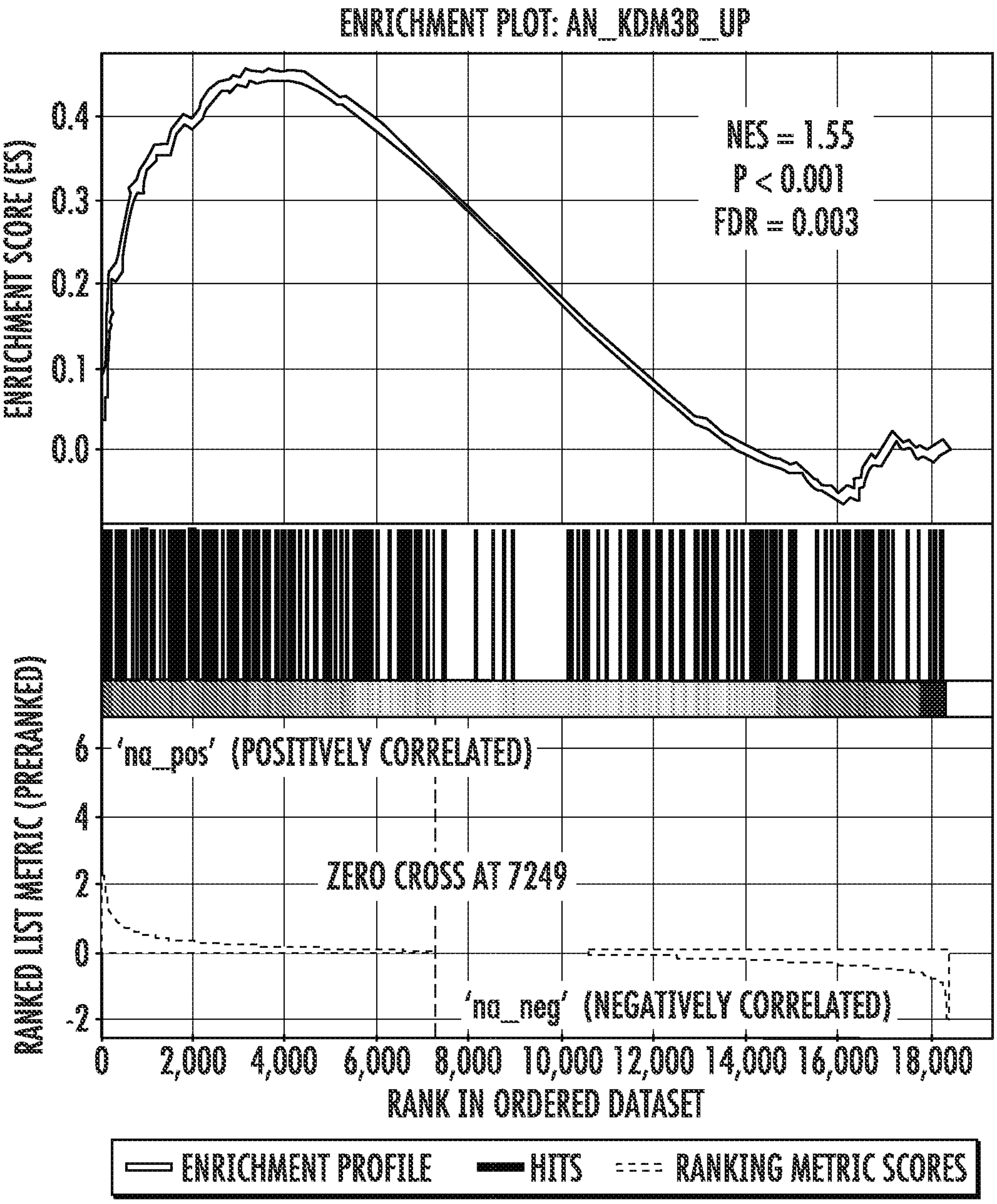
ENRICHMENT PLOT: AN_KDM3B_UP
ENRICHMENT SCORE (ES)
0.4
0.3
0.2
0.1
0.0
NES = 1.55
P < 0.001
FDR = 0.003
RANKED LIST METRIC (PRERANKED)
6
4
2
0
-2
'na_pos' (POSITIVELY CORRELATED)
ZERO CROSS AT 7249
'na_neg' (NEGATIVELY CORRELATED)
0 2,000 4,000 6,000 8,000 10,000 12,000 14,000 16,000 18,000
RANK IN ORDERED DATASET
ENRICHMENT PROFILE
HITS
RANKING METRIC SCORES

FIG. 17

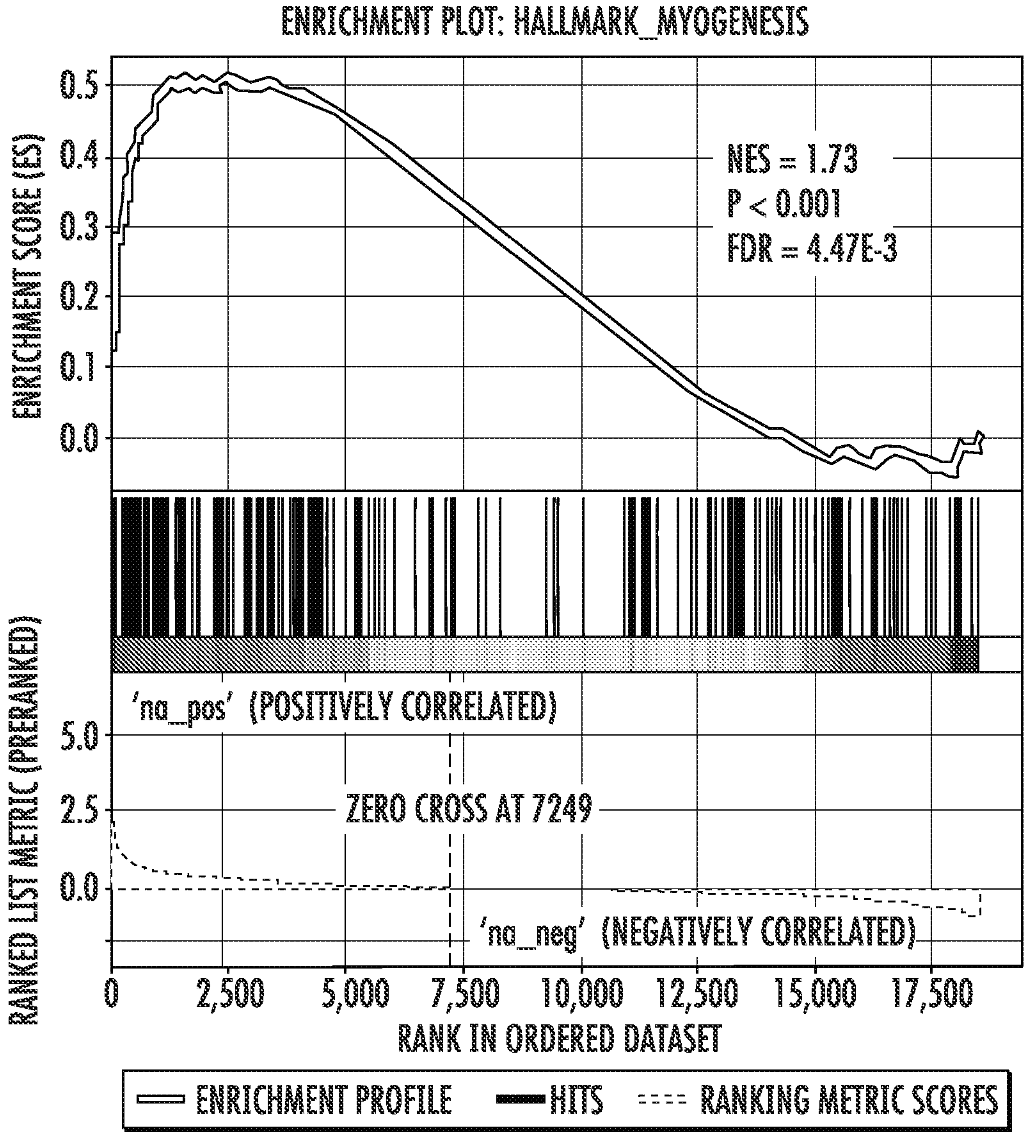
ENRICHMENT PLOT: HALLMARK_MYOGENESIS
ENRICHMENT SCORE (ES)
0.5
0.4
0.3
0.2
0.1
0.0
NES = 1.73
P < 0.001
FDR = 4.47E-3
RANKED LIST METRIC (PRERANKED)
'na_pos' (POSITIVELY CORRELATED)
5.0
2.5
0.0
ZERO CROSS AT 7249
'na_neg' (NEGATIVELY CORRELATED)
0
2,500
5,000
7,500
10,000
12,500
15,000
17,500
RANK IN ORDERED DATASET
ENRICHMENT PROFILE
HITS
RANKING METRIC SCORES

FIG. 18

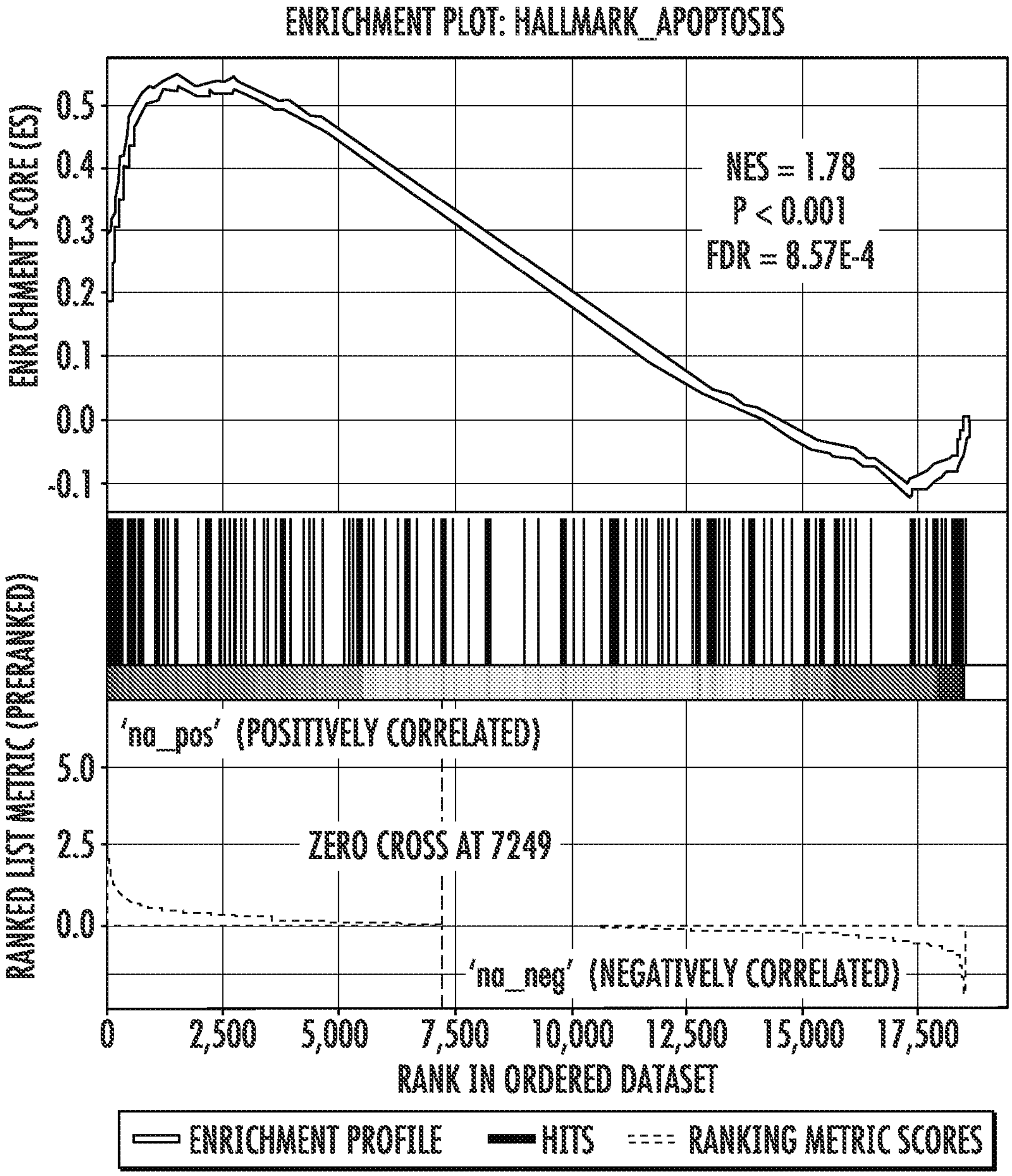
ENRICHMENT PLOT: HALLMARK_APOPTOSIS
ENRICHMENT SCORE (ES)
0.5
0.4
0.3
0.2
0.1
0.0
-0.1
NES = 1.78
P < 0.001
FDR = 8.57E-4
RANKED LIST METRIC (PRERANKED)
'na_pos' (POSITIVELY CORRELATED)
5.0
2.5
0.0
ZERO CROSS AT 7249
'na_neg' (NEGATIVELY CORRELATED)
0
2,500
5,000
7,500
10,000
12,500
15,000
17,500
RANK IN ORDERED DATASET
ENRICHMENT PROFILE
HITS
RANKING METRIC SCORES

FIG. 19

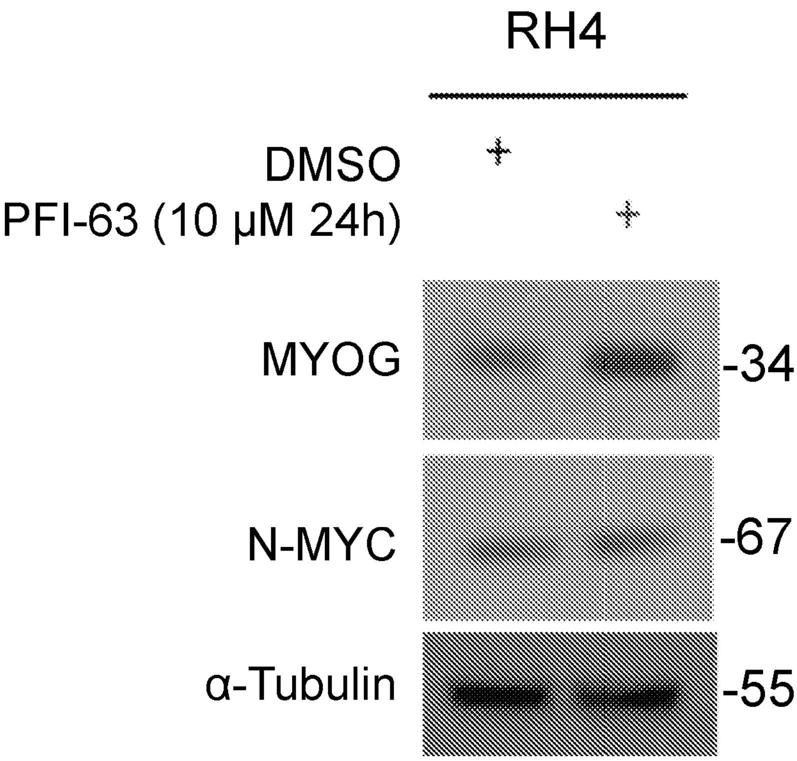


FIG. 20

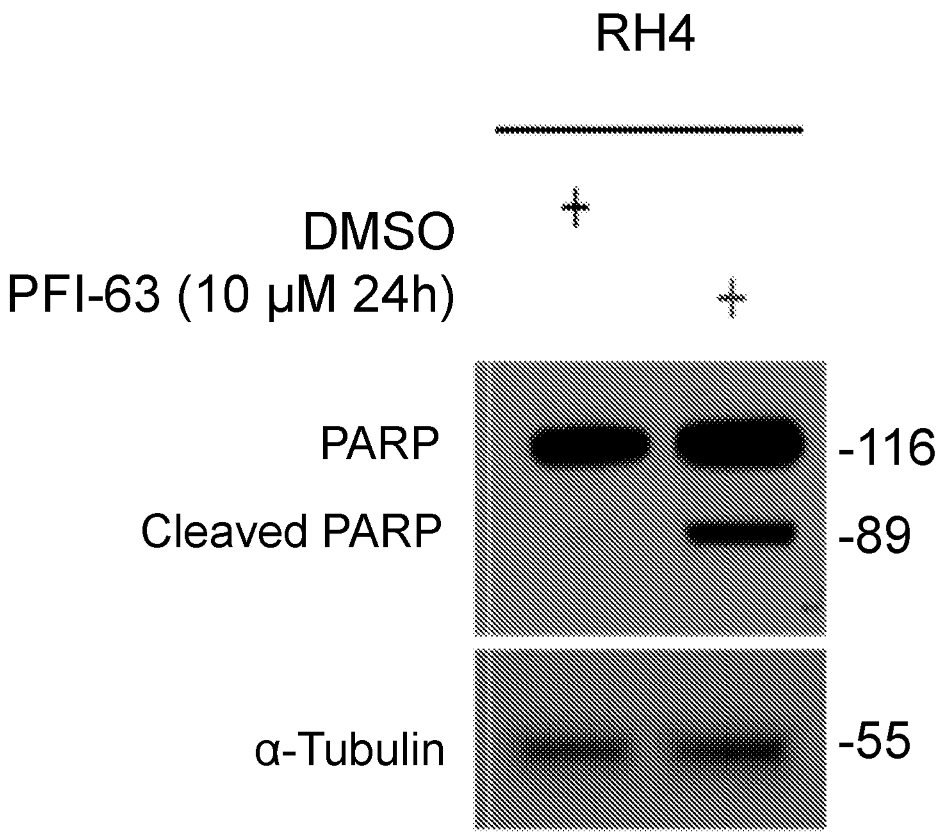


FIG. 21

| ID | NEG\|RRA | NEG\|P-VALUE | NEG\|FDR | NEG\|FDR |
|---|---|---|---|---|
| EBAUER_TARGETS_OF_PAX3_FOXO1_FUSION_UJP | 0.000374134 | 0 | 0 | 1 |
| RH4_BRD4_UP | 0.000889021 | 0.004 | 0.213333 | 5 |
| RH4_PLX1_UP | 0.00759177 | 0.015 | 0.32 | 13 |
| FOSTER_KDM1A_TARGETS_UP | 0.00705054 | 0.034 | 0.473043 | 23 |
| SP2509_LSD1i_INDUCED | 0.0100979 | 0.045 | 0.514783 | 27 |
| SCIBETTA_KDM5B_TARGETS_UP | 0.0236011 | 0.05 | 0.514783 | 28 |
| EBAUER_MYOGENIC_TARGETS_OF_PAX3_FOXO1_FUSION | 0.0307165 | 0.051 | 0.514783 | 31 |

FIG. 22

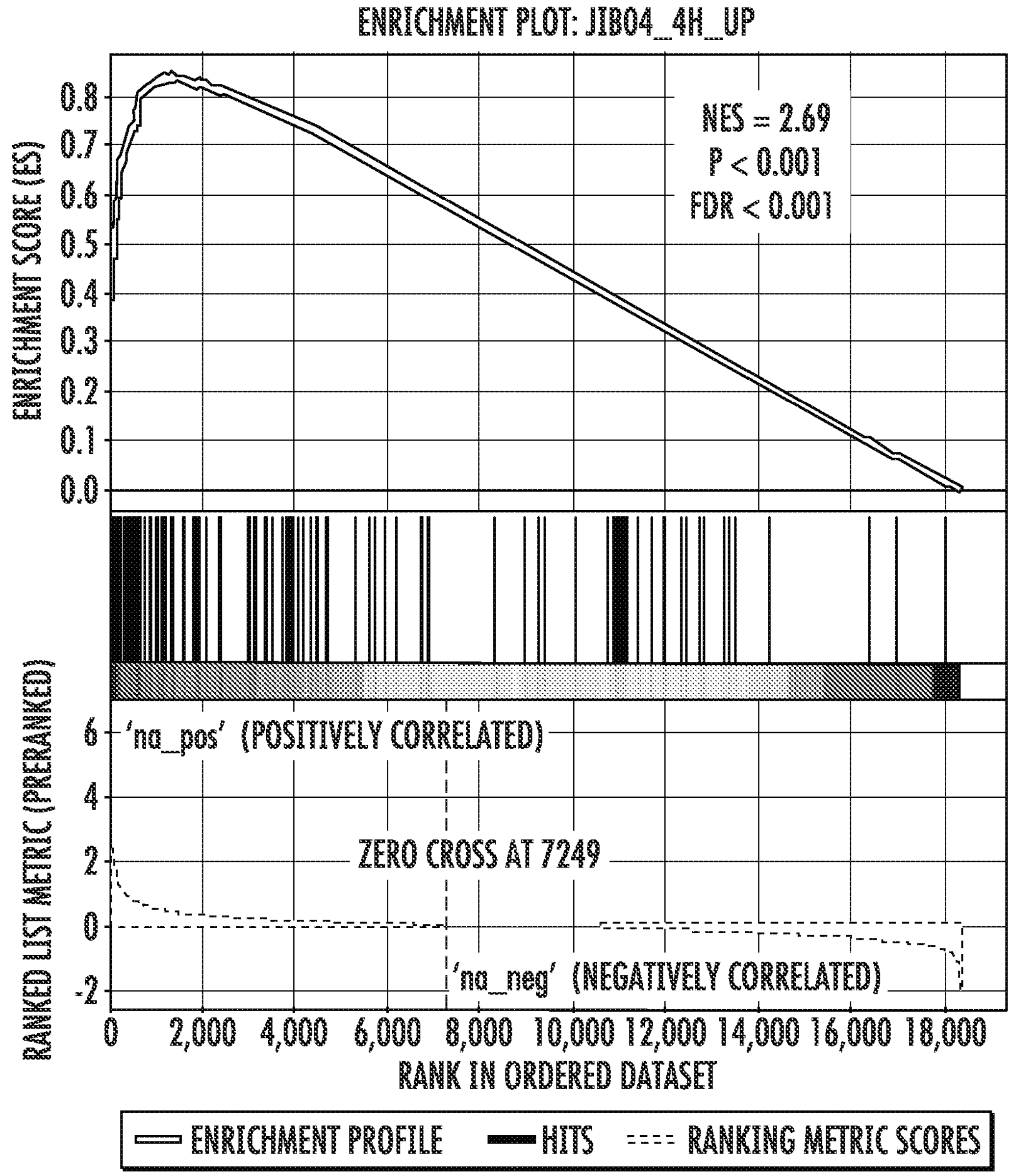
ENRICHMENT PLOT: JIB04_4H_UP
ENRICHMENT SCORE (ES)
0.8
0.7
0.6
0.5
0.4
0.3
0.2
0.1
0.0
NES = 2.69
P < 0.001
FDR < 0.001
RANKED LIST METRIC (PRERANKED)
6
4
2
0
-2
'na_pos' (POSITIVELY CORRELATED)
ZERO CROSS AT 7249
'na_neg' (NEGATIVELY CORRELATED)
0
2,000
4,000
6,000
8,000
10,000
12,000
14,000
16,000
18,000
RANK IN ORDERED DATASET
ENRICHMENT PROFILE
HITS
RANKING METRIC SCORES

FIG. 23

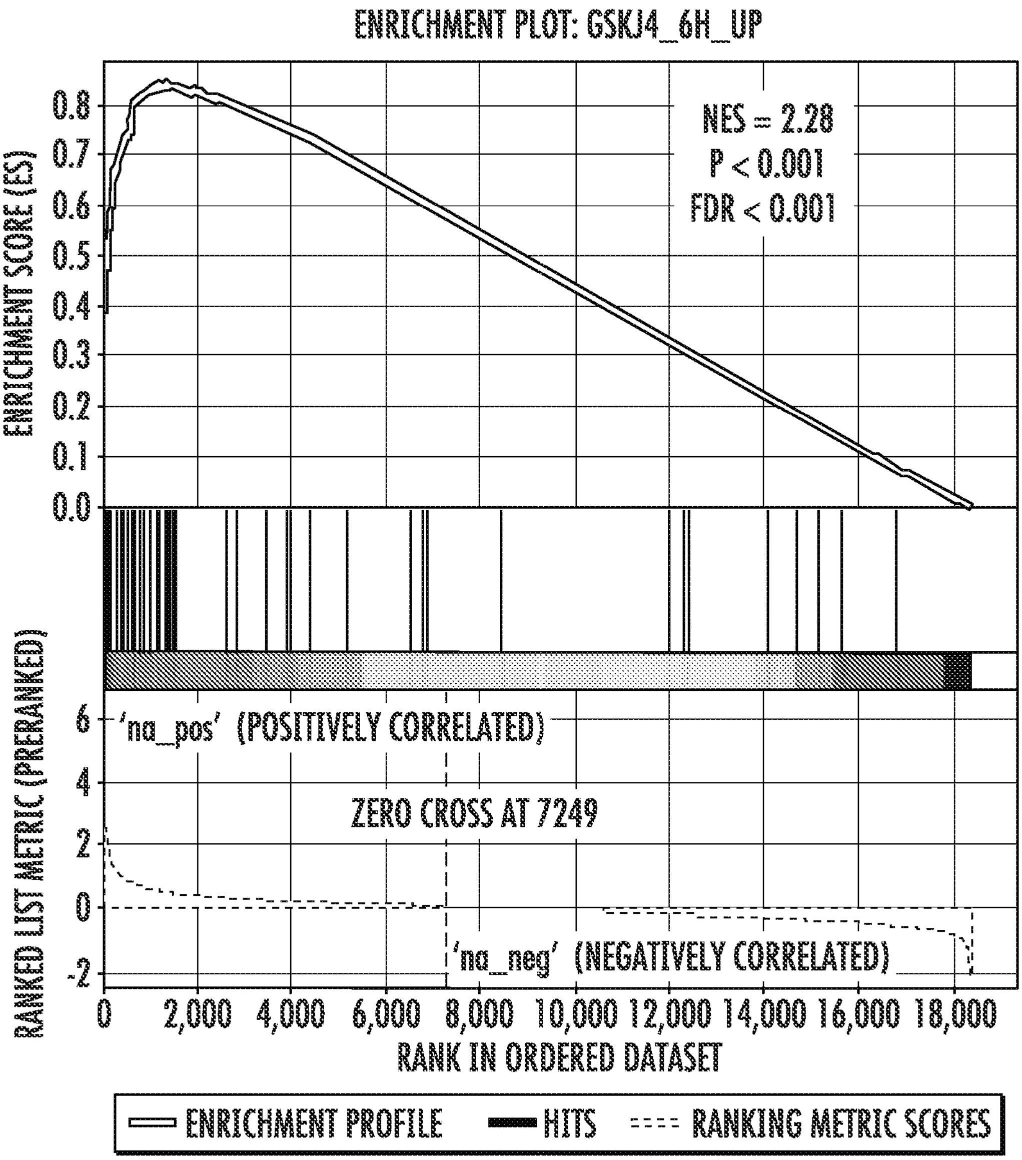
ENRICHMENT PLOT: GSKJ4_6H_UP
ENRICHMENT SCORE (ES)
0.8
0.7
0.6
0.5
0.4
0.3
0.2
0.1
0.0
NES = 2.28
P < 0.001
FDR < 0.001
RANKED LIST METRIC (PRERANKED)
6
4
2
0
-2
'na_pos' (POSITIVELY CORRELATED)
ZERO CROSS AT 7249
'na_neg' (NEGATIVELY CORRELATED)
0
2,000
4,000
6,000
8,000
10,000
12,000
14,000
16,000
18,000
RANK IN ORDERED DATASET
ENRICHMENT PROFILE
HITS
RANKING METRIC SCORES

FIG. 24

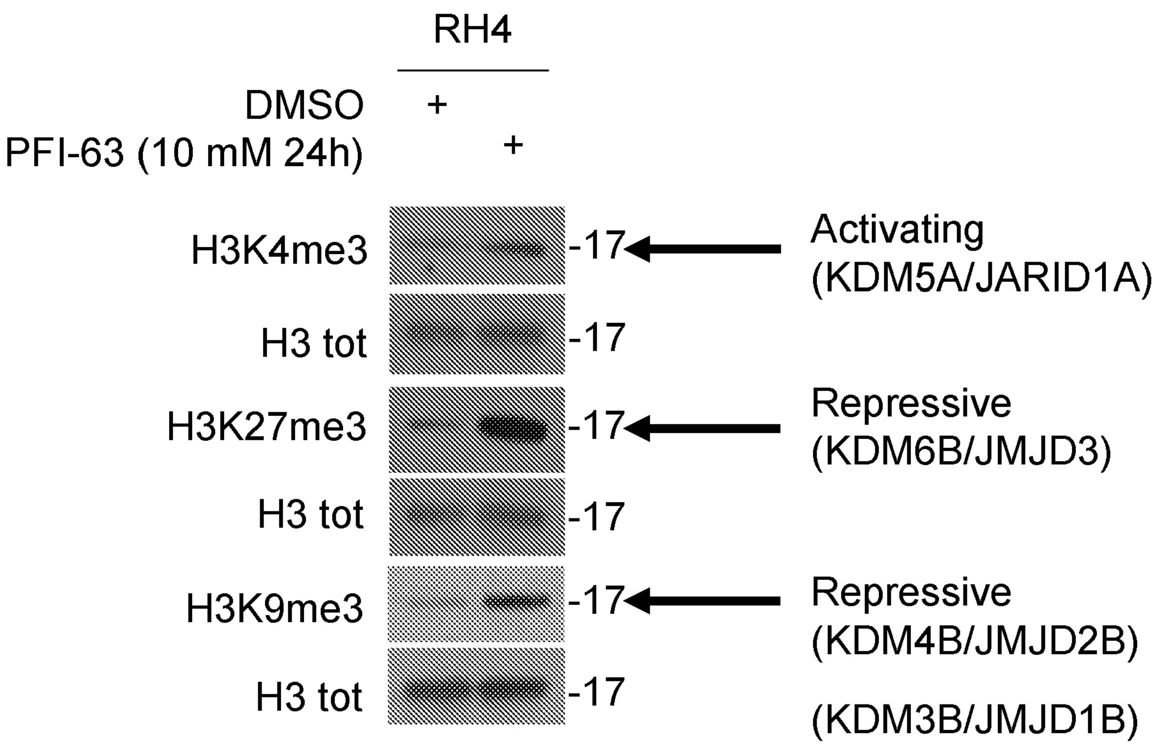
RH4
DMSO +
PFI-63 (10 mM 24h) +
H3K4me3 -17
H3 tot -17
H3K27me3 -17
H3 tot -17
H3K9me3 -17
H3 tot -17
Activating
(KDM5A/JARID1A)
Repressive
(KDM6B/JMJD3)
Repressive
(KDM4B/JMJD2B)
(KDM3B/JMJD1B)

FIG. 25

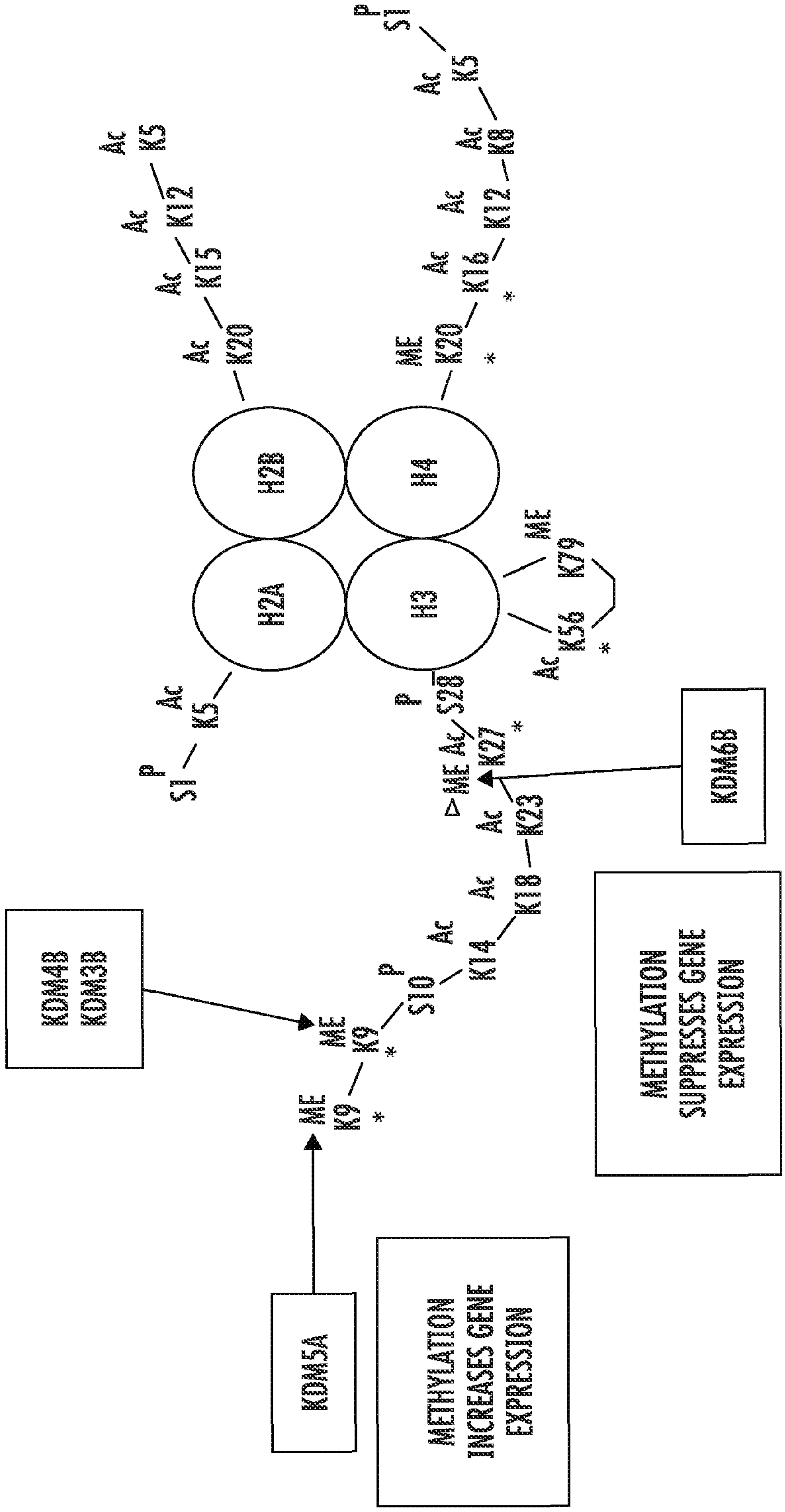
H2A
H2B
H3
H4
KDM5A
KDM4B
KDM3B
KDM6B
METHYLATION INCREASES GENE EXPRESSION
METHYLATION SUPPRESSES GENE EXPRESSION

FIG. 26

| TARGET | P-VALUE | Z-SCORE | GENE |
|---|---|---|---|
| GRM4_HUMAN | 1.26E-78 | 139.41 | GRM4 |
| KCNK9_HUMAN | 1.17E-44 | 78.42 | KCNK9 |
| KCNK3_HUMAN | 2.01E-35 | 61.84 | KCNK3 |
| SCNAA_HUMAN | 1.19E-22 | 38.91 | SCN10A |
| GRM5_HUMAN | 3.83E-18 | 30.82 | GRM5 |
| KDM6B_HUMAN | 1.11E-16 | 28.31 | KDM6B |
| GP139_HUMAN | 7.77E-16 | 26.65 | GPR139 |
| KDM4E_HUMAN | 4.40E-14 | 23.53 | KDM4E |
| KLF5_HUMAN | 5.19E-14 | 23.40 | KLF5 |
| KDM3A_HUMAN | 5.80E-14 | 23.31 | KDM3A |
| SMCA4_HUMAN | 4.21E-12 | 19.97 | SMARCA4 |
| NRAM2_HUMAN | 4.40E-12 | 19.94 | SLC11A2 |
| SMO_HUMAN | 7.20E-12 | 19.56 | SMO |
| FTO_HUMAN | 7.51E-12 | 19.52 | FTO |
| MITF_HUMAN | 1.15E-11 | 19.19 | MITF |
| Q9BRH5_HUMAN | 1.38E-11 | 19.05 | DGAT1 |
| AGTR1_HUMAN | 1.46E-11 | 19.01 | AGTR1 |
| HKDC1_HUMAN | 1.73E-11 | 18.87 | HKDC1 |
| KDM4A_HUMAN | 3.62E-11 | 18.30 | KDM4A |
| KPCB_HUMAN | 3.64E-11 | 18.29 | PRKCB |
| MPRI_HUMAN | 9.21E-11 | 17.57 | IGF2R |
| BRD7_HUMAN | 1.38E-10 | 17.25 | BRD7 |
| SC6A7_HUMAN | 4.28E-10 | 16.37 | SLC6A7 |
| EST1_HUMAN | 8.68E-10 | 15.83 | CES1 |
| CATD_HUMAN | 1.29E-09 | 15.51 | CTSD |
| PPGB_HUMAN | 2.43E-09 | 15.02 | CTSA |
| TGFB1_HUMAN | 7.09E-09 | 14.18 | TGFB1 |
| KDM4C_HUMAN | 1.63E-08 | 13.53 | KDM4C |
| KCNN1_HUMAN | 2.02E-08 | 13.37 | KCNN1 |
| HYEP_HUMAN | 7.42E-08 | 12.35 | EPHX1 |
| KDM5A_HUMAN | 1.66E-07 | 11.72 | KDM5A |
| CCR6_HUMAN | 3.11E-07 | 11.23 | CCR6 |
| IF4H_HUMAN | 3.21E-07 | 11.21 | EIF4H |
| PYGM_HUMAN | 4.08E-07 | 11.02 | PYGM |
| KCNN2_HUMAN | 5.39E-07 | 10.80 | KCNN2 |
| KDM5C_HUMAN | 5.64E-07 | 10.77 | KDM5C |
| FPPS_HUMAN | 7.47E-07 | 10.55 | FDPS |
| MAP11_HUMAN | 1.65E-06 | 9.93 | METAP1 |
| KDM2A_HUMAN | 1.75E-06 | 9.88 | KDM2A |

FIG. 27

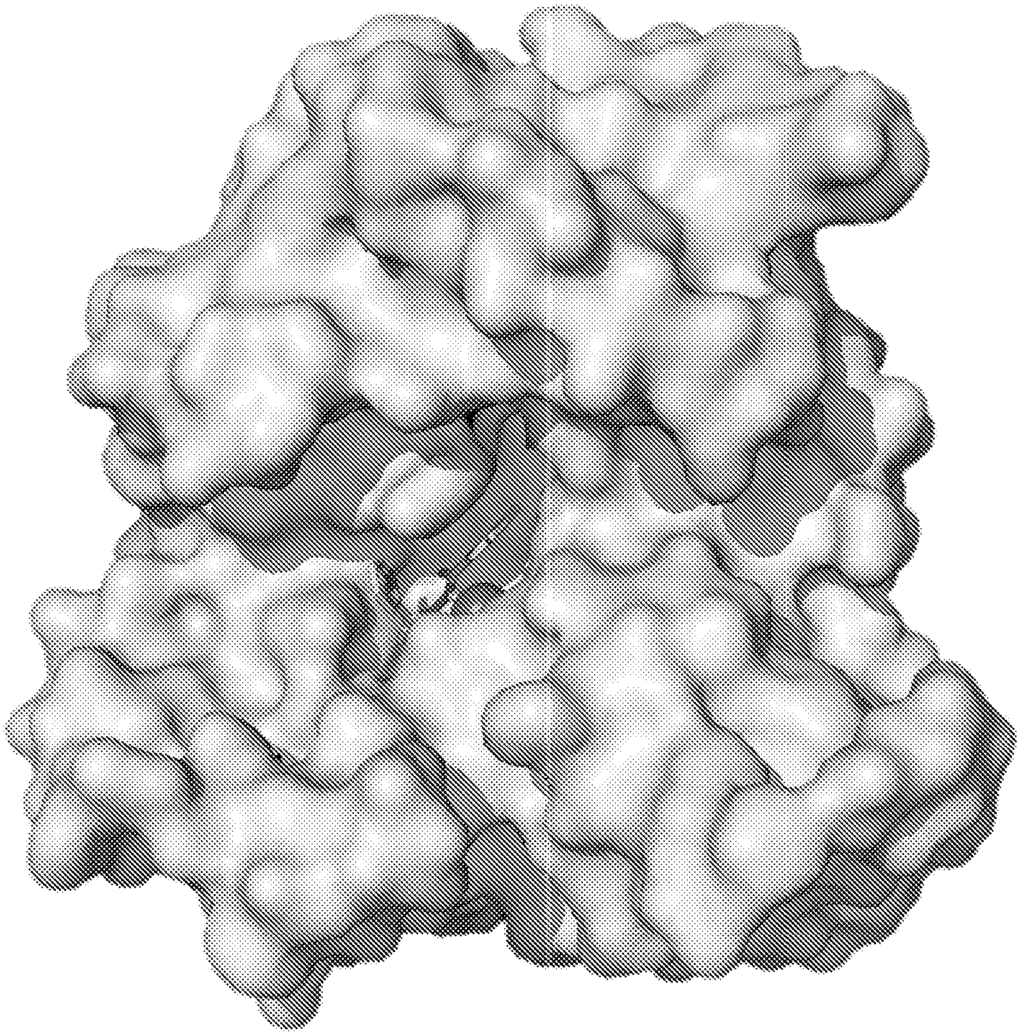

FIG. 28

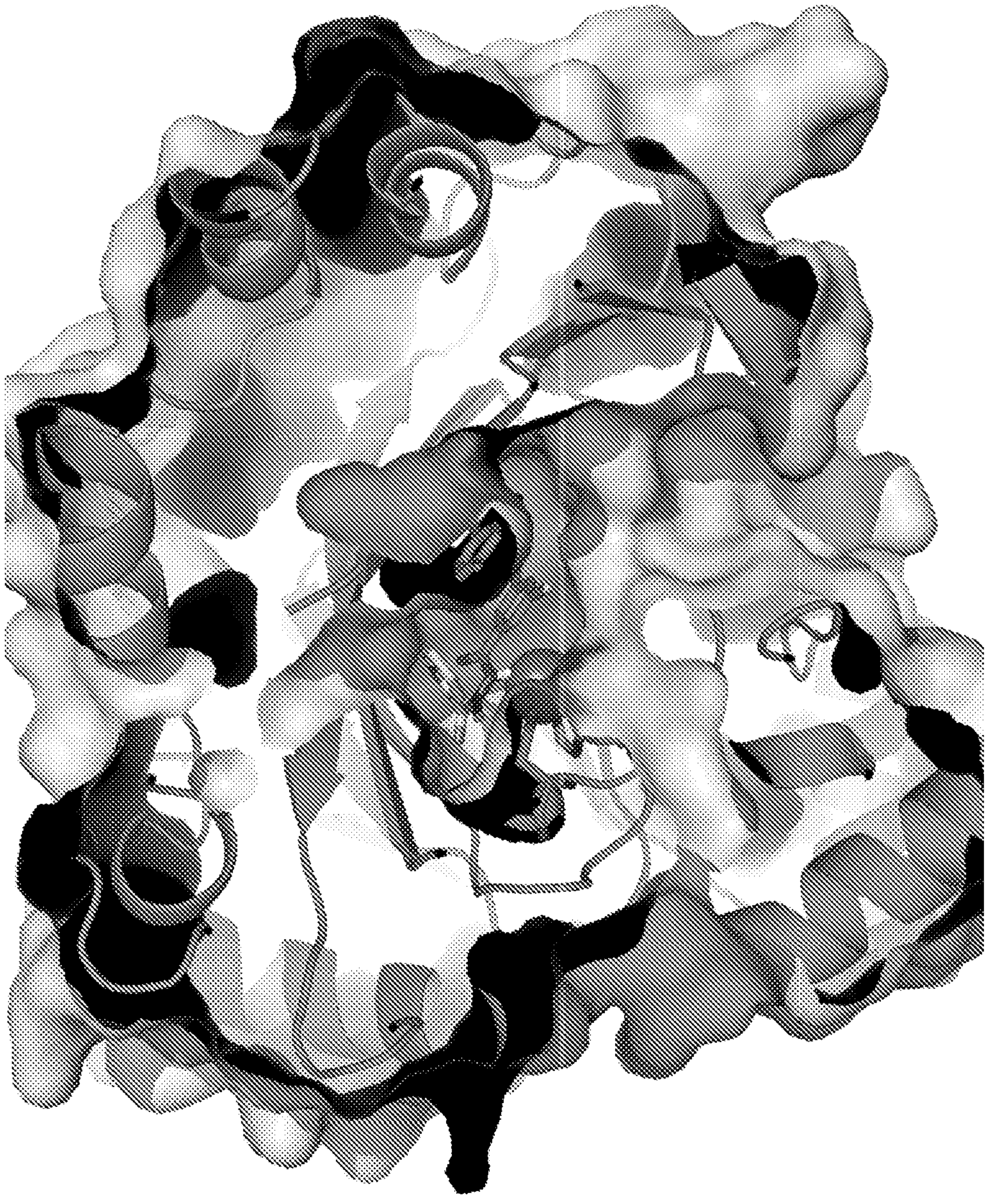

FIG. 29

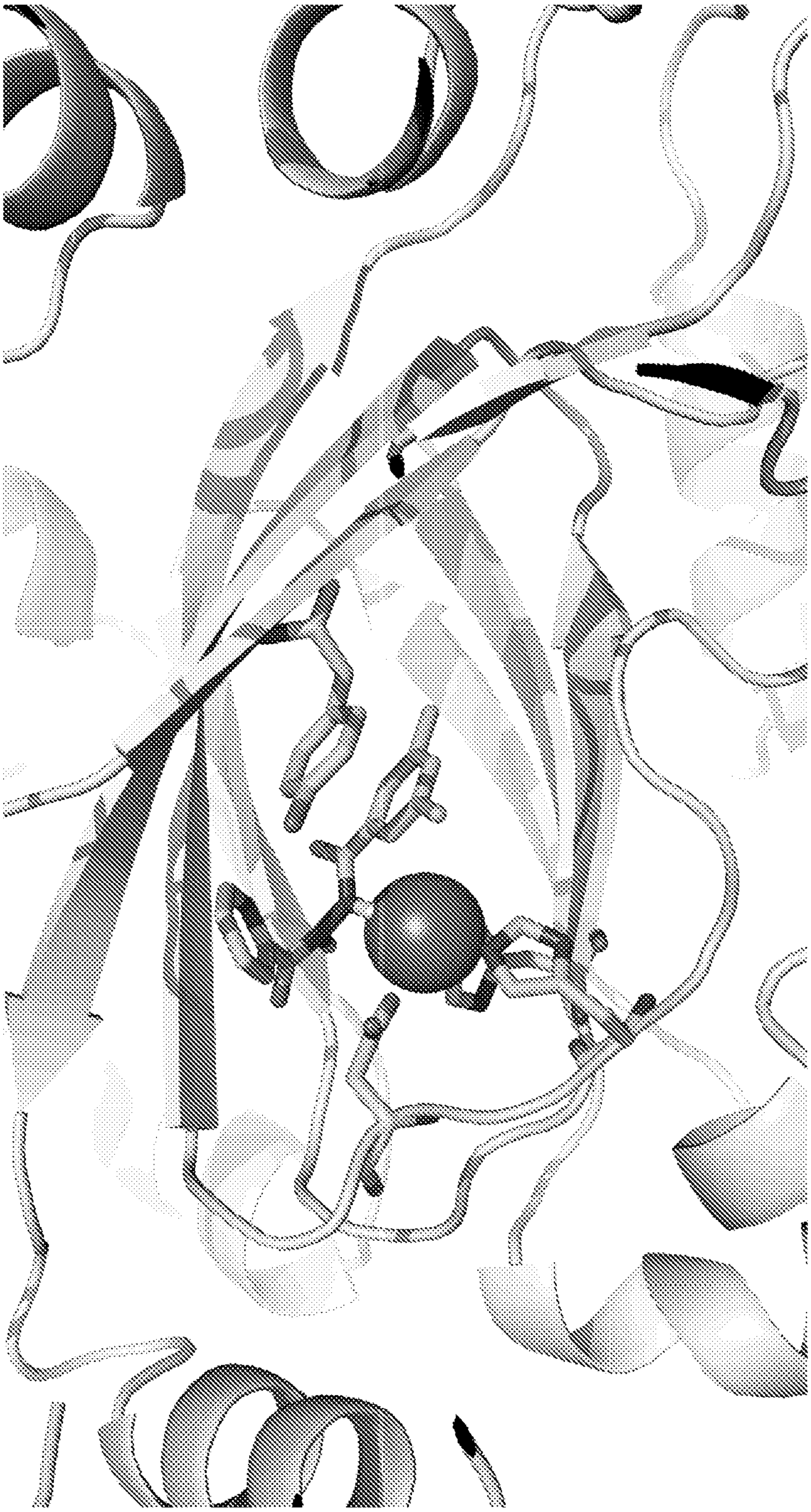

FIG. 30

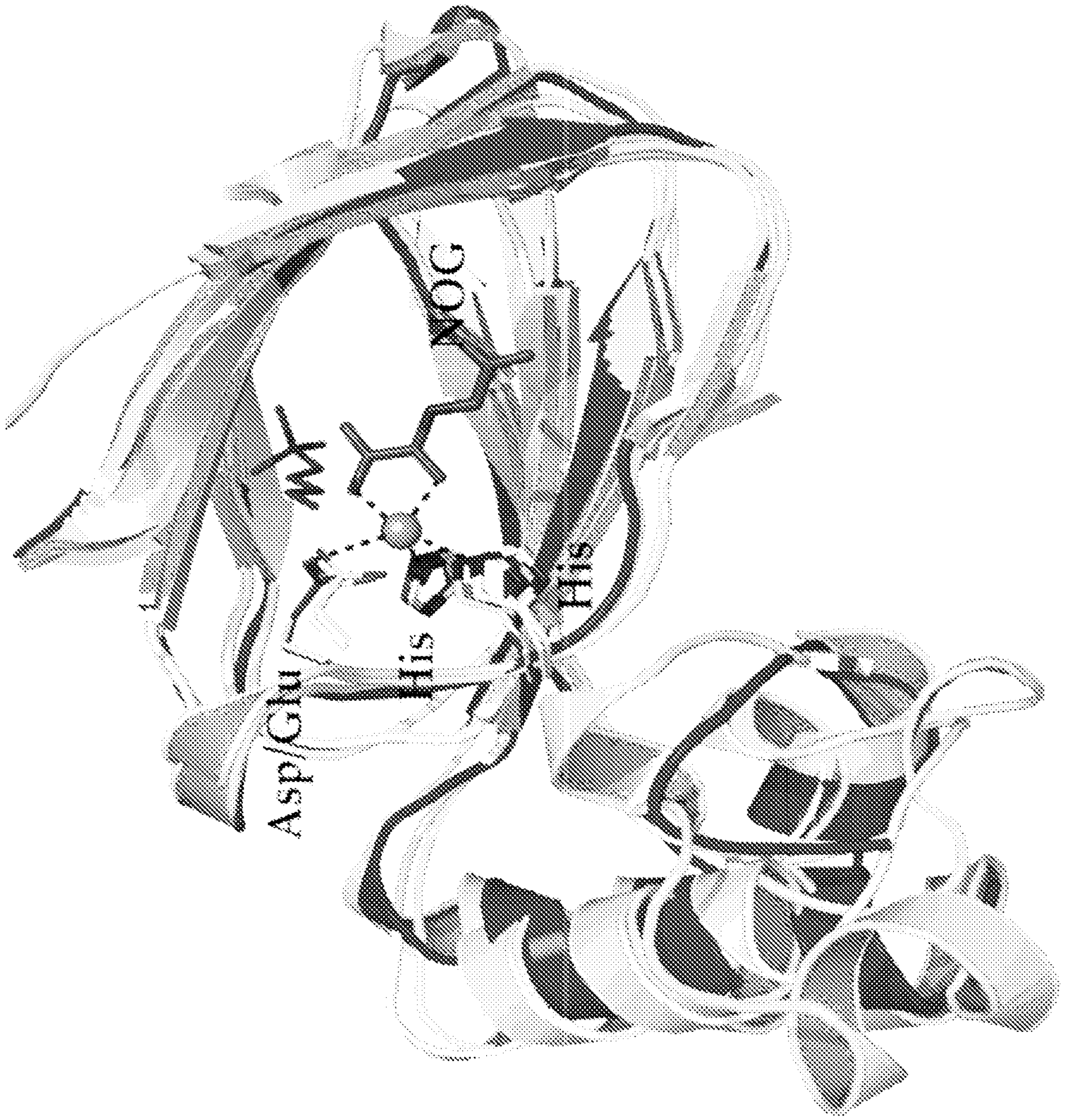


FIG. 31

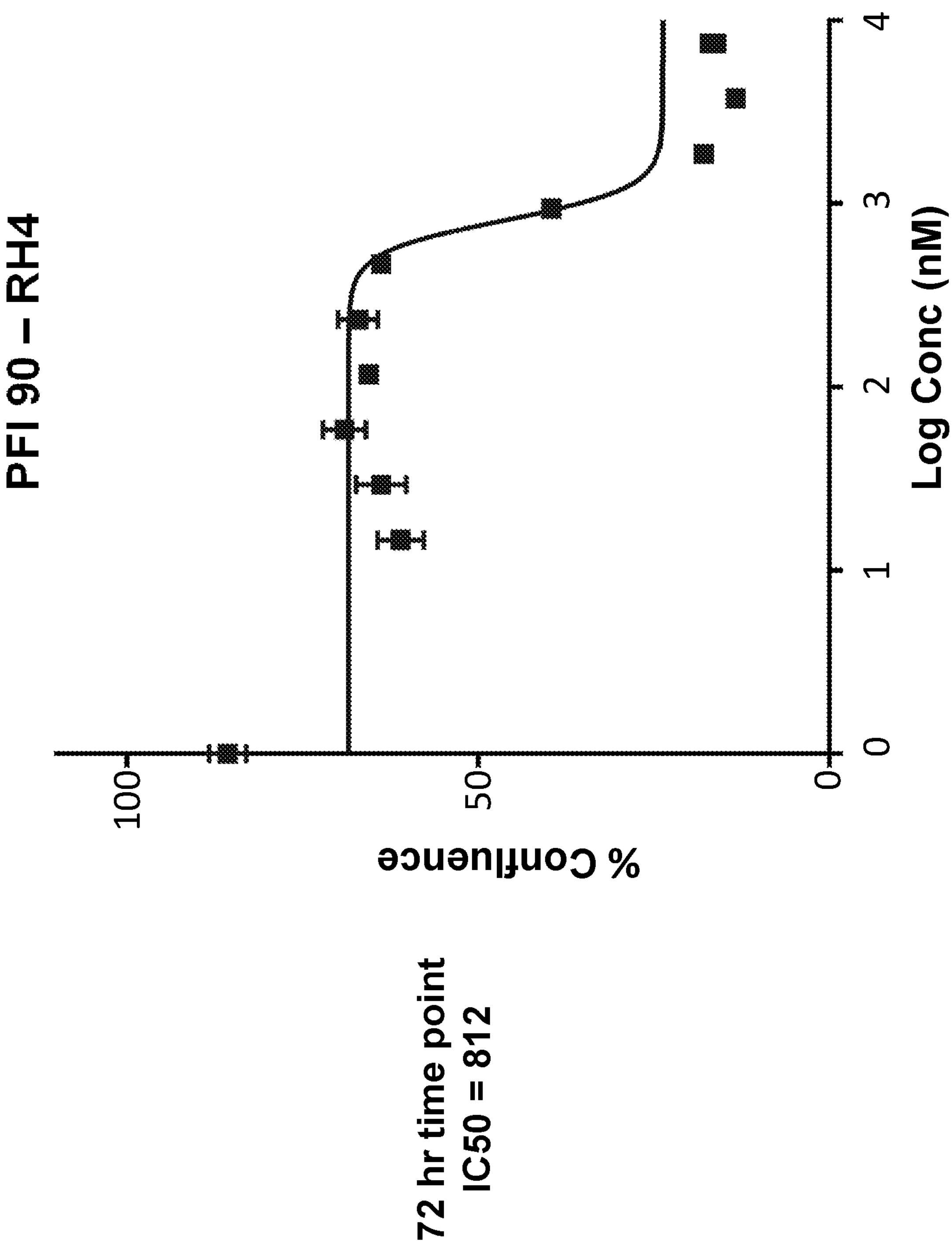
PFI 90 – RH4
% Confluence
100
50
0
0
1
2
3
4
Log Conc (nM)
72 hr time point
IC50 = 812

FIG. 32

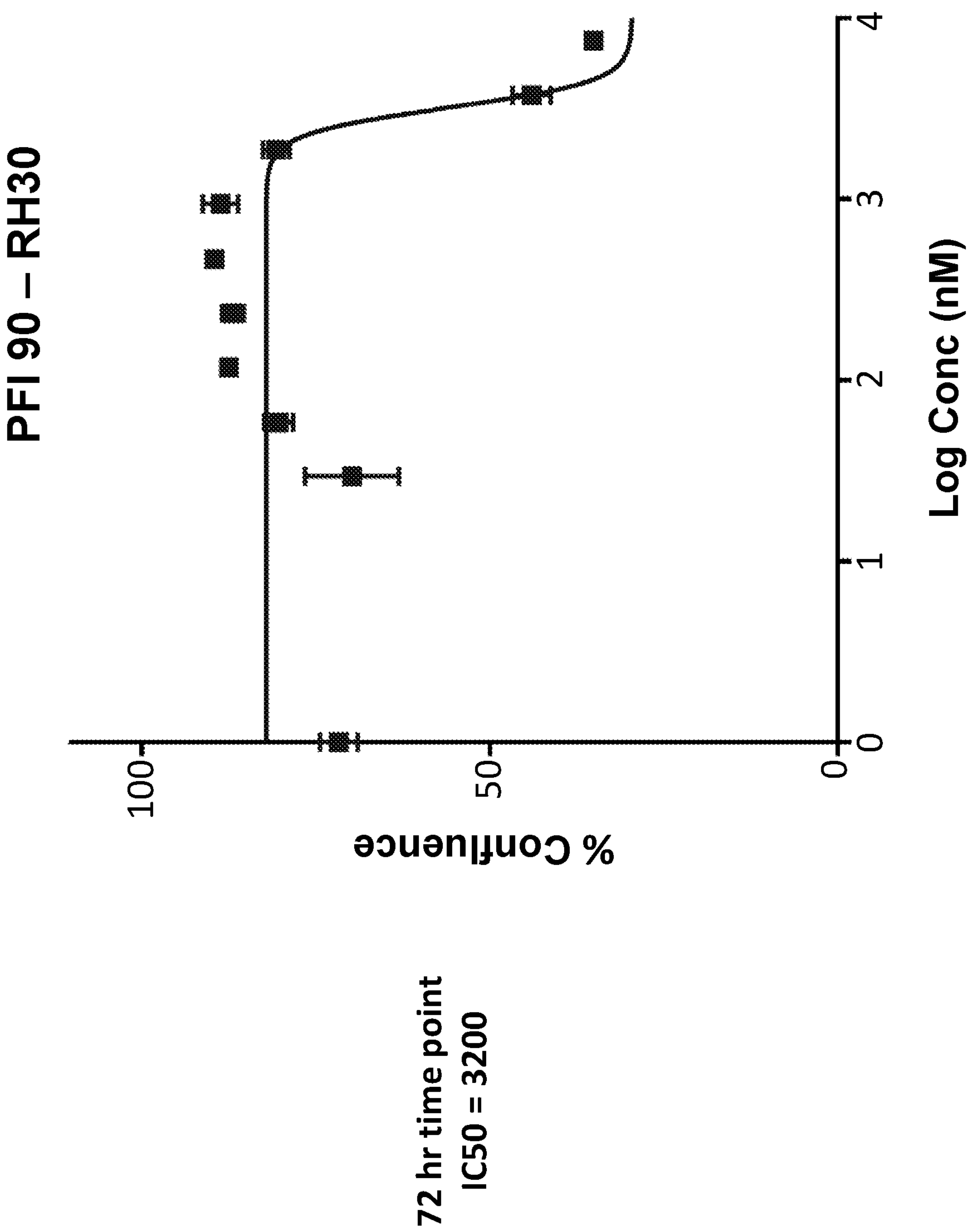
PFI 90 – RH30
% Confluence
100
50
0
0
1
2
3
4
Log Conc (nM)
72 hr time point
IC50 = 3200

FIG. 33

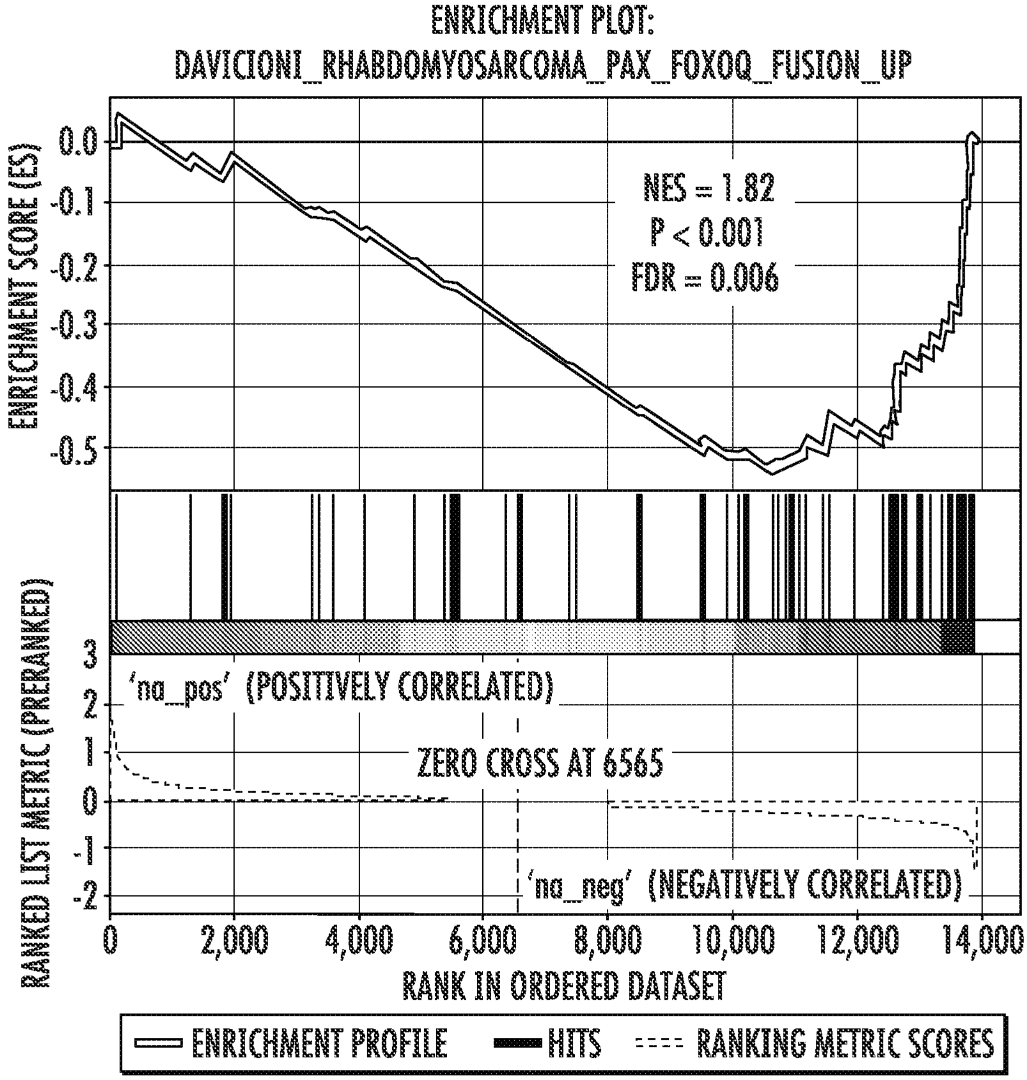
ENRICHMENT PLOT:
DAVICIONI_RHABDOMYOSARCOMA_PAX_FOXOQ_FUSION_UP
ENRICHMENT SCORE (ES)
0.0
-0.1
-0.2
-0.3
-0.4
-0.5
NES = 1.82
P < 0.001
FDR = 0.006
RANKED LIST METRIC (PRERANKED)
3
2
1
0
-1
-2
'na_pos' (POSITIVELY CORRELATED)
ZERO CROSS AT 6565
'na_neg' (NEGATIVELY CORRELATED)
0
2,000
4,000
6,000
8,000
10,000
12,000
14,000
RANK IN ORDERED DATASET
ENRICHMENT PROFILE
HITS
RANKING METRIC SCORES

FIG. 34

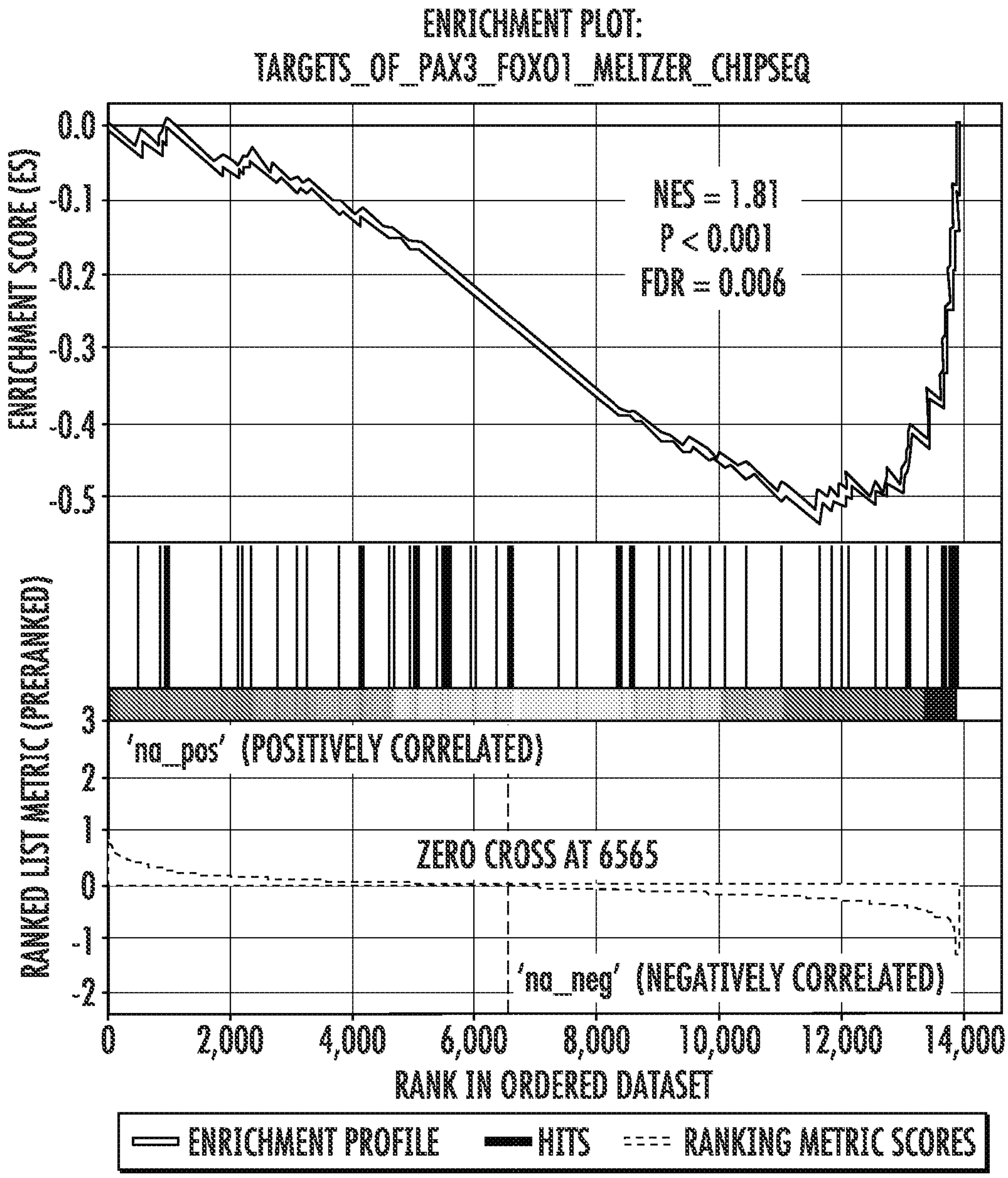
ENRICHMENT PLOT:
TARGETS_OF_PAX3_FOXO1_MELTZER_CHIPSEQ
ENRICHMENT SCORE (ES)
0.0
-0.1
-0.2
-0.3
-0.4
-0.5
NES = 1.81
P < 0.001
FDR = 0.006
RANKED LIST METRIC (PRERANKED)
3
2
1
0
-1
-2
'na_pos' (POSITIVELY CORRELATED)
ZERO CROSS AT 6565
'na_neg' (NEGATIVELY CORRELATED)
0
2,000
4,000
6,000
8,000
10,000
12,000
14,000
RANK IN ORDERED DATASET
ENRICHMENT PROFILE
HITS
RANKING METRIC SCORES

FIG. 35

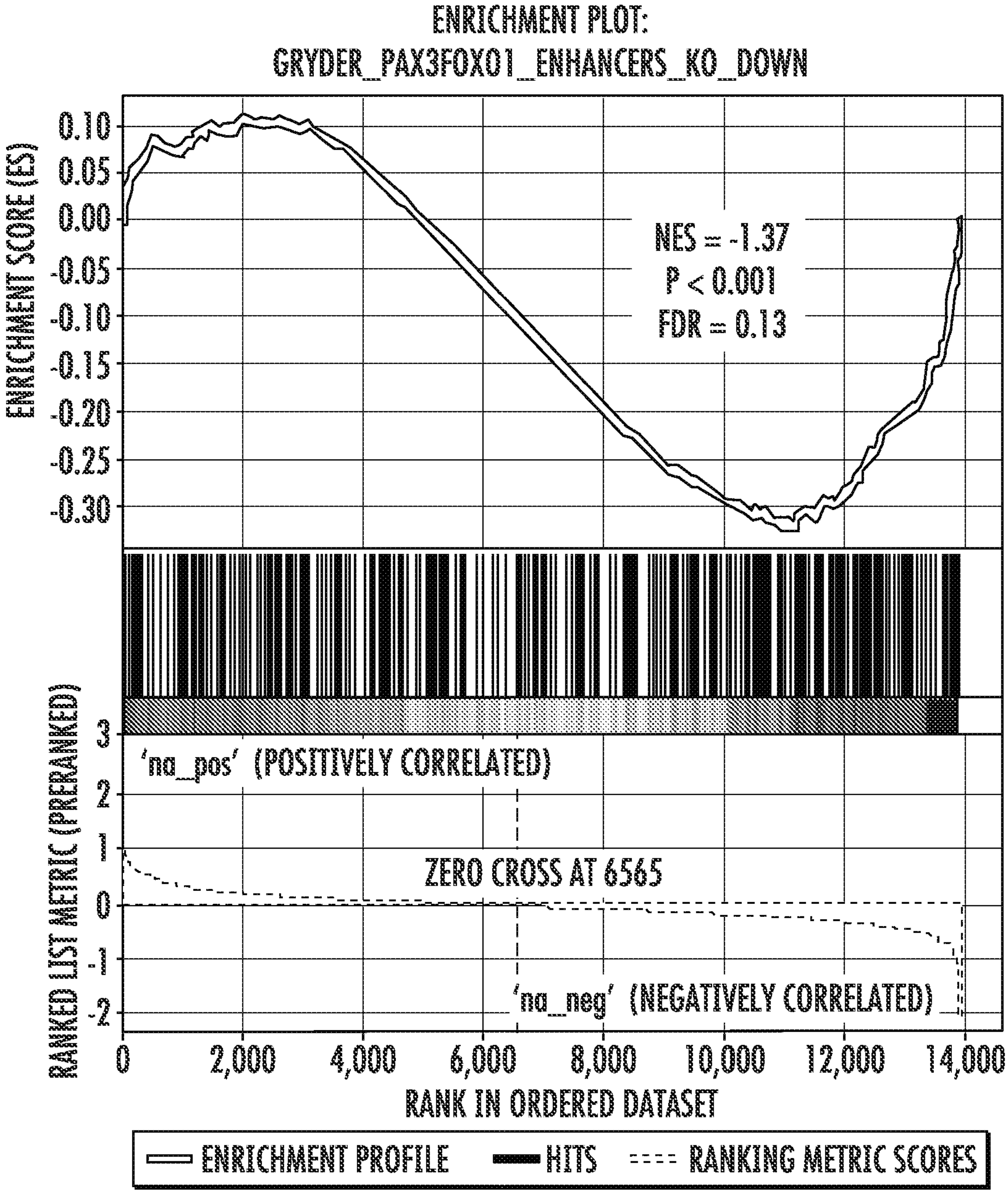
ENRICHMENT PLOT:
GRYDER_PAX3FOXO1_ENHANCERS_KO_DOWN
ENRICHMENT SCORE (ES)
0.10
0.05
0.00
-0.05
-0.10
-0.15
-0.20
-0.25
-0.30
NES = -1.37
P < 0.001
FDR = 0.13
RANKED LIST METRIC (PRERANKED)
3
2
1
0
-1
-2
'na_pos' (POSITIVELY CORRELATED)
ZERO CROSS AT 6565
'na_neg' (NEGATIVELY CORRELATED)
0
2,000
4,000
6,000
8,000
10,000
12,000
14,000
RANK IN ORDERED DATASET
ENRICHMENT PROFILE
HITS
RANKING METRIC SCORES

FIG. 36

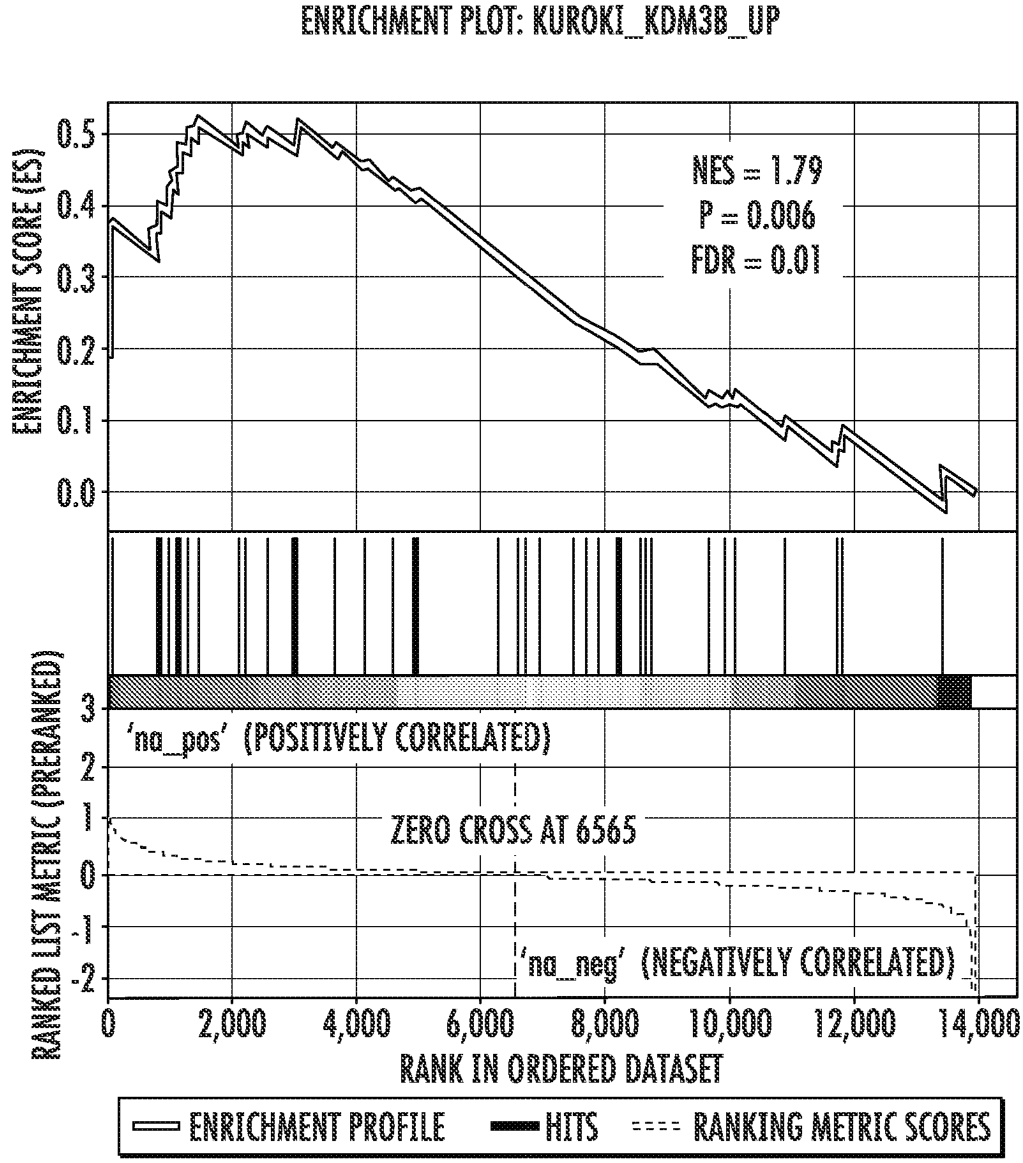
ENRICHMENT PLOT: KUROKI_KDM3B_UP
ENRICHMENT SCORE (ES)
0.5
0.4
0.3
0.2
0.1
0.0
NES = 1.79
P = 0.006
FDR = 0.01
RANKED LIST METRIC (PRERANKED)
3
2
1
0
-1
-2
'na_pos' (POSITIVELY CORRELATED)
ZERO CROSS AT 6565
'na_neg' (NEGATIVELY CORRELATED)
0
2,000
4,000
6,000
8,000
10,000
12,000
14,000
RANK IN ORDERED DATASET
ENRICHMENT PROFILE
HITS
RANKING METRIC SCORES

FIG. 37

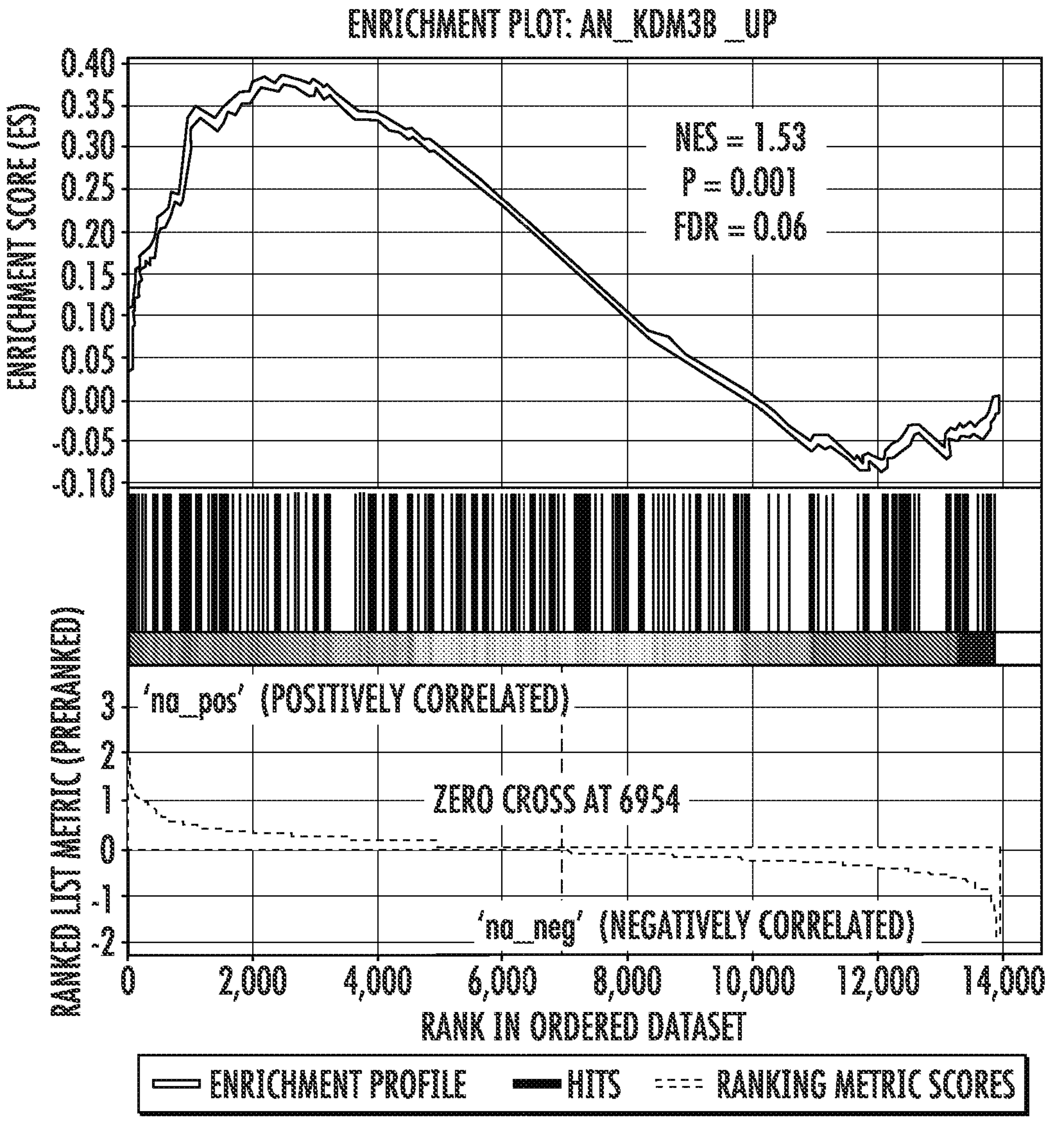
ENRICHMENT PLOT: AN_KDM3B _UP
ENRICHMENT SCORE (ES)
0.40
0.35
0.30
0.25
0.20
0.15
0.10
0.05
0.00
-0.05
-0.10
NES = 1.53
P = 0.001
FDR = 0.06
RANKED LIST METRIC (PRERANKED)
'na_pos' (POSITIVELY CORRELATED)
ZERO CROSS AT 6954
'na_neg' (NEGATIVELY CORRELATED)
3
2
1
0
-1
-2
0
2,000
4,000
6,000
8,000
10,000
12,000
14,000
RANK IN ORDERED DATASET
ENRICHMENT PROFILE
HITS
RANKING METRIC SCORES

FIG. 38

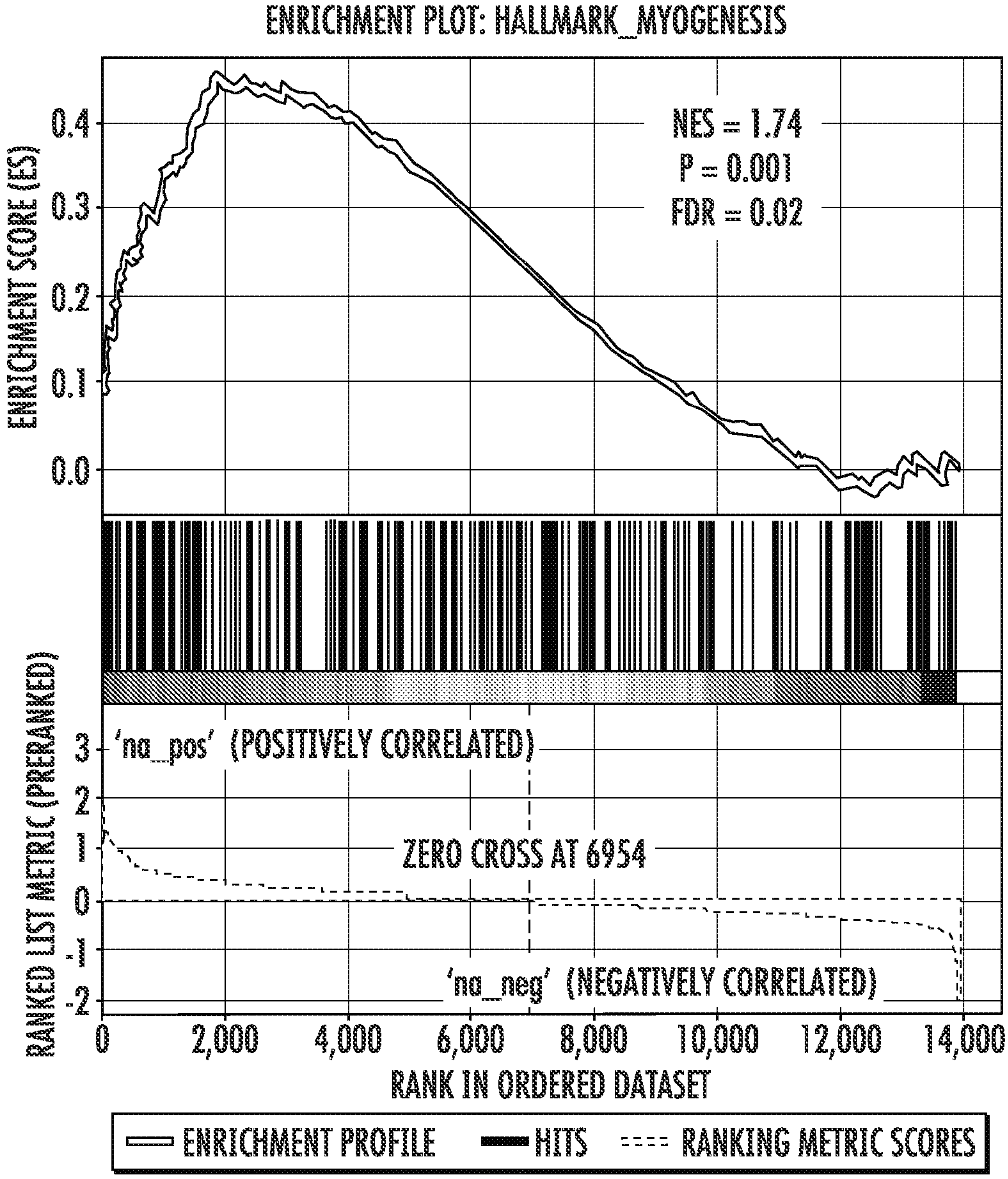
ENRICHMENT PLOT: HALLMARK_MYOGENESIS
ENRICHMENT SCORE (ES)
0.4
0.3
0.2
0.1
0.0
NES = 1.74
P = 0.001
FDR = 0.02
RANKED LIST METRIC (PRERANKED)
3
2
1
0
-1
-2
'na_pos' (POSITIVELY CORRELATED)
ZERO CROSS AT 6954
'na_neg' (NEGATIVELY CORRELATED)
0
2,000
4,000
6,000
8,000
10,000
12,000
14,000
RANK IN ORDERED DATASET
ENRICHMENT PROFILE
HITS
RANKING METRIC SCORES

FIG. 39

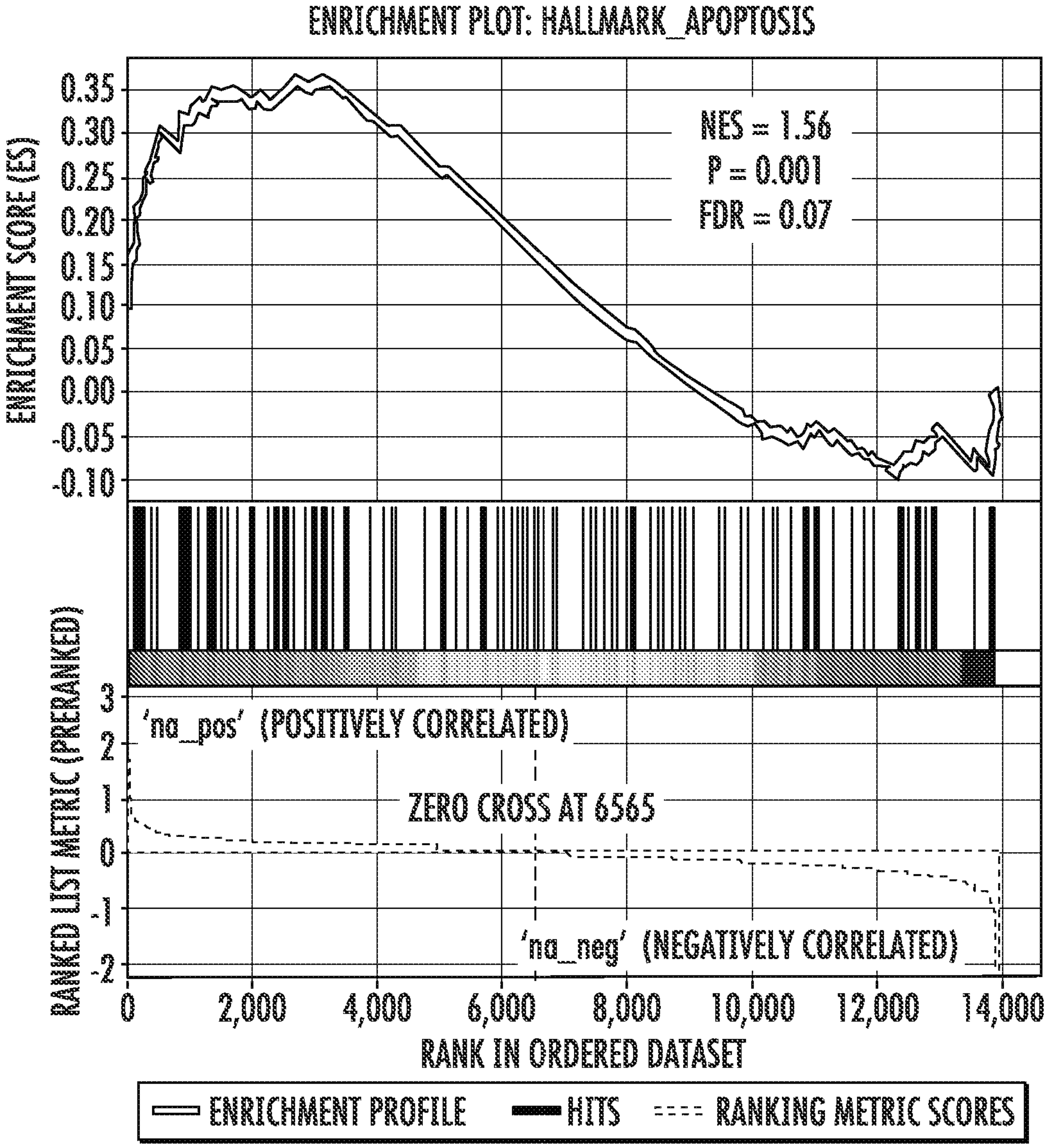
ENRICHMENT PLOT: HALLMARK_APOPTOSIS
ENRICHMENT SCORE (ES)
0.35
0.30
0.25
0.20
0.15
0.10
0.05
0.00
-0.05
-0.10
NES = 1.56
P = 0.001
FDR = 0.07
RANKED LIST METRIC (PRERANKED)
3
2
1
0
-1
-2
'na_pos' (POSITIVELY CORRELATED)
ZERO CROSS AT 6565
'na_neg' (NEGATIVELY CORRELATED)
0
2,000
4,000
6,000
8,000
10,000
12,000
14,000
RANK IN ORDERED DATASET
ENRICHMENT PROFILE
HITS
RANKING METRIC SCORES

FIG. 40

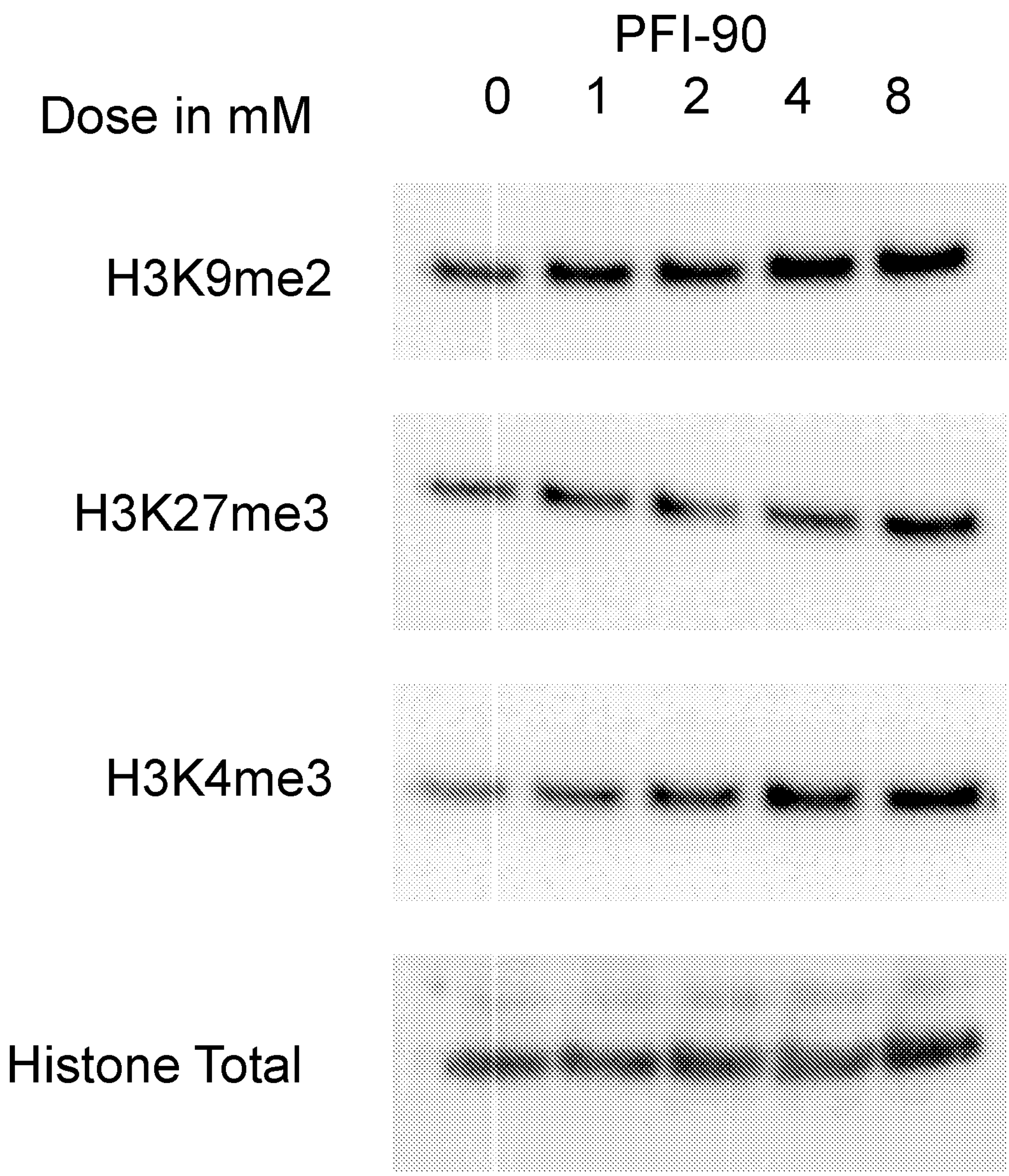
PFI-90
Dose in mM
0
1
2
4
8
H3K9me2
H3K27me3
H3K4me3
Histone Total

FIG. 41

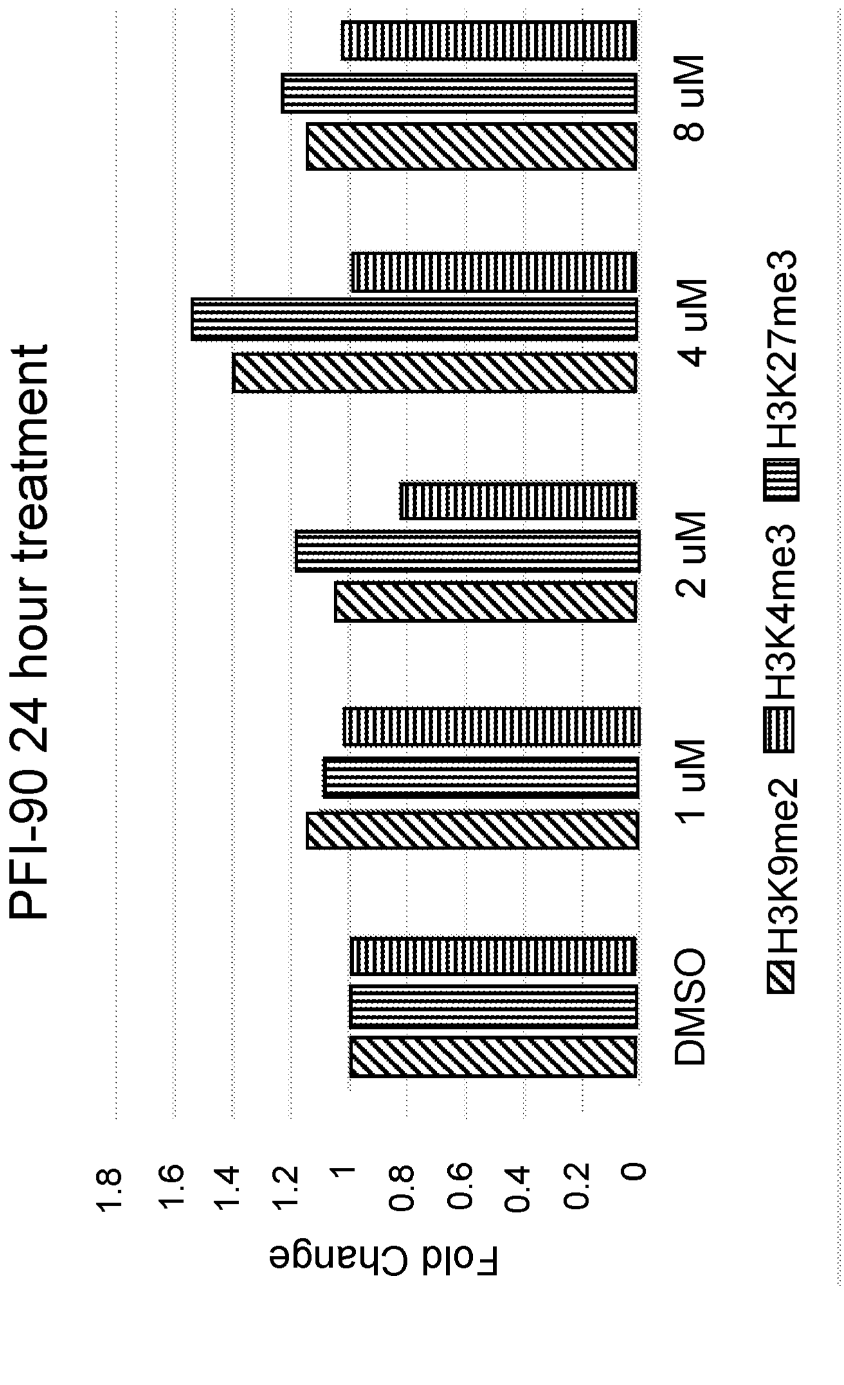
PFI-90 24 hour treatment
Fold Change
1.8
1.6
1.4
1.2
1
0.8
0.6
0.4
0.2
0
DMSO
1 uM
2 uM
4 uM
8 uM
H3K9me2
H3K4me3
H3K27me3

FIG. 42

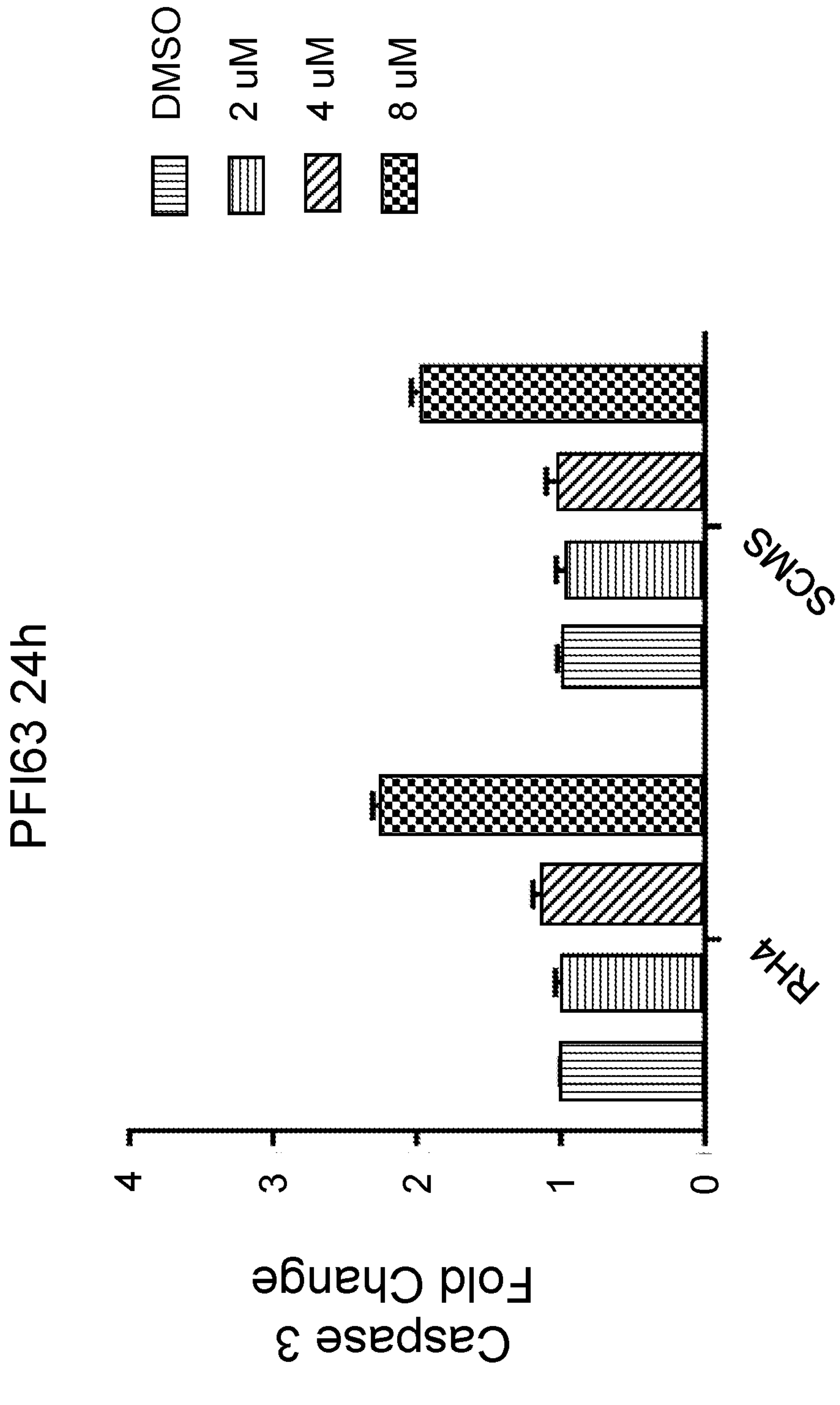
PFI63 24h
Caspase 3 Fold Change
0
1
2
3
4
RH4
SCMS
DMSO
2 uM
4 uM
8 uM

FIG. 43

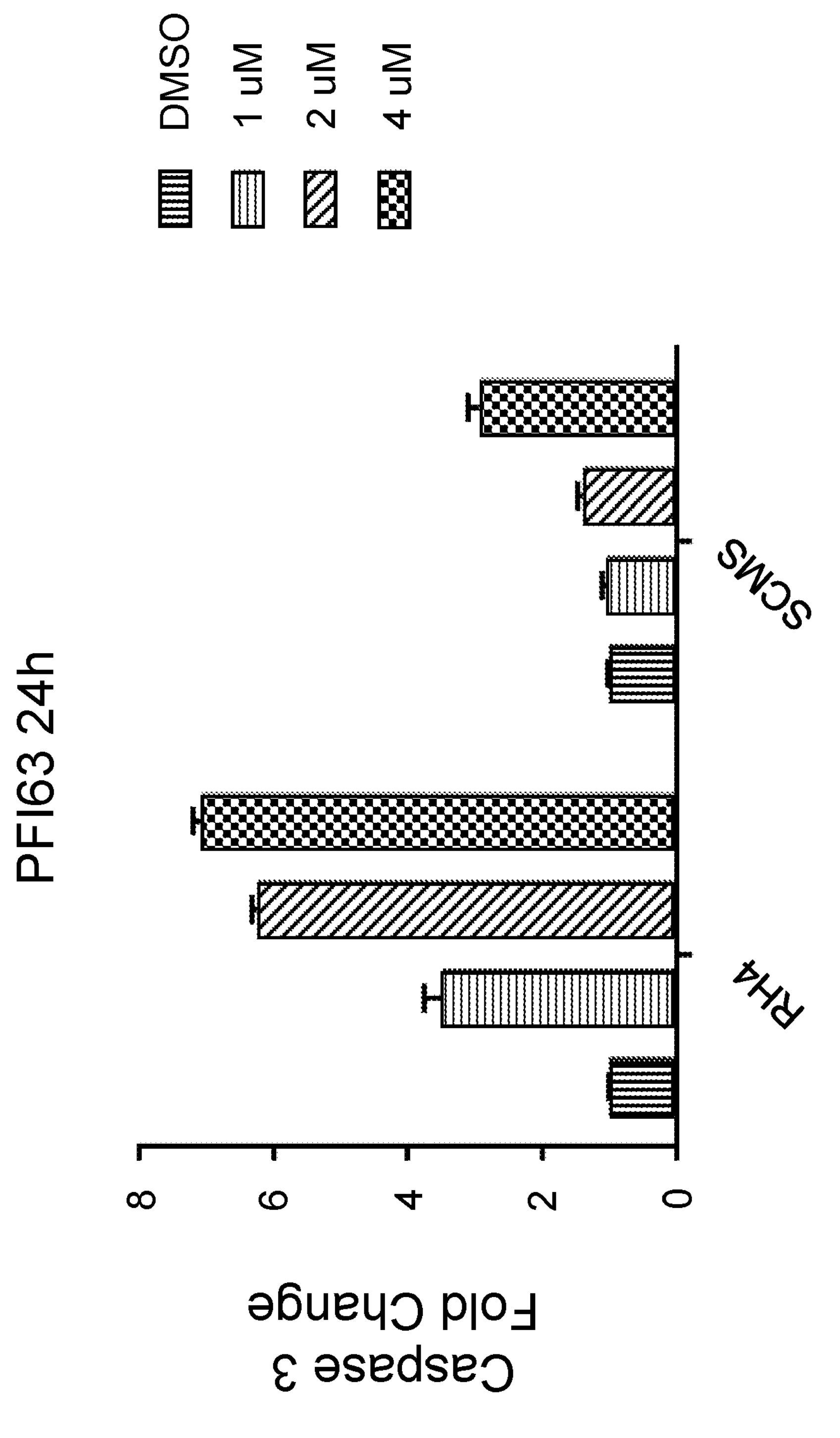
PFI63 24h
Caspase 3
Fold Change
0
2
4
6
8
RH4
SCMS
DMSO
1 uM
2 uM
4 uM

FIG. 44

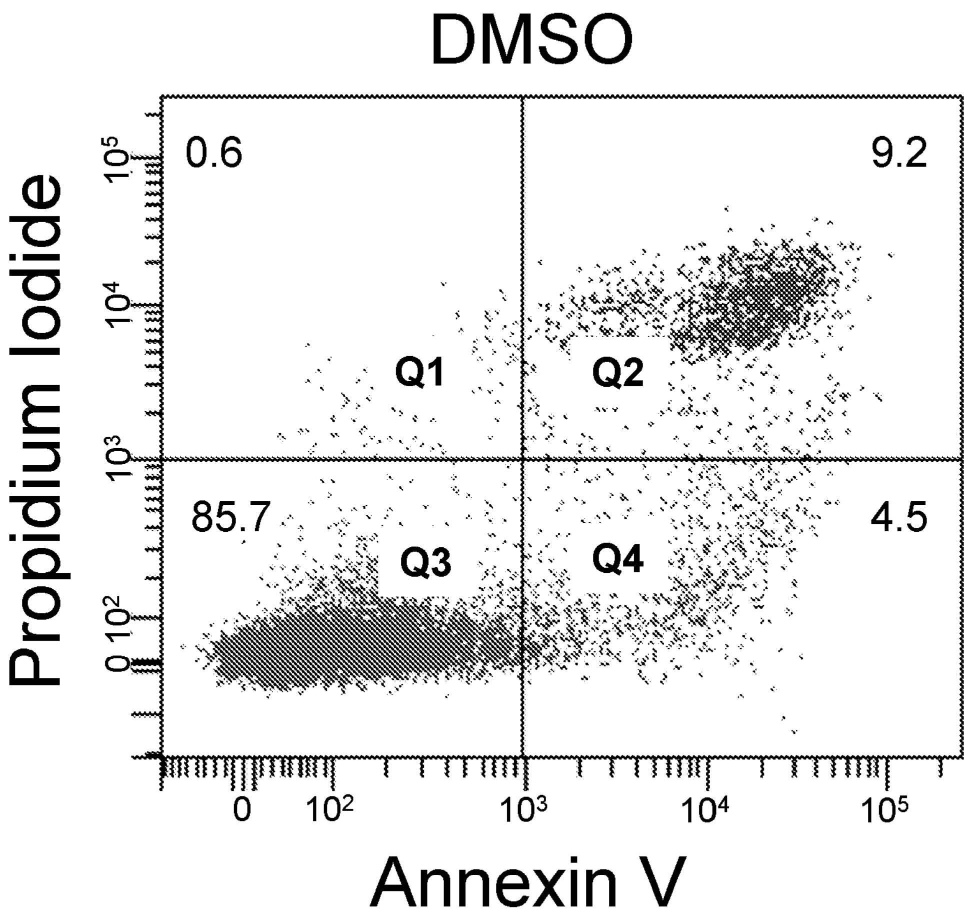
DMSO
0.6
9.2
85.7
4.5
Q1
Q2
Q3
Q4
Propidium Iodide
Annexin V
0
$10^2$
$10^3$
$10^4$
$10^5$

FIG. 45

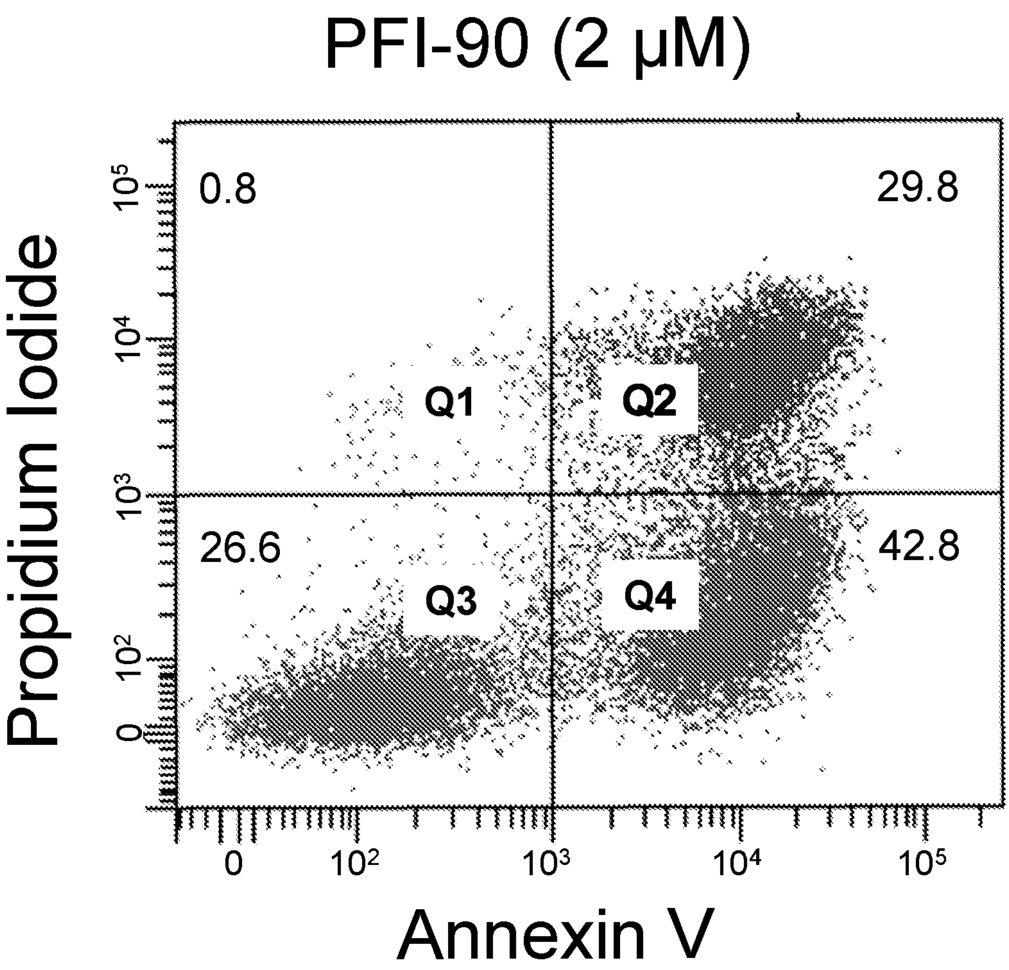
PFI-90 (2 µM)
0.8
29.8
Q1
Q2
26.6
42.8
Q3
Q4
Propidium Iodide
Annexin V
0
10²
10³
10⁴
10⁵

FIG. 46

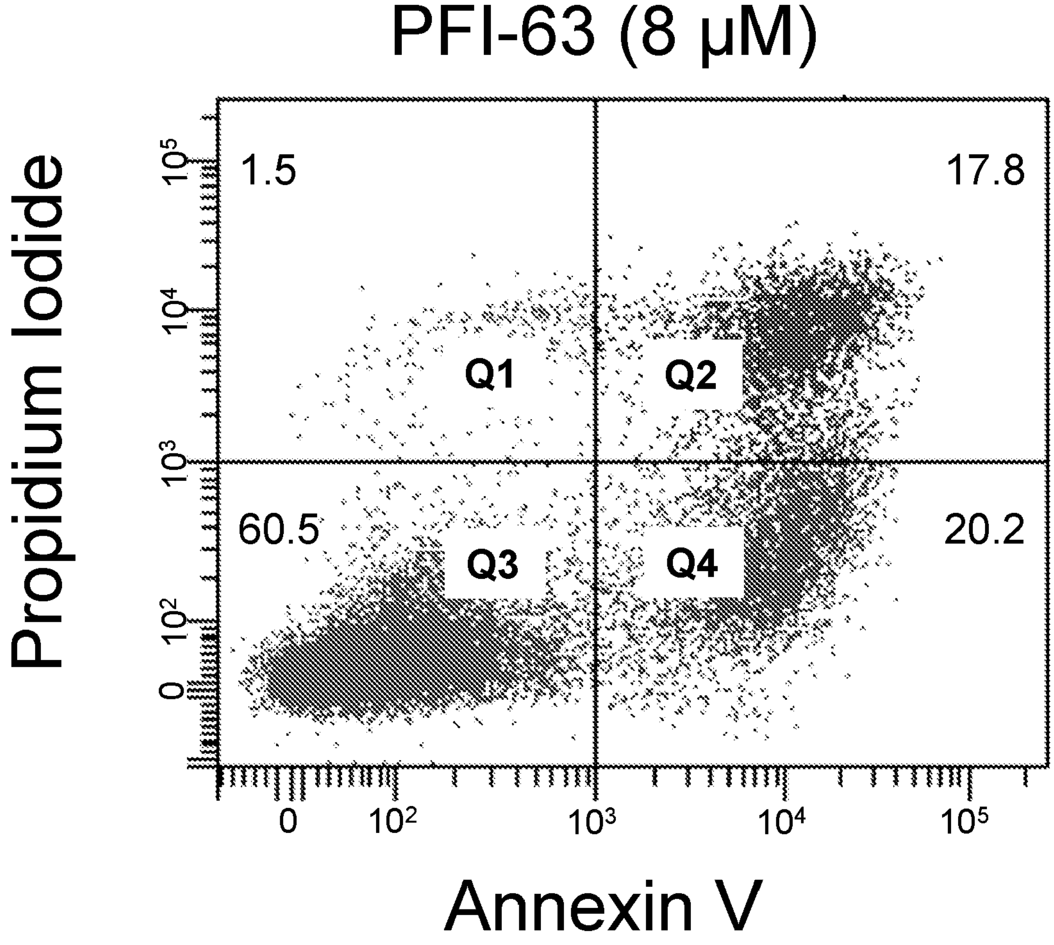
PFI-63 (8 µM)
Propidium Iodide
Annexin V
1.5
17.8
60.5
20.2
Q1
Q2
Q3
Q4
0
10^2
10^3
10^4
10^5

FIG. 47

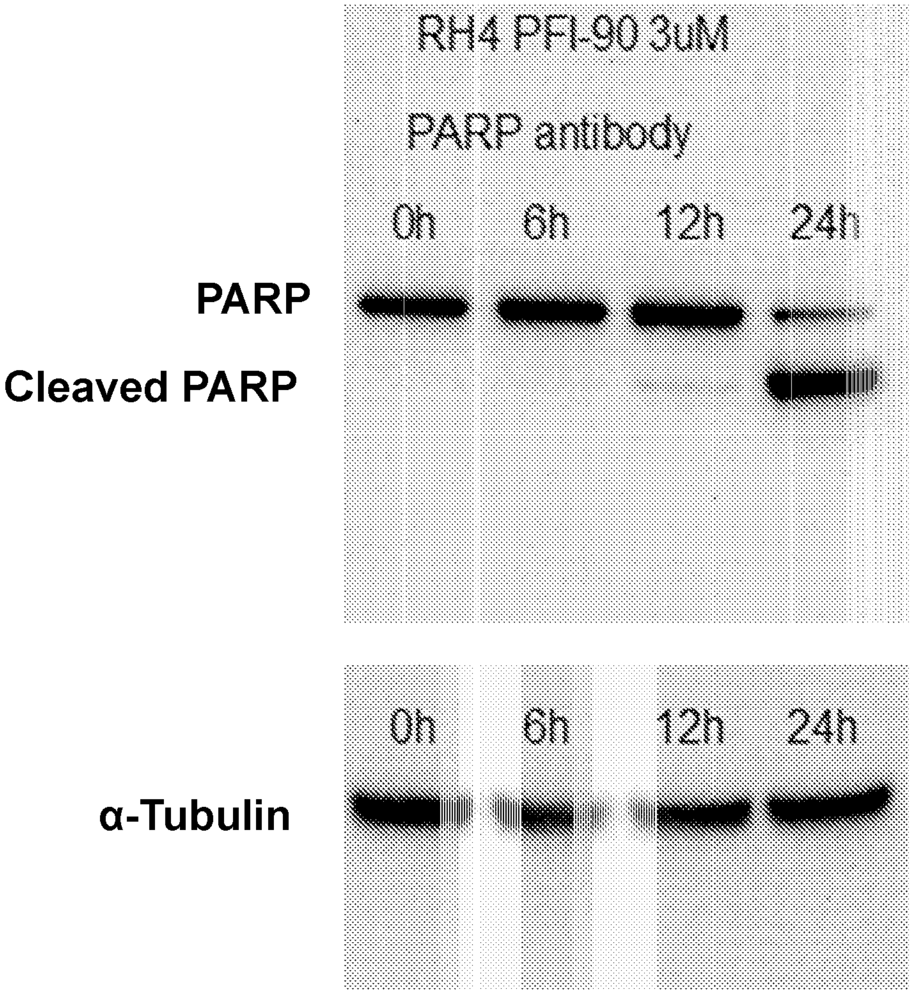
RH4 PFI-90 3uM
PARP antibody
0h
6h
12h
24h
PARP
Cleaved PARP
0h
6h
12h
24h
α-Tubulin

FIG. 48

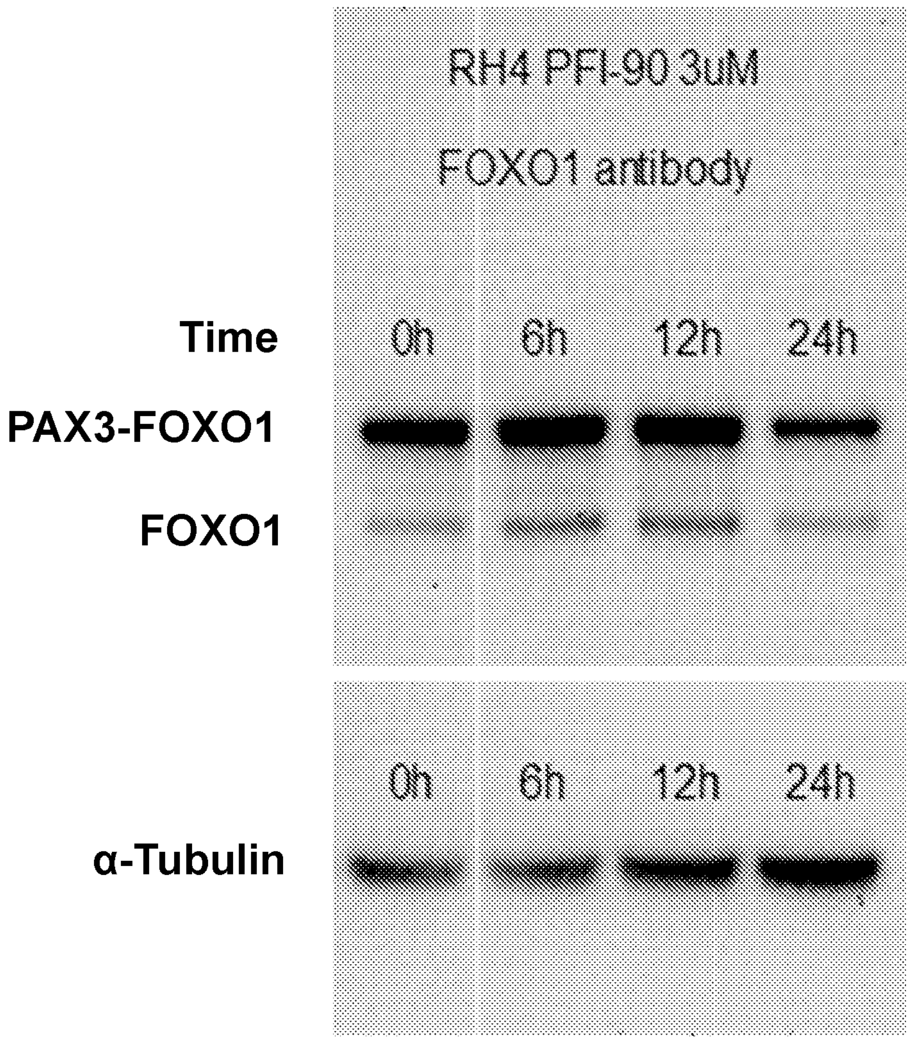
RH4 PFI-90 3uM
FOXO1 antibody
Time
0h
6h
12h
24h
PAX3-FOXO1
FOXO1
0h
6h
12h
24h
α-Tubulin

FIG. 49

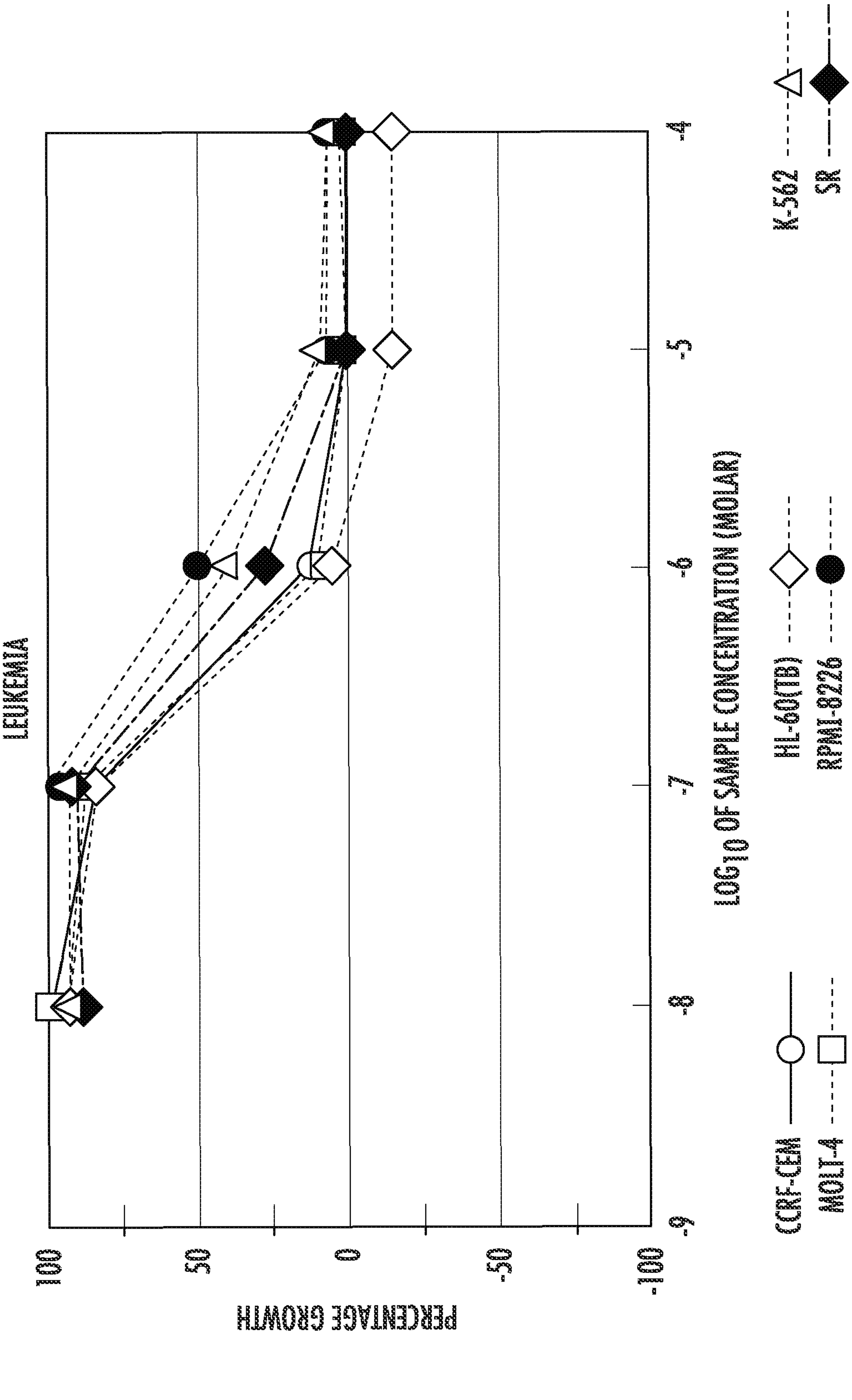
LEUKEMIA
PERCENTAGE GROWTH
100
50
0
-50
-100
-9
-8
-7
-6
-5
-4
$LOG_{10}$ OF SAMPLE CONCENTRATION (MOLAR)
CCRF-CEM
MOLT-4
HL-60(TB)
RPMI-8226
K-562
SR

FIG. 50

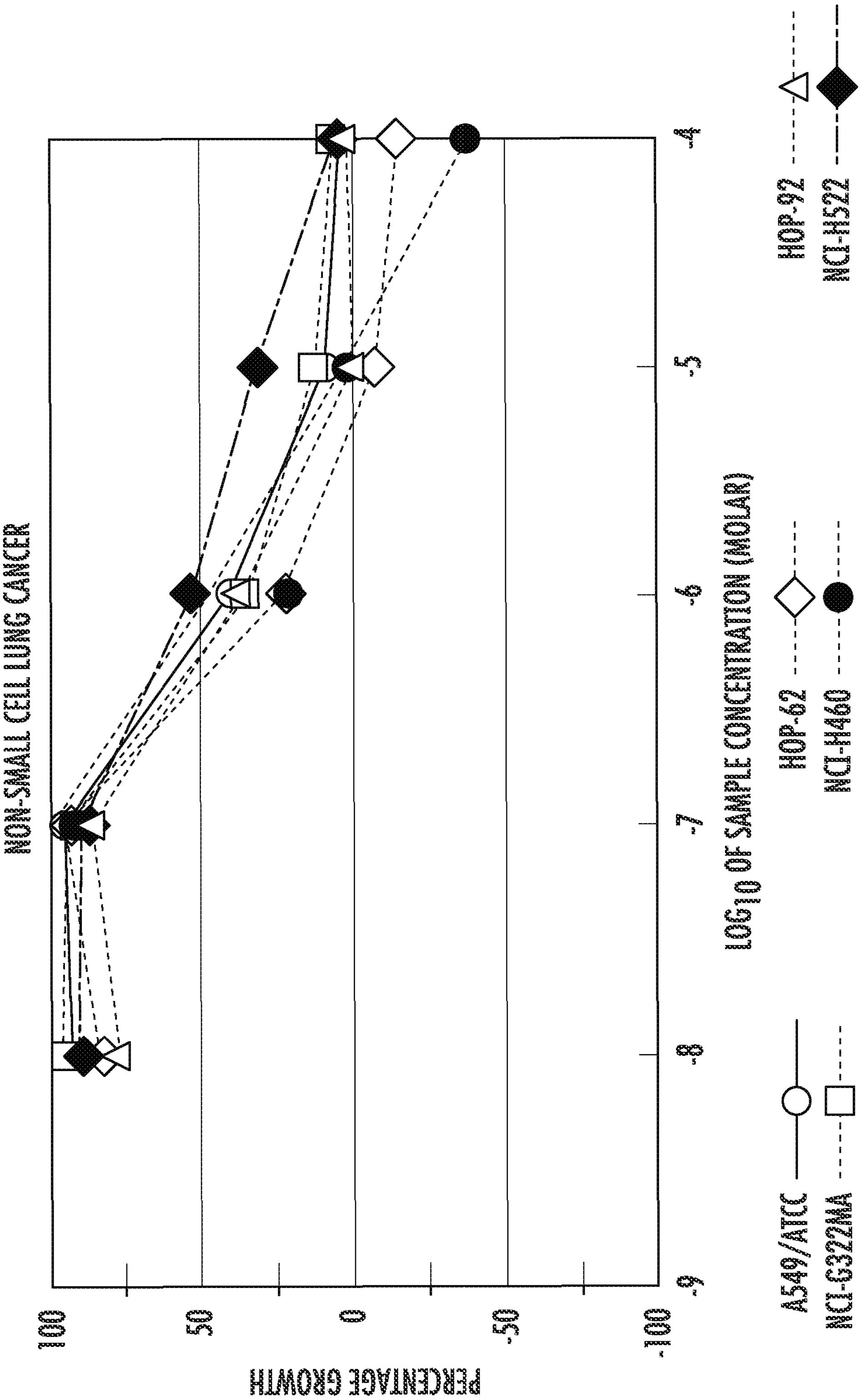

NON-SMALL CELL LUNG CANCER
PERCENTAGE GROWTH
100
50
0
-50
-100
-9
-8
-7
-6
-5
-4
LOG10 OF SAMPLE CONCENTRATION (MOLAR)
A549/ATCC
NCI-G322MA
HOP-62
NCI-H460
HOP-92
NCI-H522


FIG. 51

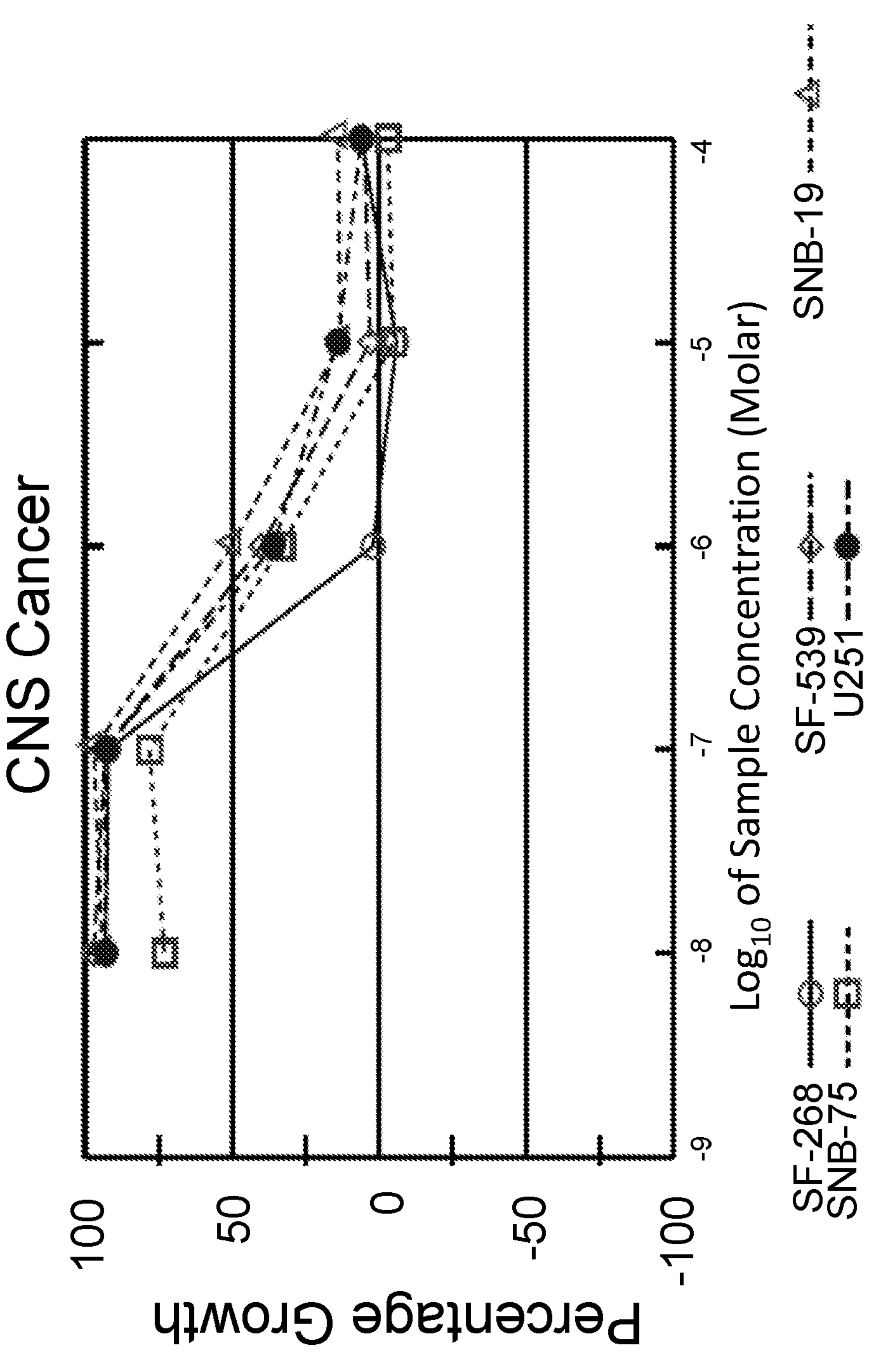
CNS Cancer
Percentage Growth
100
50
0
-50
-100
-9
-8
-7
-6
-5
-4
Log10 of Sample Concentration (Molar)
SF-268
SNB-75
SF-539
U251
SNB-19

FIG. 52

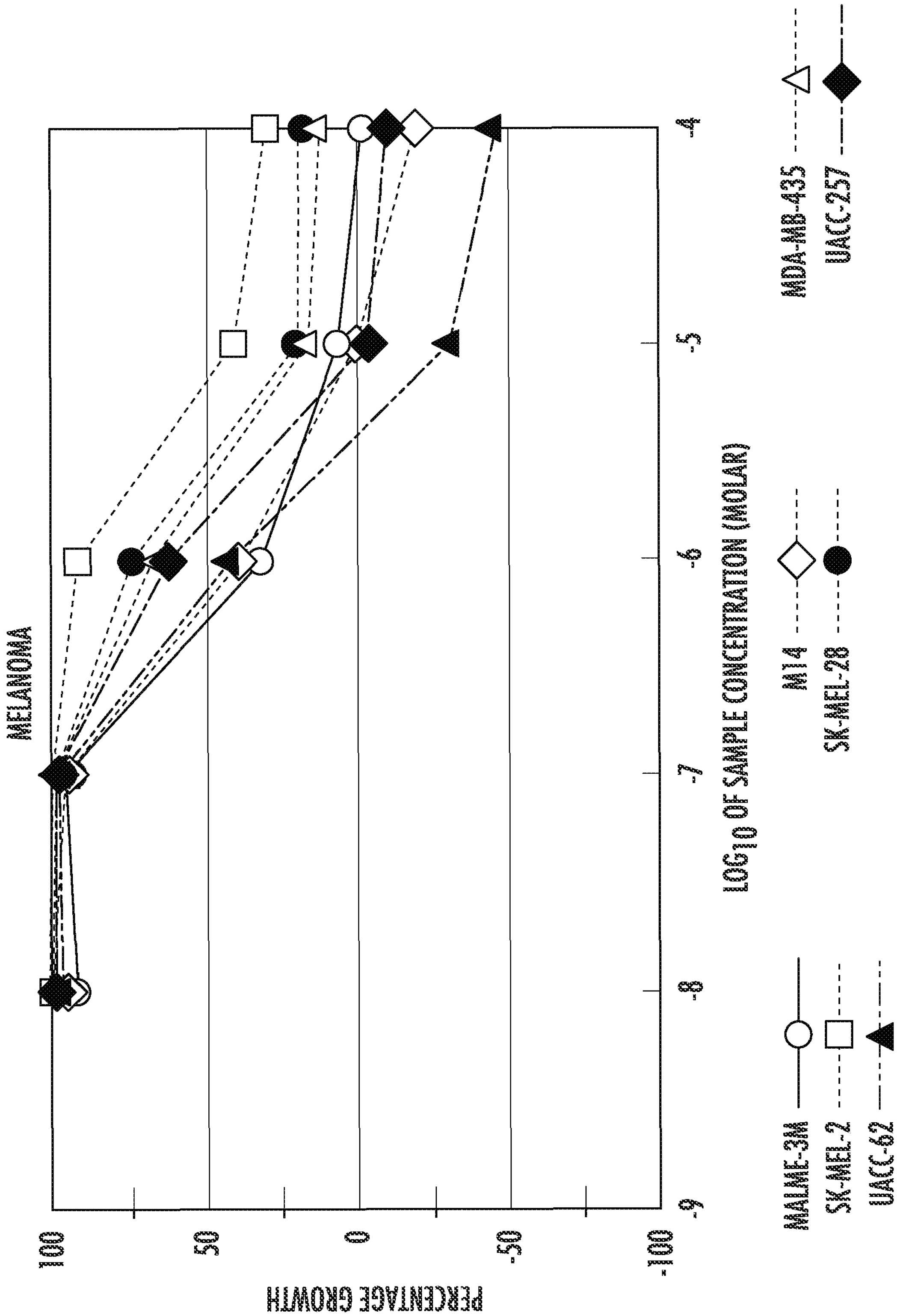
MELANOMA
PERCENTAGE GROWTH
100
50
0
-50
-100
-9
-8
-7
-6
-5
-4
LOG10 OF SAMPLE CONCENTRATION (MOLAR)
MALME-3M
SK-MEL-2
UACC-62
M14
SK-MEL-28
MDA-MB-435
UACC-257

FIG. 53

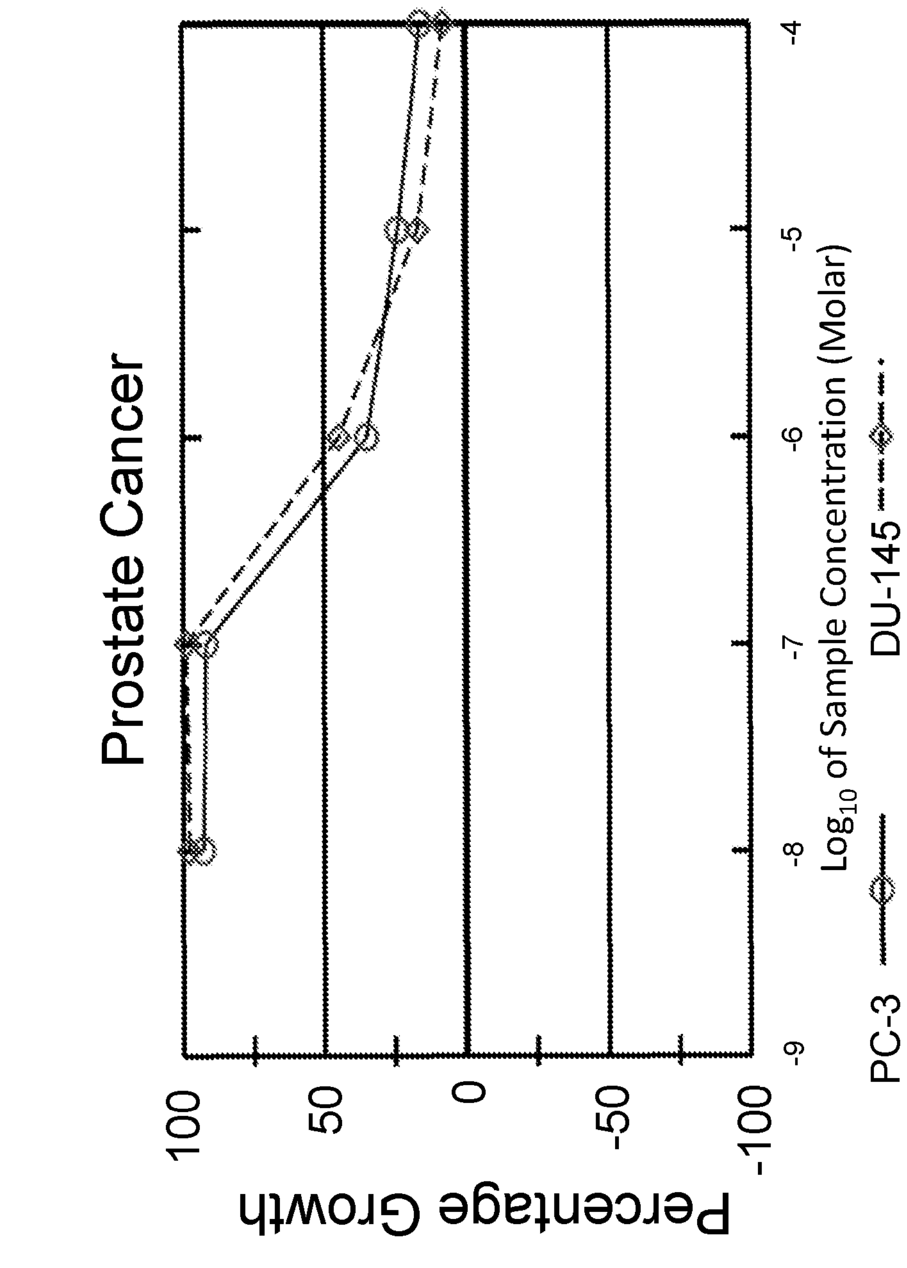

Prostate Cancer
Percentage Growth
100
50
0
-50
-100
-9
-8
-7
-6
-5
-4
Log$_{10}$ of Sample Concentration (Molar)
PC-3
DU-145


FIG. 54

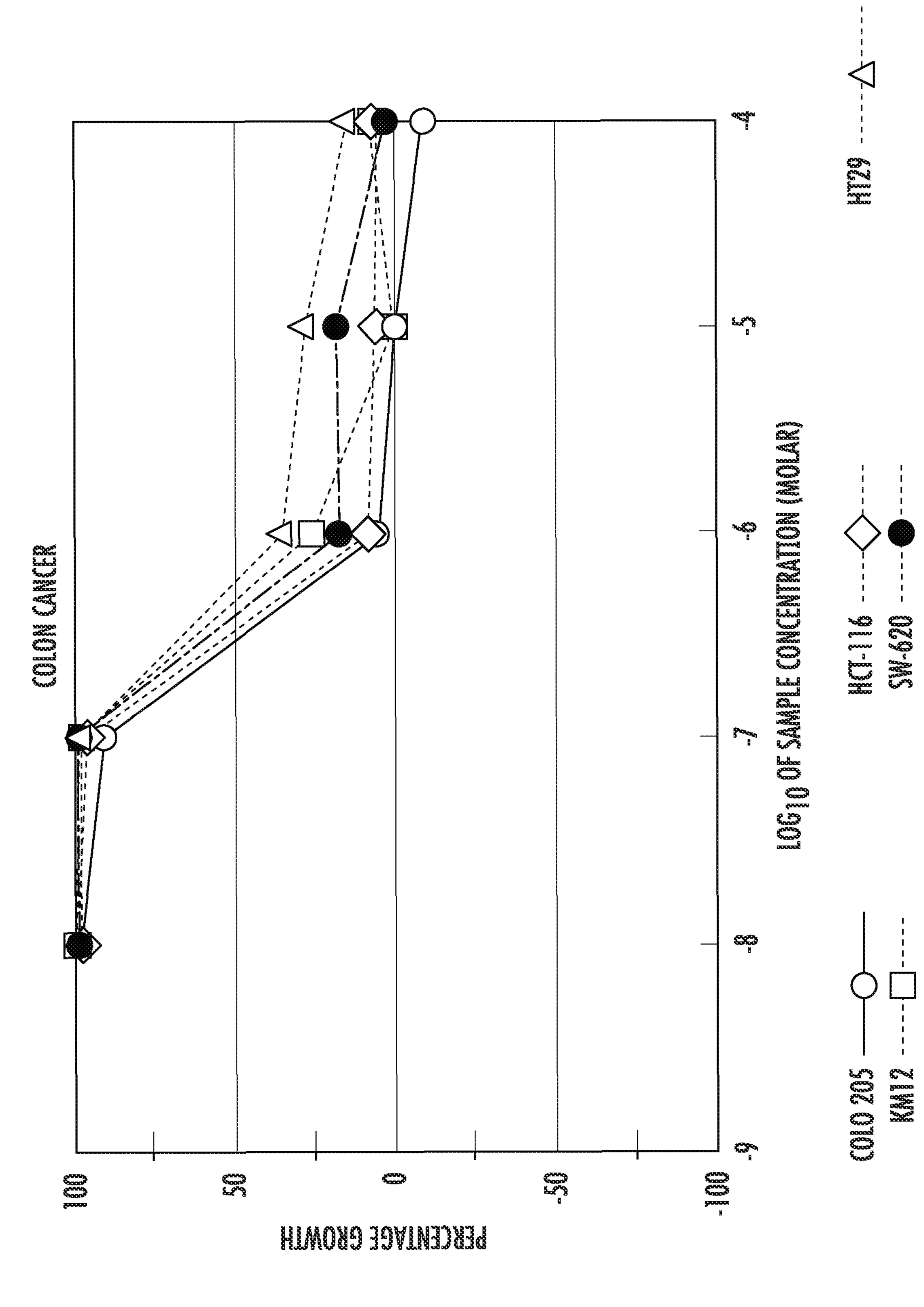
COLON CANCER
PERCENTAGE GROWTH
100
50
0
-50
-100
-9
-8
-7
-6
-5
-4
$LOG_{10}$ OF SAMPLE CONCENTRATION (MOLAR)
COLO 205
KM12
HCT-116
SW-620
HT29

FIG. 55

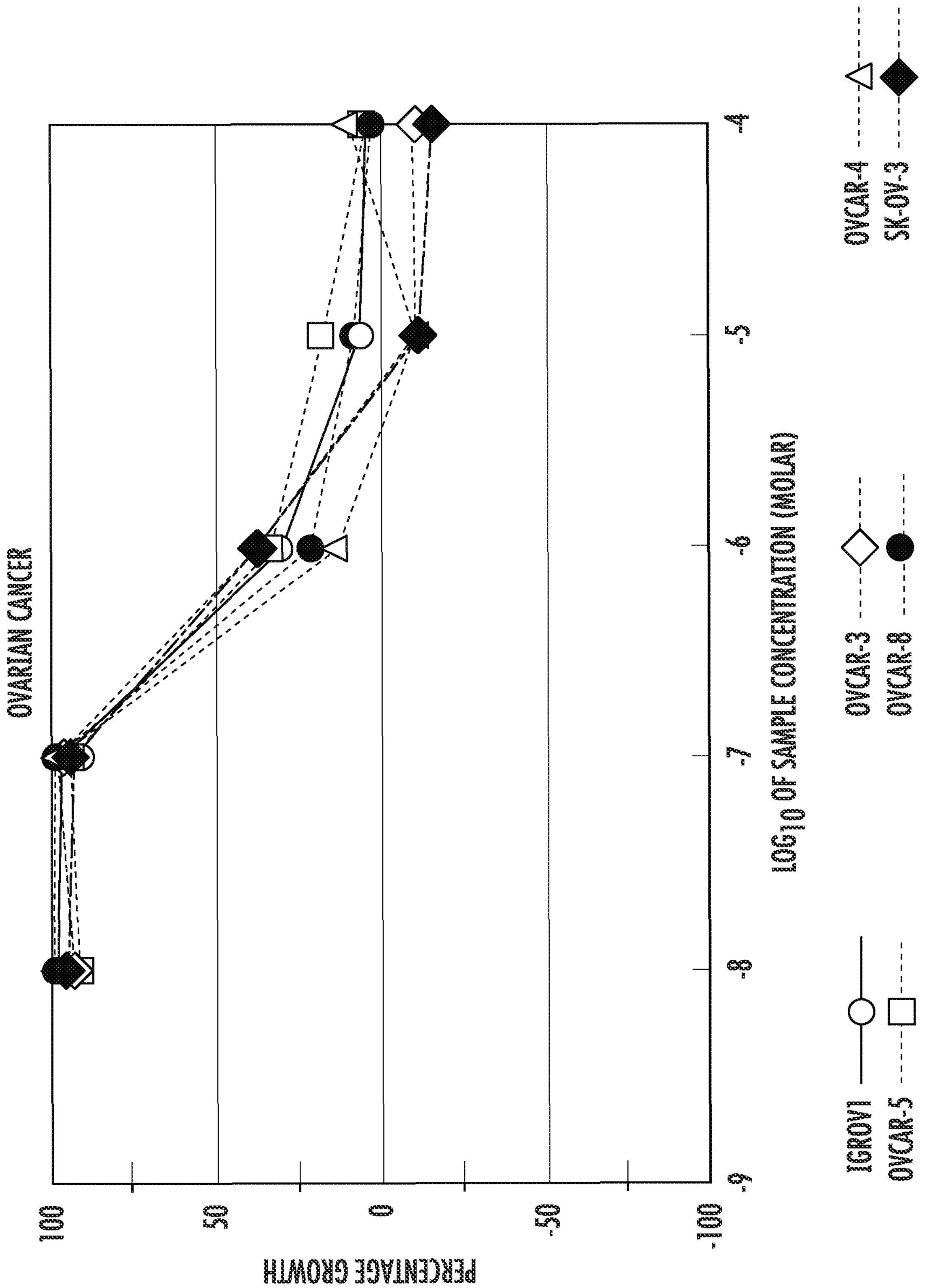
OVARIAN CANCER
PERCENTAGE GROWTH
100
50
0
-50
-100
-9
-8
-7
-6
-5
-4
LOG10 OF SAMPLE CONCENTRATION (MOLAR)
IGROV1
OVCAR-5
OVCAR-3
OVCAR-8
OVCAR-4
SK-OV-3

FIG. 56

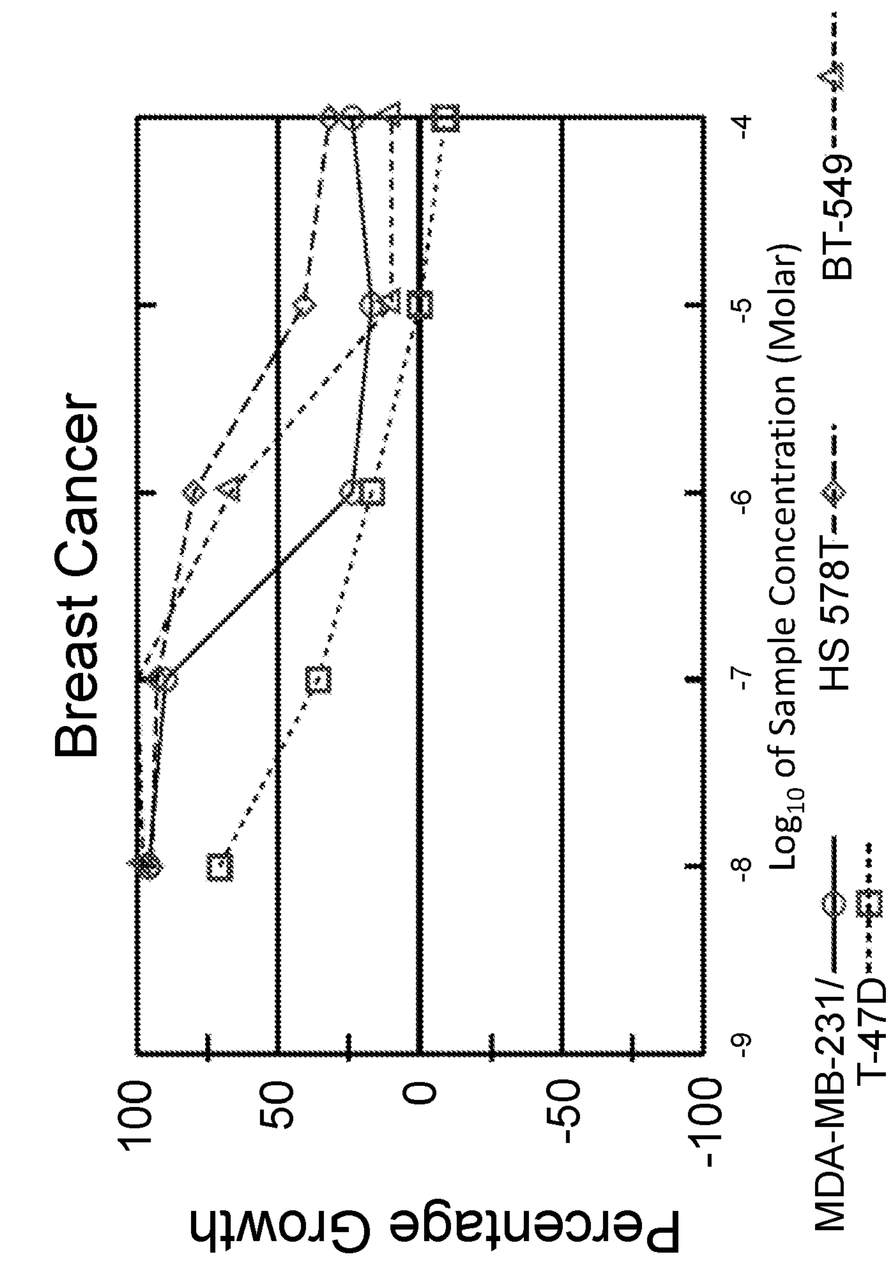
Breast Cancer
Percentage Growth
100
50
0
-50
-100
-9
-8
-7
-6
-5
-4
$Log_{10}$ of Sample Concentration (Molar)
MDA-MB-231/
HS 578T
BT-549
T-47D

FIG. 57

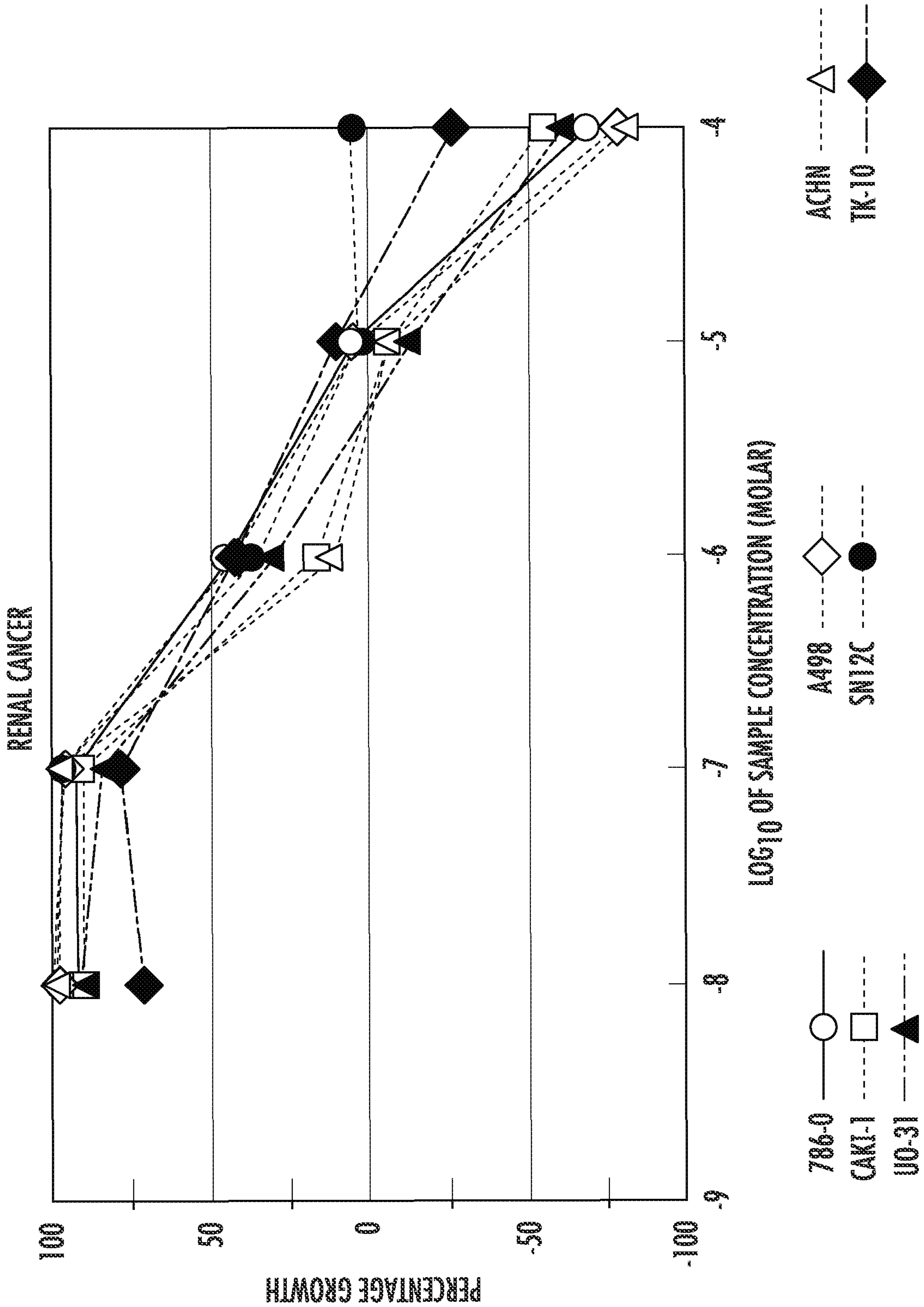
RENAL CANCER
PERCENTAGE GROWTH
100
50
0
-50
-100
-9
-8
-7
-6
-5
-4
LOG10 OF SAMPLE CONCENTRATION (MOLAR)
786-0
CAKI-1
UO-31
A498
SN12C
ACHN
TK-10

FIG. 58

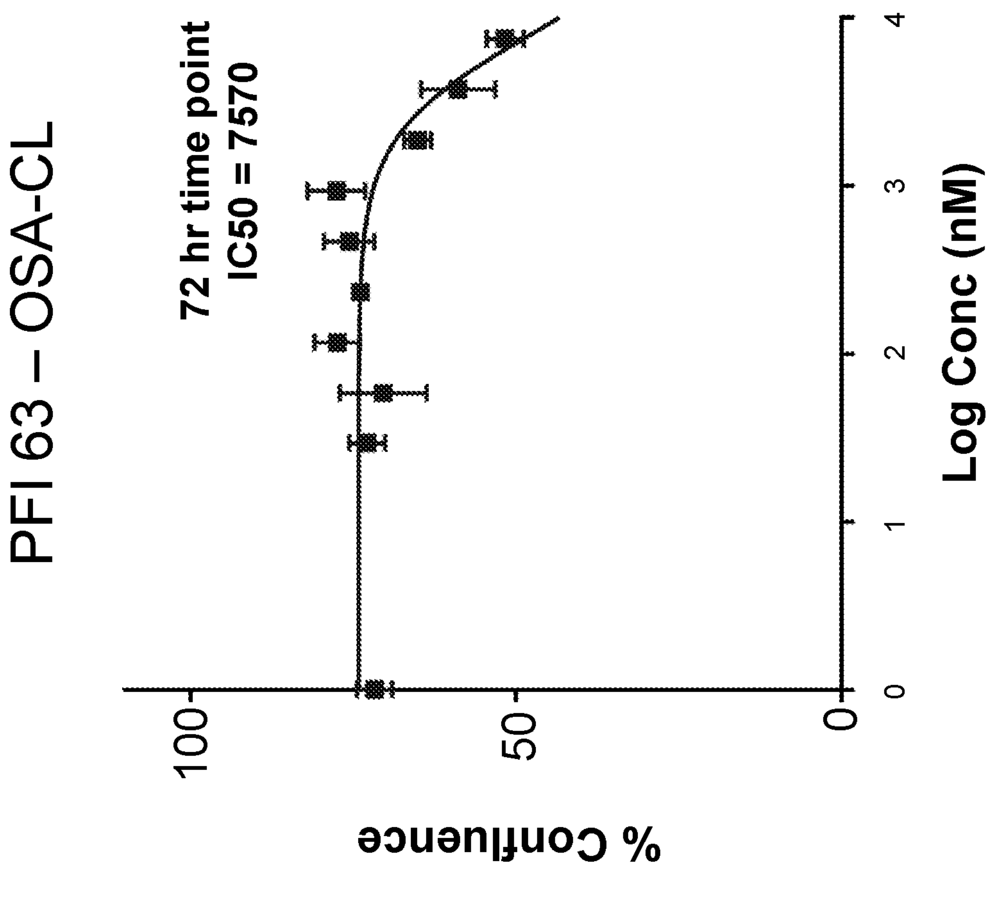
PFI 63 – OSA-CL
72 hr time point
IC50 = 7570
% Confluence
100
50
0
0
1
2
3
4
Log Conc (nM)

FIG. 59

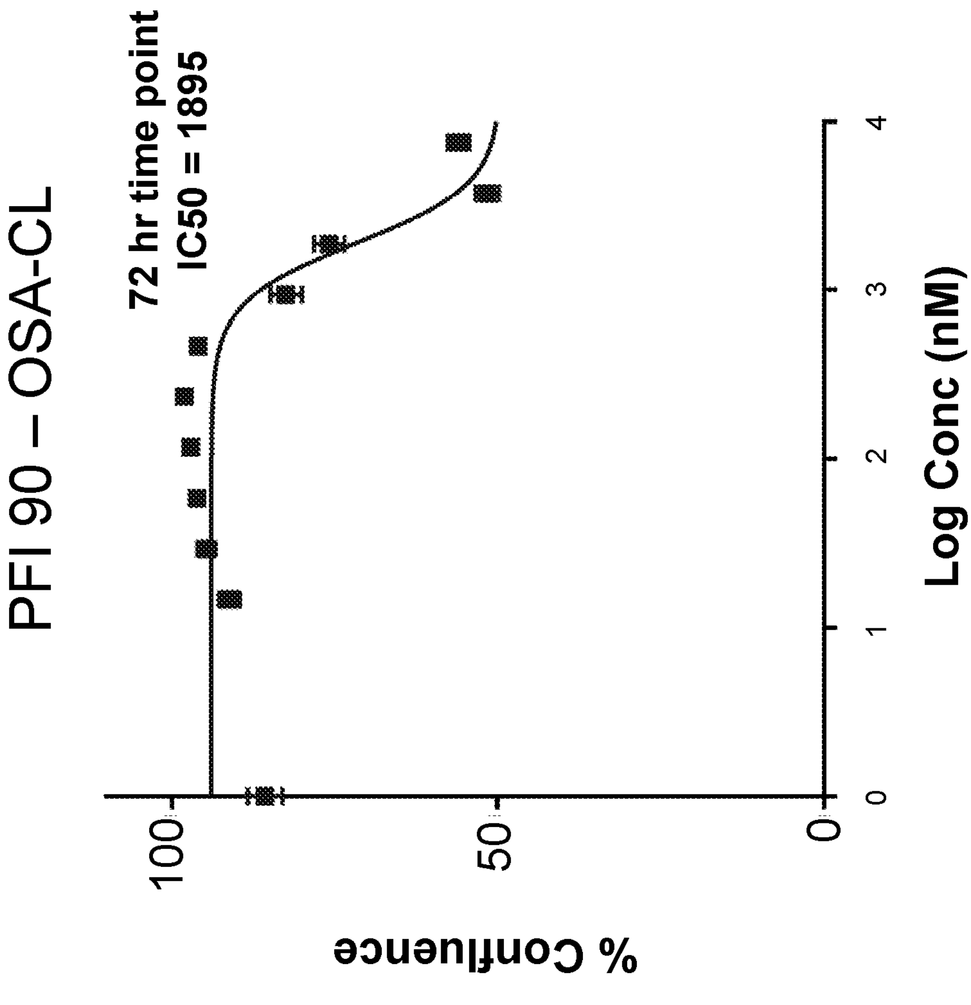
PFI 90 – OSA-CL
72 hr time point
IC50 = 1895
% Confluence
100
50
0
0
1
2
3
4
Log Conc (nM)

FIG. 60

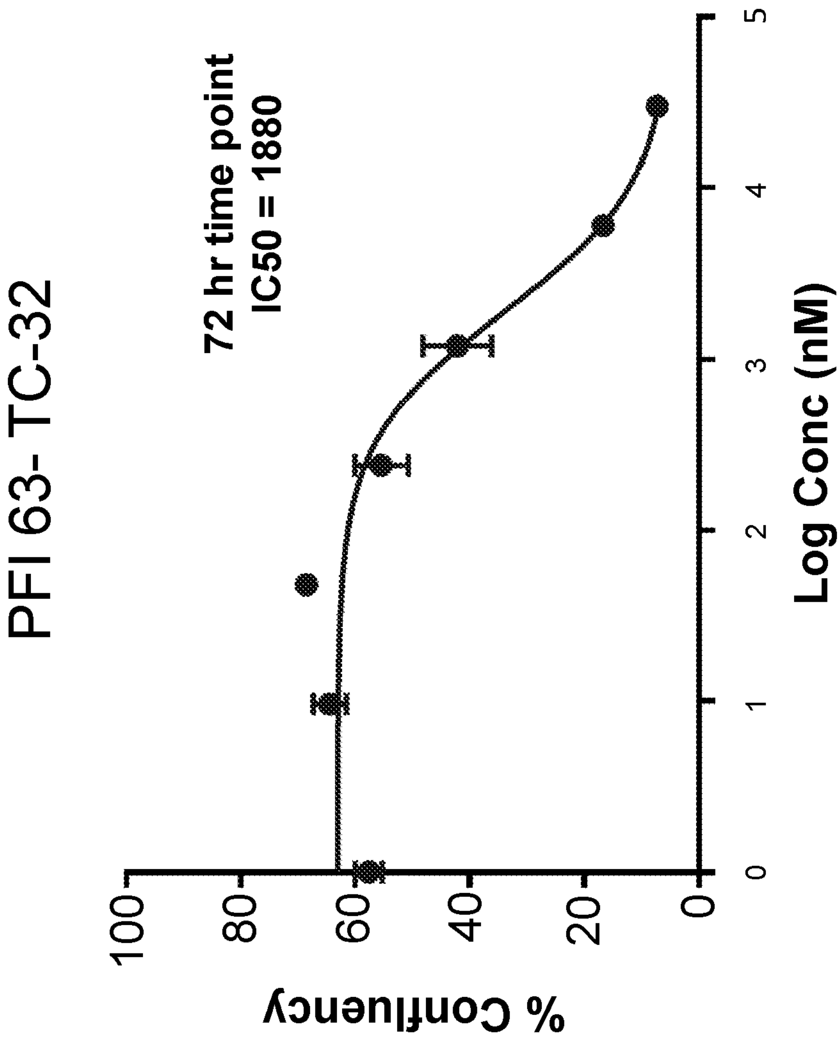
PFI 63- TC-32
72 hr time point
IC50 = 1880
% Confluency
100
80
60
40
20
0
0
1
2
3
4
5
Log Conc (nM)

FIG. 61

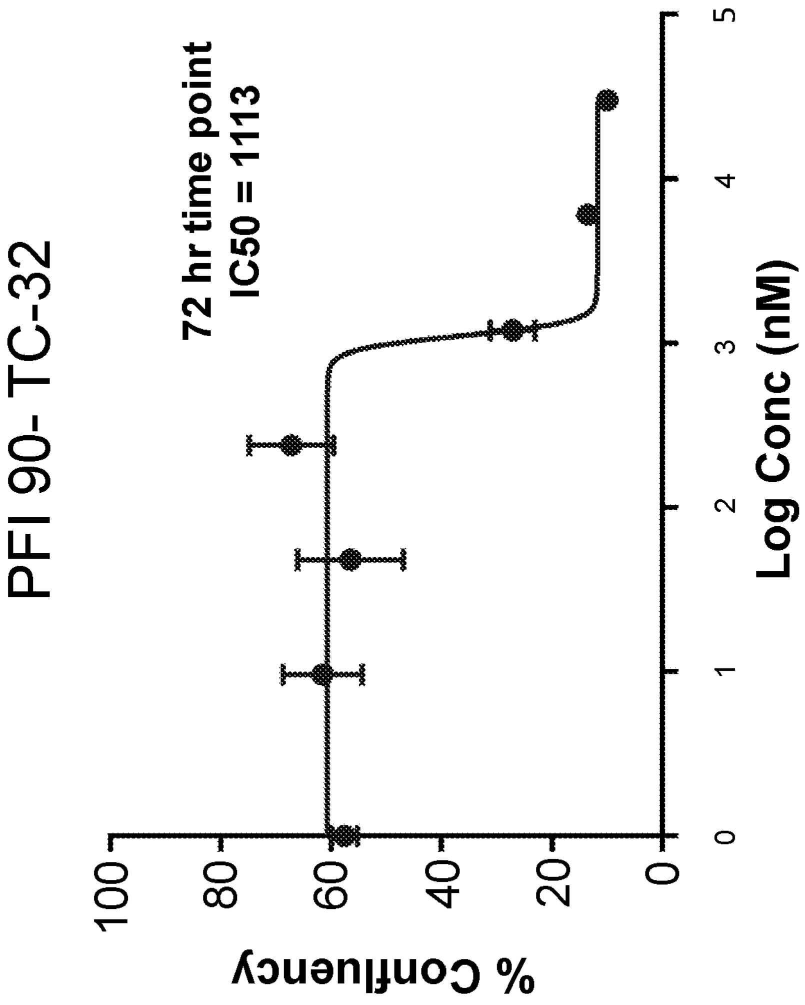
PFI 90- TC-32
72 hr time point
IC50 = 1113
% Confluency
100
80
60
40
20
0
0
1
2
3
4
5
Log Conc (nM)

FIG. 62

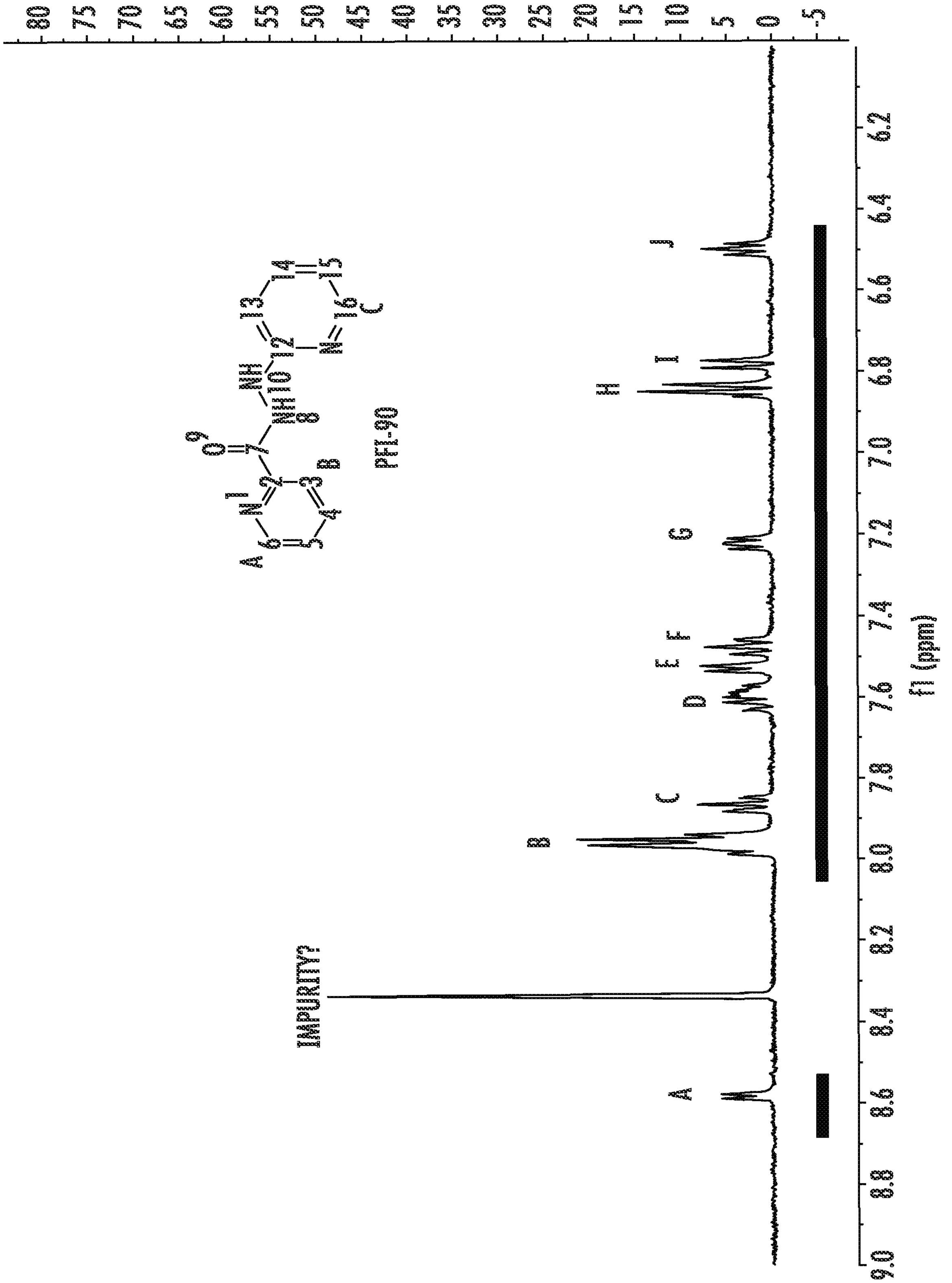


FIG. 63

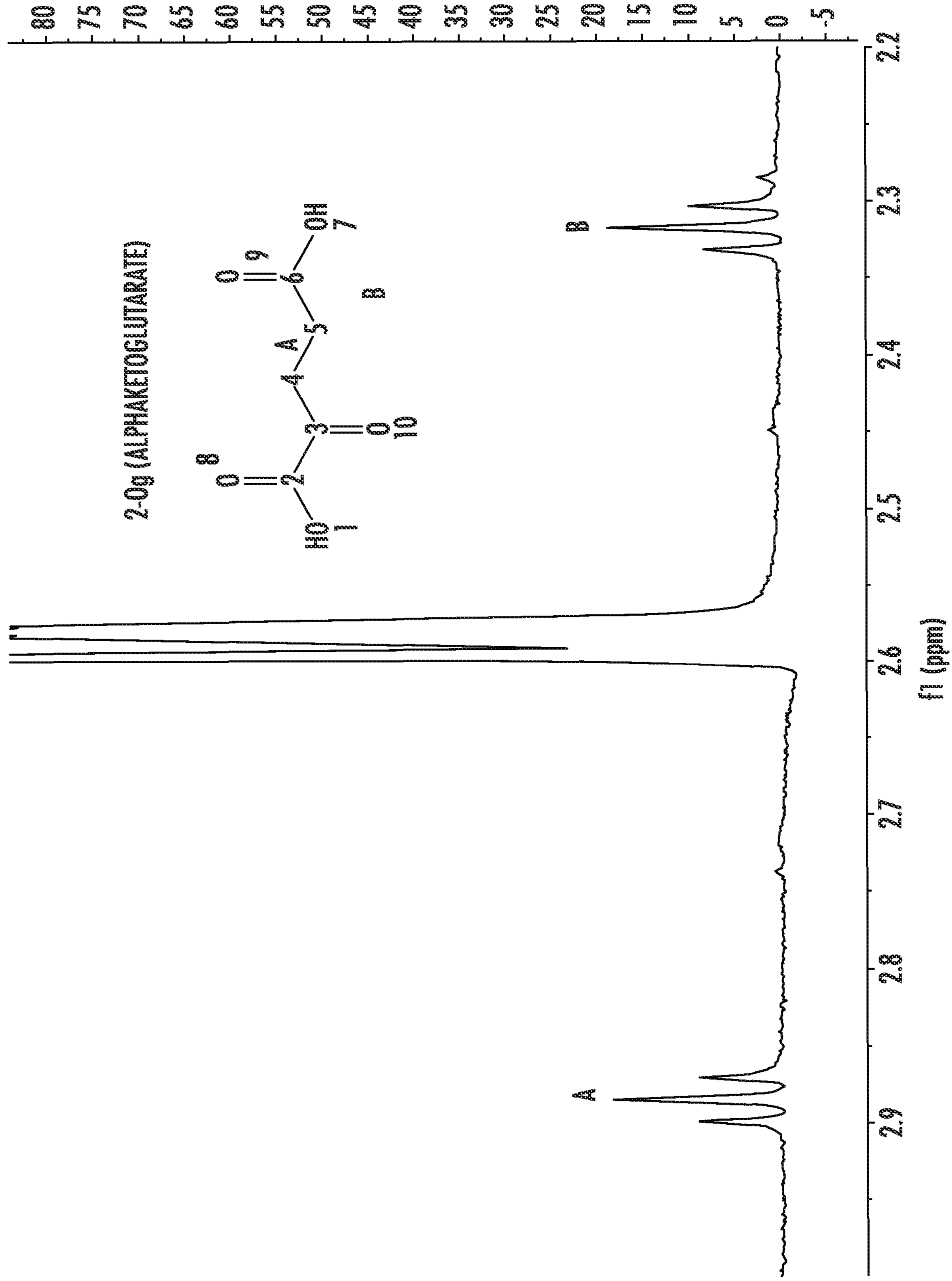


FIG. 64

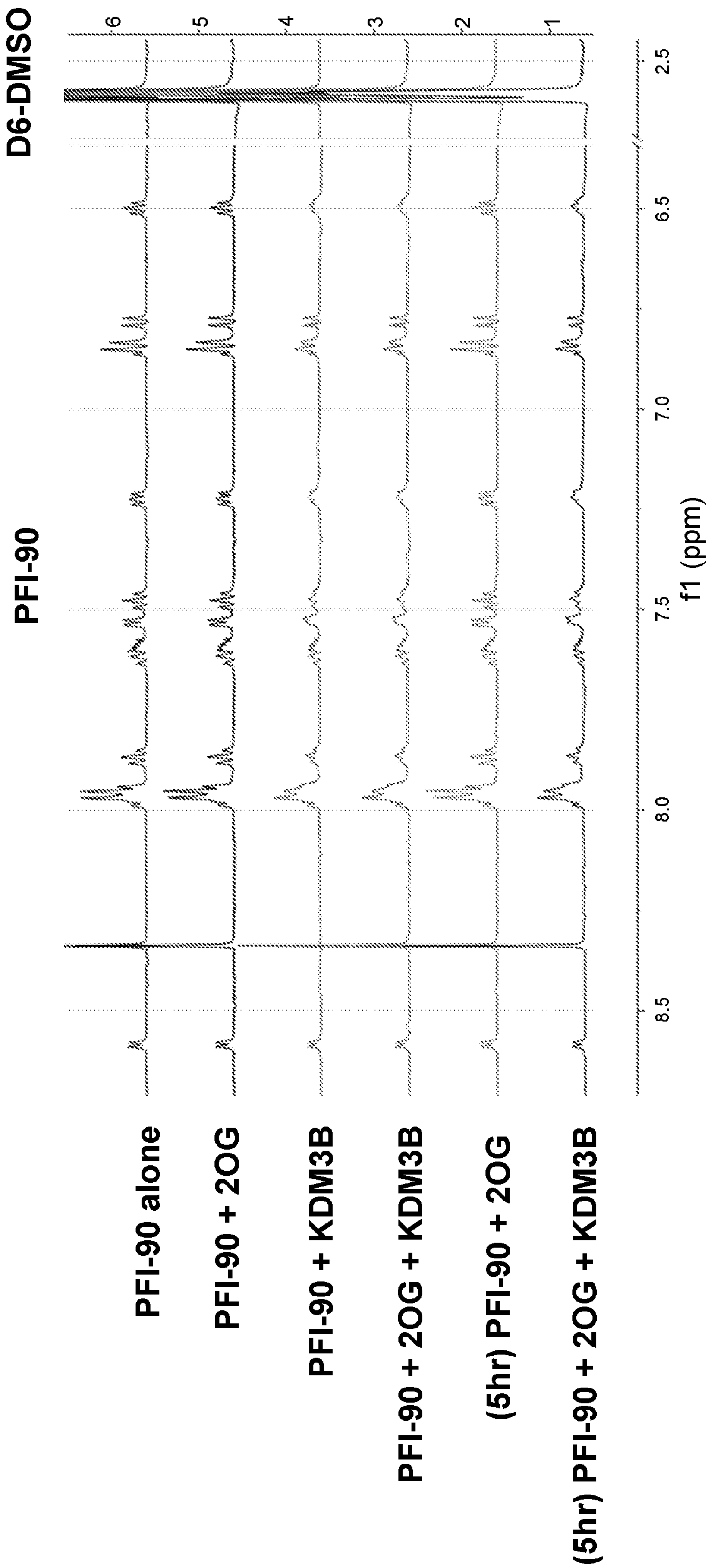
D6-DMSO
PFI-90
6
5
4
3
2
1
2.5
6.5
7.0
7.5
8.0
8.5
f1 (ppm)
PFI-90 alone
PFI-90 + 2OG
PFI-90 + KDM3B
PFI-90 + 2OG + KDM3B
(5hr) PFI-90 + 2OG
(5hr) PFI-90 + 2OG + KDM3B

FIG. 65

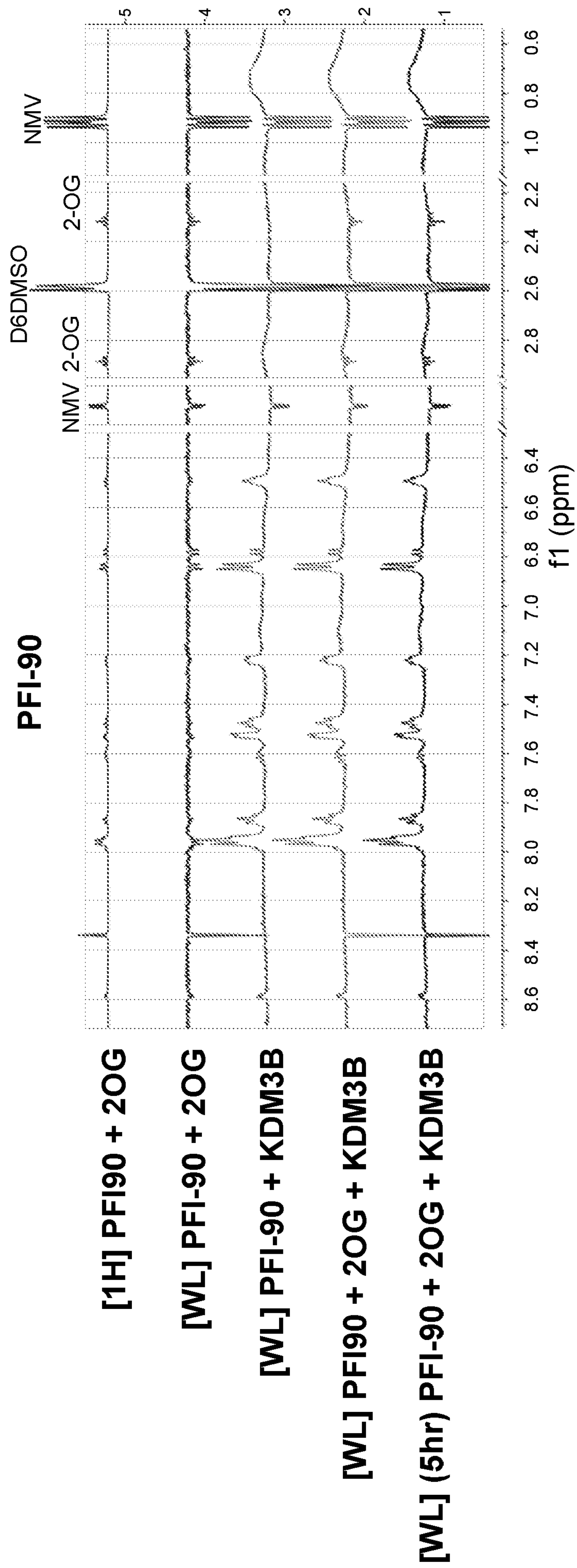
PFI-90
NMV
2-OG
D6DMSO
2-OG
NMV
5
4
3
2
1
8.6 8.4 8.2 8.0 7.8 7.6 7.4 7.2 7.0 6.8 6.6 6.4
2.8 2.6 2.4 2.2 1.0 0.8 0.6
f1 (ppm)
[1H] PFI90 + 2OG
[WL] PFI-90 + 2OG
[WL] PFI-90 + KDM3B
[WL] PFI90 + 2OG + KDM3B
[WL] (5hr) PFI-90 + 2OG + KDM3B

FIG. 66

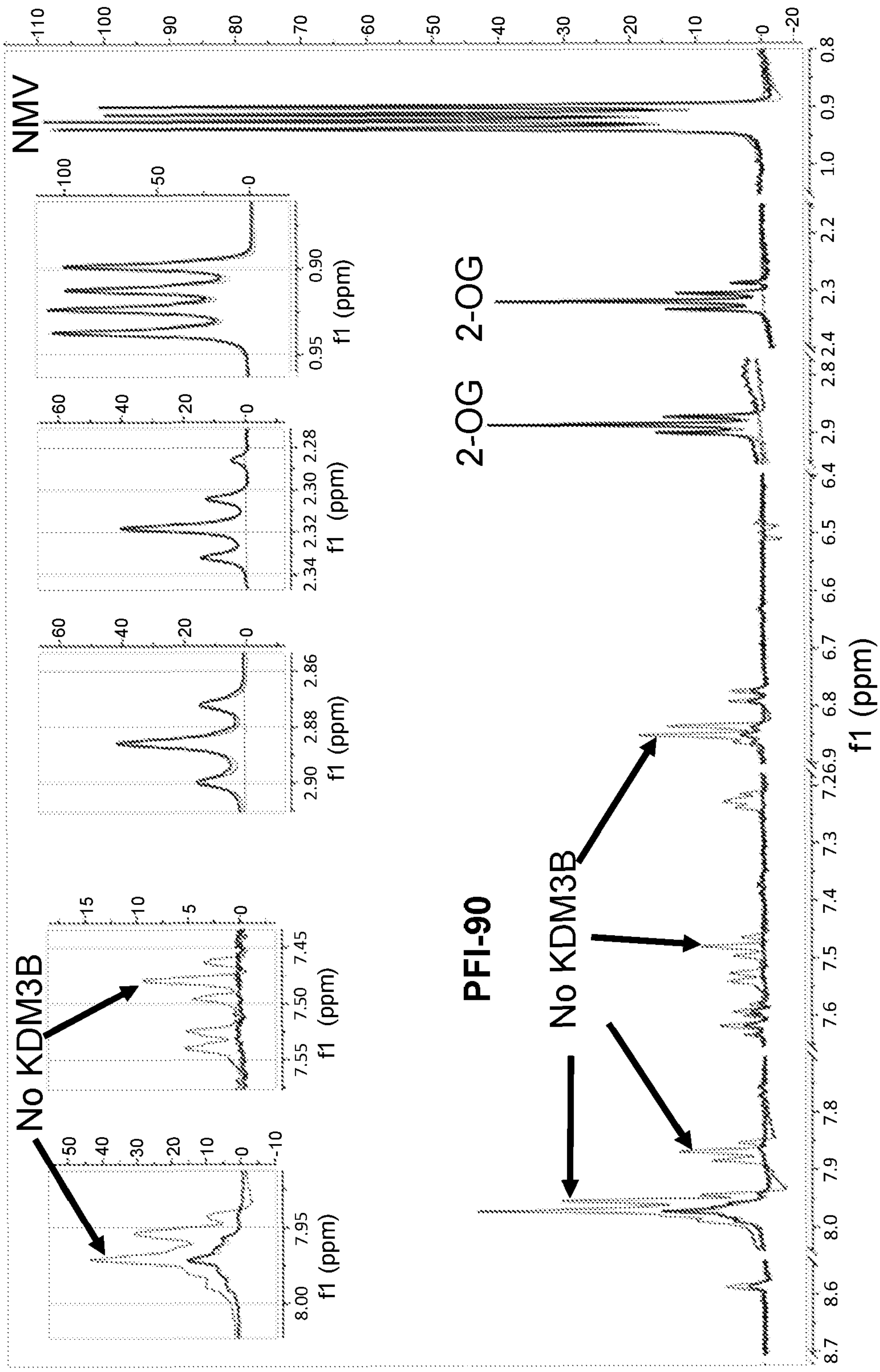


FIG. 67

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62936722
- WO 2017214413 A


### Non-patent literature cited in the description


- **GRYDER et al.** *Cancer Discov.*, 2017, vol. 7, 884-899
- **GRYDER et al.** *Nat. Commun.*, 2019, vol. 10, 3004
- **SUBRAMANIAN et al.** *Proc. Natl. Acad. Sci. USA*, 2005, vol. 102, 15545-15550
- **EBAUER et al.** *Oncogene*, 2007, vol. 26, 7267-7281
- **DAVICIONI et al.** *Cancer Res.*, 2006, vol. 66, 6936-6946
- **CAO et al.** *Cancer Res.*, 2010, vol. 70, 6497-6508
- **GRYDER et al.** *Cancer Discov*, 2017, vol. 7, 884-899
- **KUROKI et al.** *Stem Cell Reports*, 2018, vol. 10, 1340-1354
- **AN et al.** *Biochem. Biophys. Res. Commun.*, 2019, vol. 508, 576-582
- **LIBERZON et al.** *Cell Systems*, 2015, vol. 1, 417-425
- **WANG et al.** *Nat. Commun.*, 2013, vol. 4, 2035
- The Role of the Lysine Demethylases KDM5 and KDM6 in Bone Malignancies. **HOOKWAY, E.** Ph.D. Thesis. University of Oxford, 2016
- **HOFFMANN et al.** *Mol. Oncol.,*, 2012, vol. 6, 683-703
- **BRAUCHLE et al.** *PLoS ONE*, 2013, vol. 8, e60549
- **LI et al.** *Nat. Commun.*, 2017, vol. 8, 15146
- **KIM et al.** *Mol. Cell. Biol.*, 2012, vol. 32, 2917-2933
- **DALVI et al.** *Cell Reports*, 2017, vol. 19, 1669-1684
- Remington's Pharmaceutical Sciences. Mack Publishing Company, 1990, 1445
- *Journal of Pharmaceutical Science*, 1977, vol. 66, 2-19
- Pharmaceutics and Pharmacy Practice. J. B. Lippincott Company, 1982, 238-250
- **TOISSEL**. ASHP Handbook on Injectable Drugs,. 1986, 622-630
- **GROHAR et al.** *J. Natl. Cancer Inst.,*, 2011, vol. 103, 962-978
- **ZHANG et al.** *J. Biomol. Screen.*, 1999, vol. 4, 67-73
- **SHOEMAKER et al.** *Nat. Rev. Cancer,*, 2006, vol. 6, 813-823